# EUROPEAN PATENT APPLICATION

(11) **EP 4 265 612 A1**
(43) Date of publication of application: **25.10.2023**
(21) Application number: 21911295.0
(22) Date of filing: 30.11.2021
(51) Int. Cl.: C07D 405/14, C07D 409/14, C07D 491/048, C07D 495/04, C07D 403/14, H01L 51/00, H01L 51/50

(54) **HETEROCYCLIC COMPOUND, ORGANIC LIGHT-EMITTING DEVICE COMPRISING SAME, MANUFACTURING METHOD THEREFOR, AND COMPOSITION FOR ORGANIC MATERIAL LAYER**

(30) Priority: 21.12.2020 KR 20200180284
(71) Applicant: LT Materials Co., Ltd., Yongin-si, Gyeonggi-do 17118 (KR)
(72) Inventor: CHOI, Eui-Jeong, Yongin-City, Gyeonggi-do 17118 (KR); NO, Young-Seok, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/017908
(87) International publication number: WO 2022/139228

(57) **Abstract**

The present specification relates to a heterocyclic compound represented by Chemical Formula 1, an organic light emitting device comprising the same, a manufacturing method thereof, and an organic material layer composition.

## Description

### [Technical Field]

The present specification relates to a heterocyclic compound, an organic light emitting device comprising the same, a manufacturing method thereof, and an organic material layer composition.

This application claims priority to and the benefits of Korean Patent Application No. 10-2020-0180284, filed with the Korean Intellectual Property Office on December 21, 2020, the entire contents of which are incorporated herein by reference.

### [Background Art]

An organic electroluminescent device is a type of self-emission type display device, and there are advantages in that it not only has a wide viewing angle and excellent contrast, but also has fast response speed.

An organic light emitting device has a structure in which an organic thin film is disposed between two electrodes. When a voltage is applied to the organic light emitting device having such a structure, electrons and holes injected from the two electrodes combine in the organic thin film to form a pair, and then emit light while being disappeared. The organic thin film may be composed of a single layer or multiple layers as needed.

A material of the organic thin film may have a light emitting function as needed. For example, as the organic thin film material, a compound capable of forming the light emitting layer by itself may be used, or a compound capable of serving as a host or dopant of the host-dopant based light emitting layer may also be used. In addition, compounds capable of performing the roles of hole injection, hole transport, electron blocking, hole blocking, electron transport, electron injection, etc. may also be used as a material of the organic thin film.

In order to improve the performance, lifespan, or efficiency of the organic light emitting device, the development of the material of the organic thin film is continuously required.

### Prior Art Documents

(Patent Document 1) US Patent No. 4,356,429

### [Disclosure]

### [Technical Problem]

An object of the present disclosure is to provide a heterocyclic compound, an organic light emitting device comprising the same, a method for manufacturing the organic light emitting device, and an organic material layer composition.

### [Technical Solution]

In an embodiment of the present application, a heterocyclic compound represented by Chemical Formula 1 below is provided. 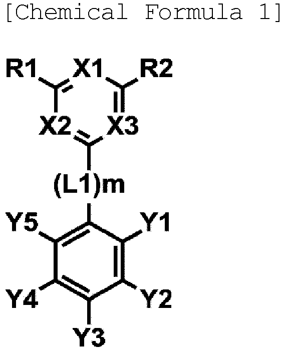
in Chemical Formula 1,
R1 and R2 are the same as or different from each other, and are each independently hydrogen; deuterium; a substituted or unsubstituted C₁-C₆₀ alkyl group; a substituted or unsubstituted C₆-C₆₀ aryl group; or a substituted or unsubstituted C₂-C₆₀ heteroaryl group,
X1 to X3 are the same as or different from each other and are each independently CRx; or N, and at least two thereof are N,
L1 is a direct bond; a substituted or unsubstituted C₆-C₆₀ arylene group; or a substituted or unsubstituted C₂-C₆₀ heteroarylene group,
Rx is hydrogen; deuterium; a substituted or unsubstituted C₆-C₆₀ aryl group; or a substituted or unsubstituted C₂-C₆₀ heteroaryl group,
two of Y1 to Y5 are represented by Chemical Formula A or Chemical Formula B below, and the remainder is hydrogen; or deuterium, 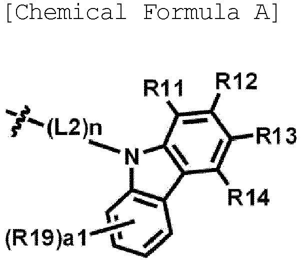 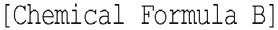 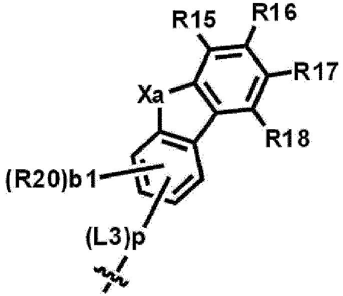
in Chemical Formula A and Chemical Formula B,
L2 and L3 are the same as or different from each other, and are each independently a direct bond; a substituted or unsubstituted C₆-C₆₀ arylene group; or a substituted or unsubstituted C₂-C₆₀ heteroarylene group,
Xa is O; S; or NRy,
R11 to R20 are the same as or different from each other, and are each independently hydrogen; or deuterium, or two or more groups adjacent to each other are bonded to each other to form a substituted or unsubstituted C₆-C₆₀ aromatic hydrocarbon ring or a substituted or unsubstituted C₂-C₆₀ heteroring,
Ry is a substituted or unsubstituted C₆-C₆₀ aryl group; or a substituted or unsubstituted C₂-C₆₀ heteroaryl group,
m, n, a1, and p are integers of 0 to 4,
b1 is an integer of 0 to 3,
when m, n, a1, b1, and p are each 2 or more, the substituents in parentheses are the same as or different from each other,
when two of Y1 to Y5 are represented by Chemical Formula B above, and Xa is O; or S, the two Chemical Formulae B are different from each other,
when two of Y1 to Y5 are represented by Chemical Formula A above, and Chemical Formula A above is substituted with Y2, the remaining Chemical Formula A is substituted with one of Y1, Y3, and Y5,
when two of Y1 to Y5 are Chemical Formula B in which Xa is NRy, and Chemical Formula B in which Xa is NRy is substituted with Y2 and Y4, at least two among R15 to R18 of Chemical Formula B in which Xa is NRy, which is substituted with Y2, are bonded to each other to form a substituted or unsubstituted C₆-C₆₀ aromatic hydrocarbon ring or a substituted or unsubstituted C₂-C₆₀ heteroring, and
when two of Y1 to Y5 are Chemical Formula B in which Xa is NRy, and Chemical Formula A, Chemical Formula A above is substituted with Y2, and Chemical Formula B in which Xa is NRy is substituted with Y4, at least two among R11 to R14 of Chemical Formula A above substituted with Y2 are bonded to each other to form a substituted or unsubstituted C₆-C₆₀ aromatic hydrocarbon ring or a substituted or unsubstituted C₂-C₆₀ heteroring.

Further, in an embodiment of the present application, there is provided an organic light emitting device comprising: a first electrode; a second electrode provided opposite to the first electrode; and one or more organic material layers provided between the first electrode and the second electrode, wherein one or more layers of the organic material layers comprise a heterocyclic compound represented by Chemical Formula 1 above.

Further, an embodiment of the present application provides an organic light emitting device in which the organic material layer comprising the heterocyclic compound of Chemical Formula 1 above further comprises a heterocyclic compound represented by Chemical Formula 2 below. 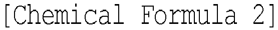 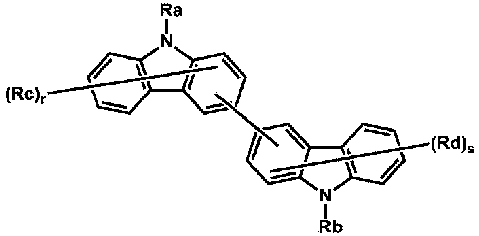
in Chemical Formula 2,
Rc and Rd are the same as or different from each other, and are each independently selected from the group consisting of: hydrogen; deuterium; a halogen group; -CN; a substituted or unsubstituted C₁-C₆₀ alkyl group; a substituted or unsubstituted C₂-C₆₀ alkenyl group; a substituted or unsubstituted C₂-C₆₀ alkynyl group; a substituted or unsubstituted C₁-C₆₀ alkoxy group; a substituted or unsubstituted C₃-C₆₀ cycloalkyl group; a substituted or unsubstituted C₂-C₆₀ heterocycloalkyl group; a substituted or unsubstituted C₆-C₆₀ aryl group; a substituted or unsubstituted C₂-C₆₀ heteroaryl group; -SiR101R102R103; - P(=O)R101R102; and -NR101R102, or two or more groups adjacent to each other are bonded to each other to form a substituted or unsubstituted C₆-C₆₀ aromatic hydrocarbon ring or a substituted or unsubstituted C₂-C₆₀ heteroring,
Ra and Rb are the same as or different from each other, and are each independently a substituted or unsubstituted C₆-C₆₀ aryl group; a substituted or unsubstituted C₂-C₆₀ heteroaryl group; -SiR101R102R103; or -P(=O)R101R102,
R101, R102, and R103 are the same as or different from each other, and are each independently hydrogen; deuterium; - CN; a substituted or unsubstituted C₁-C₆₀ alkyl group; a substituted or unsubstituted C₆-C₆₀ aryl group; or a substituted or unsubstituted C₂-C₆₀ heteroaryl group, and
r and s are integers of 0 to 7, and when r and s are each 2 or more, the substituents in parentheses are the same as or different from each other.

Further, another embodiment of the present application provides an organic material layer composition of an organic light emitting device, comprising a heterocyclic compound represented by Chemical Formula 1 above and a heterocyclic compound represented by Chemical Formula 2 above.

Finally, an embodiment of the present application provides a method for manufacturing an organic light emitting device, the method comprising the steps of: preparing a substrate; forming a first electrode on the substrate; forming one or more organic material layers on the first electrode; and forming a second electrode on the organic material layer, wherein the step of forming the organic material layer comprises a step of forming one or more organic material layers using the organic material layer composition according to an embodiment of the present application.

### [Advantageous Effects]

The compound described in the present specification can be used as a material for an organic material layer of an organic light emitting device. The compound may play a role of a hole injection material, a hole transport material, a light emitting material, an electron transport material, an electron injection material, etc. in the organic light emitting device. In particular, the compound may be used as a light emitting layer material of the organic light emitting device.

Specifically, the compound may be used alone as a light emitting material, or may be used as a host material or a dopant material of the light emitting layer. When the compound represented by Chemical Formula 1 above is used in the organic material layer, the driving voltage of the device can be lowered, the light efficiency can be improved, and the lifespan characteristics of the device can be improved by the thermal stability of the compound.

In particular, the heterocyclic compound represented by Chemical Formula 1 above and the heterocyclic compound represented by Chemical Formula 2 above can be simultaneously used as a material of the light emitting layer of the organic light emitting device. In this case, the driving voltage of the device can be lowered, the light efficiency can be improved, and the lifespan characteristics of the device can be particularly improved due to the thermal stability of the compound.

### [Description of Drawings]

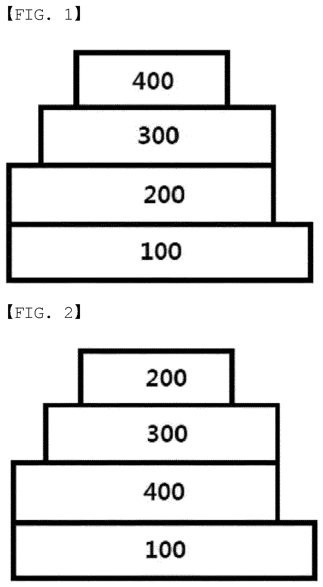

FIGS. 1 to 3 are diagrams each schematically illustrating a lamination structure of an organic light emitting device according to an embodiment of the present application.

### <Reference Numeral>

100: Substrate
200: Anode
300: Organic Material Layer
301: Hole Injection Layer
302: Hole Transport Layer
303: Light Emitting Layer
304: Hole Blocking Layer
305: Electron Transport Layer
306: Electron Injection Layer
400: Cathode

### [Mode for Disclosure]

Hereinafter, the present application will be described in detail.

In the present specification, the halogen may be fluorine, chlorine, bromine, or iodine.

In the present specification, the alkyl group may comprise a linear or branched chain having 1 to 60 carbon atoms, and may be further substituted by other substituents. The number of carbon atoms of the alkyl group may be 1 to 60, specifically 1 to 40, and more specifically, 1 to 20. Specific examples of the alkyl group may comprise a methyl group, an ethyl group, a propyl group, an n-propyl group, an isopropyl group, a butyl group, an n-butyl group, an isobutyl group, a tert-butyl group, a sec-butyl group, a 1-methyl-butyl group, an 1-ethyl-butyl group, a pentyl group, an n-pentyl group, an isopentyl group, a neopentyl group, a tert-pentyl group, a hexyl group, an n-hexyl group, an 1-methylpentyl group, a 2-methylpentyl group, a 4-methyl-2-pentyl group, a 3,3-dimethylbutyl group, a 2-ethylbutyl group, a heptyl group, an n-heptyl group, an 1-methylhexyl group, a cyclopentylmethyl group, a cyclohexylmethyl group, an octyl group, an n-octyl group, a tert-octyl group, an 1-methylheptyl group, a 2-ethylhexyl group, a 2-propylpentyl group, an n-nonyl group, a 2,2-dimethylheptyl group, an 1-ethyl-propyl group, an 1,1-dimethyl-propyl group, an isohexyl group, a 2-methylpentyl group, a 4-methylhexyl group, a 5-methylhexyl group, etc., but are not limited thereto.

In the present specification, the alkenyl group may comprise a linear or branched chain having 2 to 60 carbon atoms, and may be further substituted by other substituents. The number of carbon atoms of the alkenyl group may be 2 to 60, specifically 2 to 40, and more specifically 2 to 20. Specific examples of the alkenyl group may comprise a vinyl group, an 1-propenyl group, an isopropenyl group, an 1-butenyl group, a 2-butenyl group, a 3-butenyl group, an 1-pentenyl group, a 2-pentenyl group, a 3-pentenyl group, a 3-methyl-1-butenyl group, an 1,3-butadienyl group, an allyl group, an 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stylbenyl group, a styrenyl group, etc., but are not limited thereto.

In the present specification, the alkynyl group may comprise a linear or branched chain having 2 to 60 carbon atoms, and may be further substituted by other substituents. The number of carbon atoms of the alkynyl group may be 2 to 60, specifically 2 to 40, and more specifically 2 to 20.

In the present specification, the alkoxy group may be a linear, branched, or cyclic chain. The number of carbon atoms of the alkoxy group is not particularly limited, but is preferably 1 to 20. Specific examples of the alkoxy group may comprise methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, etc., but are not limited thereto.

In the present specification, the cycloalkyl group may comprise a monocyclic or polycyclic ring having 3 to 60 carbon atoms, and may be further substituted by other substituents. Here, the polycyclic ring refers to a group in which a cycloalkyl group is directly connected or condensed with other ring group. Here, although the other ring group may be a cycloalkyl group, it may also be a different type of ring group, for example, a heterocycloalkyl group, an aryl group, a heteroaryl group, or the like. The number of carbon atoms of the cycloalkyl group may be 3 to 60, specifically 3 to 40, and more specifically 5 to 20. Specific examples of the cycloalkyl group may comprise a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, etc., but are not limited thereto.

In the present specification, the heterocycloalkyl group may comprise O, S, Se, N, or Si as a heteroatom, may comprise a monocyclic or polycyclic ring having 2 to 60 carbon atoms, and may be further substituted by other substituents. Here, the polycyclic ring refers to a group in which a heterocycloalkyl group is directly connected or condensed with other ring group. Here, although the other ring group may be a heterocycloalkyl group, it may also be a different type of ring group, for example, a cycloalkyl group, an aryl group, a heteroaryl group, or the like. The number of carbon atoms of the heterocycloalkyl group may be 2 to 60, specifically 2 to 40, and more specifically 3 to 20.

In the present specification, the aryl group may comprise a monocyclic or polycyclic ring having 6 to 60 carbon atoms, and may be further substituted by other substituents. Here, the polycyclic ring means a group in which an aryl group is directly connected or condensed with other ring group. Here, although the other ring group may be an aryl group, it may also be a different type of ring group, for example, a cycloalkyl group, a heterocycloalkyl group, a heteroaryl group, or the like. The number of carbon atoms of the aryl group may be 6 to 60, specifically 6 to 40, and more specifically 6 to 25. Specific examples of the aryl group may comprise a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a 2,3-dihydro-1H-indenyl group, condensed ring groups thereof, etc., but are not limited thereto.

In the present specification, the fluorenyl group may be substituted, and adjacent substituents may be bonded to each other to form a ring.

When the fluorenyl group is substituted, it may be structures below, etc., but is not limited thereto.

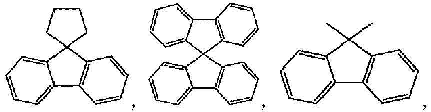

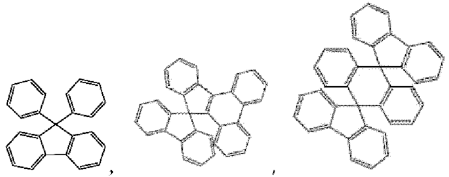

In the present specification, the heteroaryl group may comprise S, O, Se, N, or Si as a heteroatom, may comprise a monocyclic or polycyclic ring having 2 to 60 carbon atoms, and may be further substituted by other substituents. Here, the polycyclic ring refers to a group in which a heteroaryl group is directly connected or condensed with other ring group. Here, although the other ring group may be a heteroaryl group, it may also be a different type of ring group, for example, a cycloalkyl group, a heterocycloalkyl group, an aryl group, or the like. The number of carbon atoms of the heteroaryl group may be 2 to 60, specifically 2 to 40, and more specifically 3 to 25. Specific examples of the heteroaryl group may comprise a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a deoxynyl group, a triazinyl group, a tetrazinyl group, a quinolyl group, an isoquinolyl group, a quinazolinyl group, an isoquinazolinyl group, a quinozolilyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilol group, a spirobi (dibenzosilole) group, dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiazinyl group, a phthalazinyl group, a naphthylidinyl group, a phenanthrolinyl group, a benzo[c][1,2,5]thiadiazolyl group, a 5,10-dihydrodibenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group, etc., but are not limited thereto.

In the present specification, the amine group may be selected from the group consisting of: a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group may comprise a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, etc., but are not limited thereto.

In the present specification, the arylene group means one having two bonding positions in the aryl group, that is, a divalent group. The description of the aryl group described above may be applied except that each of these is a divalent group. Further, the heteroarylene group means one having two bonding positions in the heteroaryl group, that is, a divalent group. The description of the heteroaryl group described above may be applied except that each of these is a divalent group.

In the present specification, the phosphine oxide group may be represented by -P(=O)R₁₀₁R₁₀₂, R₁₀₁ and R₁₀₂ may be the same as or different from each other, and may be each independently a substituent consisting of at least one of: hydrogen; deuterium; a halogen group; an alkyl group; an alkenyl group; an alkoxy group; a cycloalkyl group; an aryl group; and a heterocyclic group. The phosphine oxide group may specifically comprise a diphenylphosphineoxide group, a dinaphthylphosphineoxide group, etc., but is not limited thereto.

In the present specification, the silyl group may be a substituent which comprises Si, and to which the Si atom is directly connected as a radical, and may be represented by - SiR₁₀₄R₁₀₅R₁₀₆, and R₁₀₄ to R₁₀₆ may be the same as or different from each other and each independently a substituent consisting of at least one of hydrogen; deuterium; a halogen group; an alkyl group; an alkenyl group; an alkoxy group; a cycloalkyl group; an aryl group; and a heterocyclic group. Specific examples of the silyl group may comprise a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, etc., but are not limited thereto.

In the present specification, the "adjacent" group may mean a substituent substituted on an atom directly connected to the atom in which the corresponding substituent is substituted, a substituent positioned to be sterically closest to the corresponding substituent, or another substituent substituted on the atom in which the corresponding substituent is substituted. For example, two substituents substituted at an ortho position in a benzene ring and two substituents substituted at the same carbon in an aliphatic ring may be interpreted as groups "adjacent" to each other.

The structures exemplified by the above-described cycloalkyl group, cycloheteroalkyl group, aryl group and heteroaryl group may be applied except that the aliphatic or aromatic hydrocarbon ring or heteroring that adjacent groups may form is not a monovalent group.

In the present specification, the term "substitution" means that a hydrogen atom bonded to a carbon atom of a compound is changed to another substituent, and the position to be substituted is not limited as long as it is a position at which the hydrogen atom is substituted, that is, a position where the substituent is substitutable, and when two or more substituents are substituted, two or more substituents may be the same as or different from each other.

In the present specification, "substituted or unsubstituted" means that it is substituted or unsubstituted with one or more substituents selected from the group consisting of: C₁-C₆₀ linear or branched alkyl; C₂-C₆₀ linear or branched alkenyl; C₂-C₆₀ linear or branched alkynyl; C₃-C₆₀ monocyclic or polycyclic cycloalkyl; C₂-C₆₀ monocyclic or polycyclic heterocycloalkyl; C₆-C₆₀ monocyclic or polycyclic aryl; C₂-C₆₀ monocyclic or polycyclic heteroaryl; -SiRR'R"; - P(=O)RR'; C₁-C₂₀ alkylamine; C₆-C₆₀ monocyclic or polycyclic arylamine; and C₂-C₆₀ monocyclic or polycyclic heteroarylamine, or is substituted or unsubstituted with a substituent to which two or more substituents selected from the above-exemplified substituents are connected, and

R, R' and R" are the same as or different from each other, and are each independently a substituted or unsubstituted C₁-C₆₀ alkyl group; a substituted or unsubstituted C₆-C₆₀ aryl group; or a substituted or unsubstituted C₂-C₆₀ heteroaryl group.

In the present specification, "when a substituent is not indicated in a chemical formula or compound structure" means that a hydrogen atom is bonded to a carbon atom. However, since deuterium (²H, Deuterium) is an isotope of hydrogen, some hydrogen atoms may be deuterium.

In an embodiment of the present application, "when a substituent is not indicated in a chemical formula or compound structure" may mean that all positions where a substituent can come are hydrogen or deuterium. That is, deuterium may be an isotope of hydrogen, and some hydrogen atoms may be deuterium that is an isotope, and the content of deuterium may be 0 to 100% at this time.

In an embodiment of the present application, in the case of "when a substituent is not indicated in a chemical formula or compound structure", if deuterium is not explicitly excluded, such as the content of deuterium of 0%, the content of hydrogen of 100%, and all of the substituents being hydrogen, hydrogen and deuterium may be mixed and used in the compound.

In an embodiment of the present application, deuterium is an element having a deuteron consisting of one proton and one neutron as one of the isotopes of hydrogen as a nucleus, it may be expressed as hydrogen-2, and an element symbol may also be written as D or 2H.

In an embodiment of the present application, although isotopes meaning atoms which have the same atomic number (Z), but have different mass numbers (A) have the same number of protons, they may also be interpreted as elements with different numbers of neutrons.

In an embodiment of the present application, when the total number of substituents that a basic compound may have is defined as T1, and the number of specific substituents among them is defined as T2, the meaning of the content T% of the specific substituents may be defined as T2/T1×100 = T%.

That is, as an example, the 20% content of deuterium in the phenyl group represented by 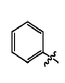 may be expressed as 20% when the total number of substituents that the phenyl group may have is 5 (T1 in Equation) and the number of deuteriums among them is 1 (T2 in Equation). That is, it may be represented by the structural formula below that the content of deuterium in the phenyl group is 20%.

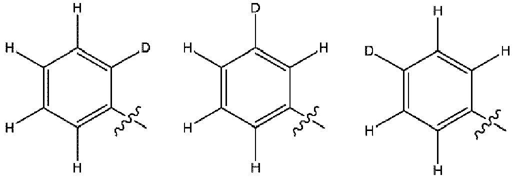

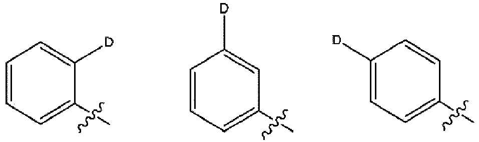

Further, in an embodiment of the present application, in the case of "a phenyl group having a deuterium content of 0%", it may mean a phenyl group that does not contain a deuterium atom, that is, has 5 hydrogen atoms.

In an embodiment of the present application, there is provided a heterocyclic compound represented by Chemical Formula 1 above.

In an embodiment of the present application, the heterocyclic compound represented by Chemical Formula 1 above may be substituted or unsubstituted again with deuterium, and the content of deuterium may be 0 to 100%, preferably 20 to 100%, and more preferably 40 to 100%.

In an embodiment of the present application, R1 and R2 may be the same as or different from each other, and may be each independently hydrogen; deuterium; a substituted or unsubstituted C₁-C₆₀ alkyl group; a substituted or unsubstituted C₆-C₆₀ aryl group; or a substituted or unsubstituted C₂-C₆₀ heteroaryl group.

In another embodiment, R1 and R2 may be the same as or different from each other, and may be each independently hydrogen; deuterium; a substituted or unsubstituted C₁-C₄₀ alkyl group; a substituted or unsubstituted C₆-C₄₀ aryl group; or a substituted or unsubstituted C₂-C₄₀ heteroaryl group.

In another embodiment, R1 and R2 may be the same as or different from each other, and may be each independently a substituted or unsubstituted C₆-C₄₀ aryl group.

In another embodiment, R1 and R2 may be the same as or different from each other, and may be each independently a C₆-C₄₀ aryl group.

In another embodiment, R1 and R2 may be the same as or different from each other, and may be each independently a C₆-C₂₀ aryl group.

In another embodiment, R1 and R2 may be the same as or different from each other, and may be each independently a C₆-C₂₀ monocyclic aryl group; or a C₁₀-C₂₀ polycyclic aryl group.

In another embodiment, R1 and R2 may be the same as or different from each other, and may be each independently a phenyl group; a biphenyl group; or a naphthyl group.

In an embodiment of the present application, R1 and R2 may be substituted or unsubstituted again with deuterium, and at this time, in R1 and R2, the content of deuterium may be 0 to 100%, preferably 20 to 100%, and more preferably 40 to 100%.

In an embodiment of the present application, X1 to X3 may be the same as or different from each other and each independently CRx; or N, and at least two may be N.

In an embodiment of the present application, X1 to X3 may be N.

In an embodiment of the present application, X1 and X2 may be N, and X3 may be CRx.

In an embodiment of the present application, X1 and X3 may be N, and X2 may be CRx.

In an embodiment of the present application, X2 and X3 may be N, and X1 may be CRx.

In an embodiment of the present application, Rx may be hydrogen; deuterium; a substituted or unsubstituted C₆-C₆₀ aryl group; or a substituted or unsubstituted C₂-C₆₀ heteroaryl group.

In another embodiment, Rx may be hydrogen; deuterium; a substituted or unsubstituted C₆-C₄₀ aryl group; or a substituted or unsubstituted C₂-C₄₀ heteroaryl group.

In another embodiment, Rx may be hydrogen; deuterium; a substituted or unsubstituted C₆-C₂₀ aryl group; or a substituted or unsubstituted C₂-C₂₀ heteroaryl group.

In another embodiment, Rx may be hydrogen; or deuterium.

In another embodiment, Rx may be hydrogen.

In an embodiment of the present application, L1 may be a direct bond; a substituted or unsubstituted C₆-C₆₀ arylene group; or a substituted or unsubstituted C₂-C₆₀ heteroarylene group.

In another embodiment, L1 may be a direct bond; or a substituted or unsubstituted C₆-C₆₀ arylene group.

In another embodiment, L1 may be a direct bond; or a substituted or unsubstituted C₆-C₄₀ arylene group.

In another embodiment, L1 may be a direct bond; or a substituted or unsubstituted C₆-C₂₀ arylene group.

In another embodiment, L1 may be a direct bond; or a C₆-C₂₀ arylene group.

In another embodiment, L1 may be a direct bond; or a phenylene group.

In an embodiment of the present application, L1 may be substituted or unsubstituted again with deuterium, and the content of deuterium may be 0 to 100%, preferably 20 to 100%, and more preferably 40 to 100%.

In an embodiment of the present application, Chemical Formula 1 above may be represented by any one of Chemical Formulae 3 to 6 below. 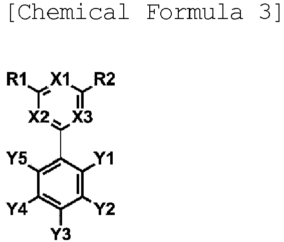 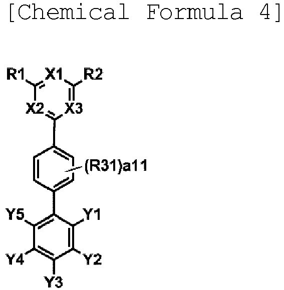 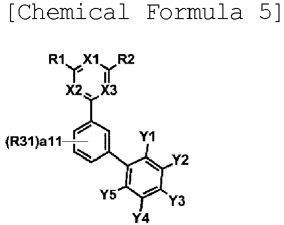 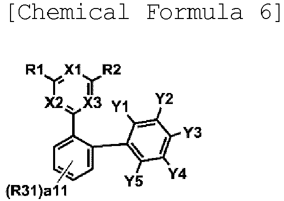
in Chemical Formulae 3 to 6,
the definition of each substituent is the same as that in Chemical Formula 1 above,
R31 is hydrogen; or deuterium, and
a11 is an integer of 0 to 4, and when a11 is 2 or more, the substituents in parentheses are the same as or different from each other.

In an embodiment of the present application, Chemical Formula B above may be represented by any one of Chemical Formulae B-1 to B-4 below. 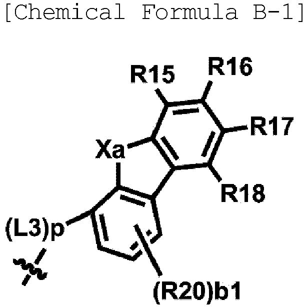 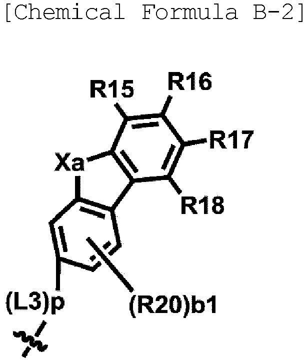 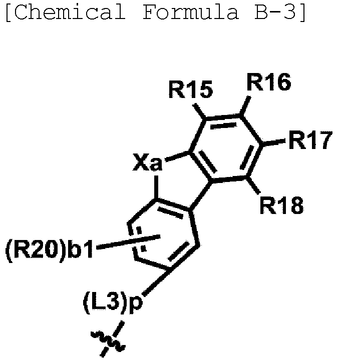 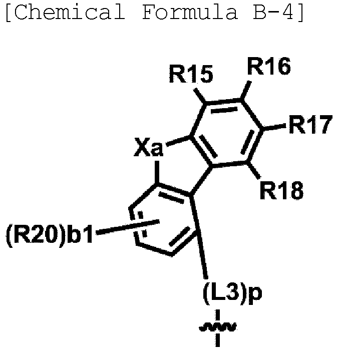
in Chemical Formulae B-1 to B-4,
the definition of each substituent is the same as the definition in Chemical Formula B above.

In an embodiment of the present application, two of Y1 to Y5 are represented by Chemical Formula B above, and when Xa is O; or S, the two Chemical Formulae B above are different from each other.

At this time, the fact that the two Chemical Formulae B above are different from each other means that the types of substituents or the positions of the substituents are different from each other, for example, it is the case that, even if Xa, R15 to R18, L3, and p of Chemical Formulae B-1 and B-2 above are all the same, the positions of the substituents are different from each other, and it can also be expressed that the two Chemical Formulae B above are different from each other.

In an embodiment of the present application, when two of Y1 to Y5 are represented by Chemical Formula B above, and Chemical Formula B above is substituted with Y1, the remaining Chemical Formula B above may be substituted with Y2.

In an embodiment of the present application, when two of Y1 to Y5 are represented by Chemical Formula B above, and Chemical Formula B above is substituted with Y1, the remaining Chemical Formula B above may be substituted with Y3.

In an embodiment of the present application, when two of Y1 to Y5 are represented by Chemical Formula B above, and Chemical Formula B above is substituted with Y1, the remaining Chemical Formula B above may be substituted with Y4.

In an embodiment of the present application, when two of Y1 to Y5 are represented by Chemical Formula B above, and Chemical Formula B above is substituted with Y1, the remaining Chemical Formula B above may be substituted with Y5.

In an embodiment of the present application, when two of Y1 to Y5 are represented by Chemical Formula B above, and Chemical Formula B above is substituted with Y2, the remaining Chemical Formula B above may be substituted with Y3.

In an embodiment of the present application, when two of Y1 to Y5 are represented by Chemical Formula B above, and Chemical Formula B above is substituted with Y2, the remaining Chemical Formula B above may be substituted with Y4.

In an embodiment of the present application, when two of Y1 to Y5 are represented by Chemical Formula B above, and Chemical Formula B above is substituted with Y2, the remaining Chemical Formula B above may be substituted with Y5.

In an embodiment of the present application, when two of Y1 to Y5 are represented by Chemical Formula A above, and Chemical Formula A above is substituted with Y1, the remaining Chemical Formula A above may be substituted with Y2.

In an embodiment of the present application, when two of Y1 to Y5 are represented by Chemical Formula A above, and Chemical Formula A above is substituted with Y1, the remaining Chemical Formula A above may be substituted with Y3.

In an embodiment of the present application, when two of Y1 to Y5 are represented by Chemical Formula A above, and Chemical Formula A above is substituted with Y1, the remaining Chemical Formula A above may be substituted with Y4.

In an embodiment of the present application, when two of Y1 to Y5 are represented by Chemical Formula A above, and Chemical Formula A above is substituted with Y1, the remaining Chemical Formula A above may be substituted with Y5.

In an embodiment of the present application, when two of Y1 to Y5 are represented by Chemical Formula A above, and Chemical Formula A above is substituted with Y2, the remaining Chemical Formula A above may be substituted with Y3.

In an embodiment of the present application, when two of Y1 to Y5 are represented by Chemical Formula A above, and Chemical Formula A above is substituted with Y2, the remaining Chemical Formula A above may be substituted with Y5.

In an embodiment of the present application, when two of Y1 to Y5 are represented by Chemical Formulae A and B above, and one of Chemical Formulae A and B above is substituted with Y1, the rest may be substituted with Y2.

In an embodiment of the present application, when two of Y1 to Y5 are represented by Chemical Formulae A and B above, and one of Chemical Formulae A and B above is substituted with Y1, the rest may be substituted with Y3.

In an embodiment of the present application, when two of Y1 to Y5 are represented by Chemical Formulae A and B above, and one of Chemical Formulae A and B above is substituted with Y1, the rest may be substituted with Y4.

In an embodiment of the present application, when two of Y1 to Y5 are represented by Chemical Formulae A and B above, and one of Chemical Formulae A and B above is substituted with Y1, the rest may be substituted with Y5.

In an embodiment of the present application, when two of Y1 to Y5 are represented by Chemical Formulae A and B above, and one of Chemical Formulae A and B above is substituted with Y2, the rest may be substituted with Y3.

In an embodiment of the present application, when two of Y1 to Y5 are represented by Chemical Formulae A and B above, and one of Chemical Formulae A and B above is substituted with Y2, the rest may be substituted with Y4.

In an embodiment of the present application, when two of Y1 to Y5 are represented by Chemical Formulae A and B above, and one of Chemical Formulae A and B above is substituted with Y2, the rest may be substituted with Y5.

In an embodiment of the present application, L2 and L3 may be the same as or different from each other, and may be each independently a direct bond; a substituted or unsubstituted C₆-C₆₀ arylene group; or a substituted or unsubstituted C₂-C₆₀ heteroarylene group.

In another embodiment, L2 and L3 may be the same as or different from each other, and may be each independently a direct bond; a substituted or unsubstituted C₆-C₄₀ arylene group; or a substituted or unsubstituted C₂-C₄₀ heteroarylene group.

In another embodiment, L2 and L3 may be the same as or different from each other, and may be each independently a direct bond; or a substituted or unsubstituted C₆-C₄₀ arylene group.

In another embodiment, L2 and L3 may be the same as or different from each other, and may be each independently a direct bond; or a substituted or unsubstituted C₆-C₂₀ arylene group.

In another embodiment, L2 and L3 may be the same as or different from each other, and may be each independently a direct bond; or a C₆-C₂₀ arylene group.

In another embodiment, L2 and L3 may be the same as or different from each other, and may be each independently a direct bond; or a phenylene group.

In an embodiment of the present application, L2 and L3 may be substituted or unsubstituted again with deuterium, and the content of deuterium may be 0 to 100%, preferably 20 to 100%, and more preferably 40 to 100%.

In an embodiment of the present application, R11 to R20 may be the same as or different from each other and each independently hydrogen; or deuterium, or two or more groups adjacent to each other may be bonded to each other to form a substituted or unsubstituted C₆-C₆₀ aromatic hydrocarbon ring or a substituted or unsubstituted C₂-C₆₀ heteroring.

In another embodiment, R11 to R20 may be the same as or different from each other and each independently hydrogen; or deuterium, or two or more groups adjacent to each other may be bonded to each other to form a substituted or unsubstituted C₆-C₄₀ aromatic hydrocarbon ring or a substituted or unsubstituted C₂-C₄₀ heteroring.

In another embodiment, R11 to R20 may be the same as or different from each other and each independently hydrogen; or deuterium, or two or more groups adjacent to each other may be bonded to each other to form a C₆-C₄₀ aromatic hydrocarbon ring which is substituted or unsubstituted with a C₁-C₂₀ alkyl group, or a C₂-C₄₀ heteroring.

In another embodiment, R11 to R20 may be the same as or different from each other and each independently hydrogen; or deuterium, or two or more groups adjacent to each other may be bonded to each other to form a benzofuran ring; a benzothiophene ring; or an indene ring substituted or unsubstituted with a methyl group.

In an embodiment of the present application, when Xa of Chemical Formula B is O; or S, R15 to R20 of Chemical Formula B above may be hydrogen.

In an embodiment of the present application, Chemical Formula A above may be represented by Chemical Formula A-1-1 or A-1-2 below. 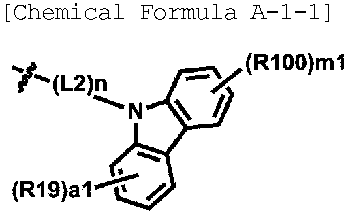 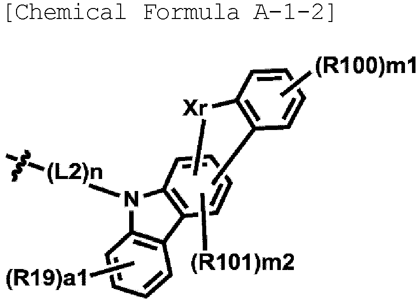
in Chemical Formulae A-1-1 and A-1-2,
the definitions of L2, R19, a1, and n are the same as those in Chemical Formula A above,
Xr is O; S; CRR'; or NR,
R100 and R101 are the same as or different from each other, and are each independently hydrogen; or deuterium,
m1 is an integer of 0 to 4, m2 is an integer of 0 to 2,
when m1 is an integer of 2 or more, or m2 is 2, the substituents in parentheses are the same as or different from each other, and
R and R' are the same as or different from each other, and are each independently a substituted or unsubstituted C₁-C₆₀ alkyl group; or a substituted or unsubstituted C₆-C₆₀ aryl group.

In an embodiment of the present application, Chemical Formula B above may be represented by Chemical Formula B-1-1 or B-1-2 below. 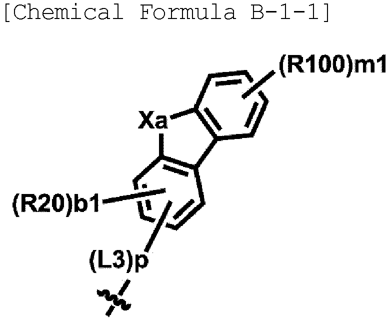 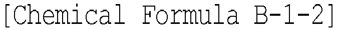 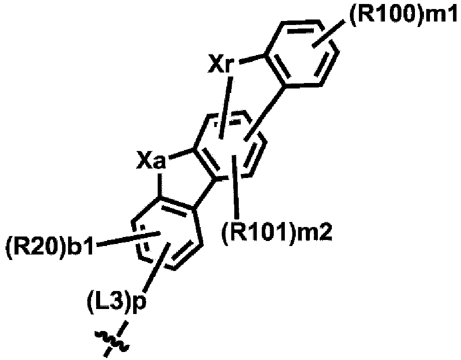
in Chemical Formulae B-1-1 and B-1-2,
the definitions of L3, Xa, R20, b1, and p are the same as those in Chemical Formula B above,
Xr is O; S; CRR'; or NR,
R and R' are the same as or different from each other, and are each independently a substituted or unsubstituted C₁-C₆₀ alkyl group; or a substituted or unsubstituted C₆-C₆₀ aryl group,
R100 and R101 are the same as or different from each other, and are each independently hydrogen; or deuterium,
m1 is an integer of 0 to 4, m2 is an integer of 0 to 2, and
when m1 is an integer of 2 or more, or m2 is 2, the substituents in parentheses are the same as or different from each other.
R and R' are the same as or different from each other, and are each independently a substituted or unsubstituted C₁-C₆₀ alkyl group; or a substituted or unsubstituted C₆-C₆₀ aryl group.

In an embodiment of the present application, Chemical Formula A-1-2 above may be represented by any one selected from structural formulae below.

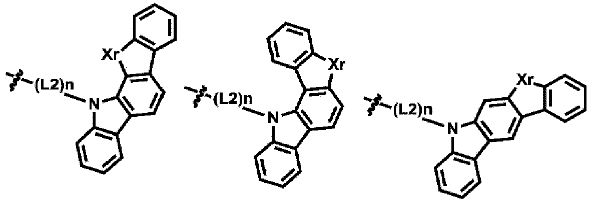

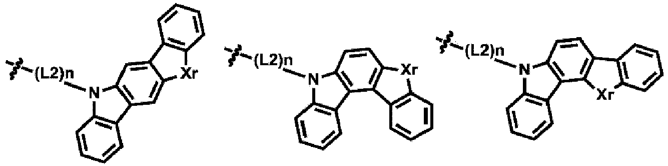

In the structural formulae,
the definitions of Xr, L2, and n are the same as those in Chemical Formula A-1-2 above.

In an embodiment of the present application, Chemical Formula B-1-2 above may be represented by any one selected from structural formulae below.

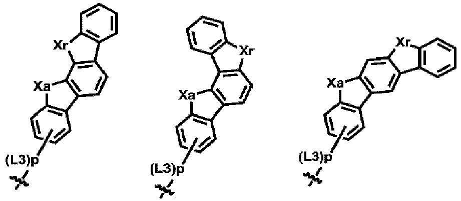

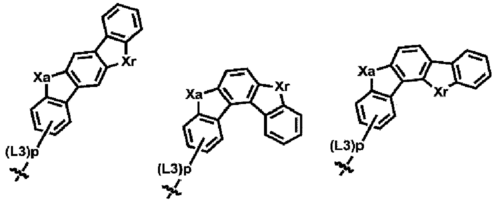

In the structural formulae,
the definitions of Xr, L3, and p are the same as those in Chemical Formula A-1-2 above.

In an embodiment of the present application, Ry may be a substituted or unsubstituted C₆-C₆₀ aryl group; or a substituted or unsubstituted C₂-C₆₀ heteroaryl group.

In another embodiment, Ry may be a substituted or unsubstituted C₆-C₄₀ aryl group; or a substituted or unsubstituted C₂-C₄₀ heteroaryl group.

In another embodiment, Ry may be a substituted or unsubstituted C₆-C₄₀ aryl group.

In another embodiment, Ry may be a C₆-C₄₀ aryl group.

In another embodiment, Ry may be a C₆-C₂₀ aryl group.

In another embodiment, Ry may be a C₆-C₁₀ aryl group.

In another embodiment, Ry may be a phenyl group.

In an embodiment of the present application, R and R' are the same as or different from each other, and are each independently a substituted or unsubstituted C₁-C₆₀ alkyl group; or a substituted or unsubstituted C₆-C₆₀ aryl group.

In another embodiment, R and R' are the same as or different from each other, and are each independently a substituted or unsubstituted C₁-C₄₀ alkyl group; or a substituted or unsubstituted C₆-C₄₀ aryl group.

In another embodiment, R and R' are the same as or different from each other, and are each independently a substituted or unsubstituted C₁-C₄₀ alkyl group.

In another embodiment, R and R' are the same as or different from each other, and are each independently a C₁-C₄₀ alkyl group.

In another embodiment, R and R' are the same as or different from each other, and are each independently a C₁-C₂₀ alkyl group.

In another embodiment, R and R' are the same as or different from each other, and are each independently a linear C₁-C₂₀ alkyl group.

In another embodiment, R and R' are the same as or different from each other, and are each independently a methyl group.

In an embodiment of the present application, there is provided a heterocyclic compound in which Chemical Formula 1 above is represented by any one of compounds below.

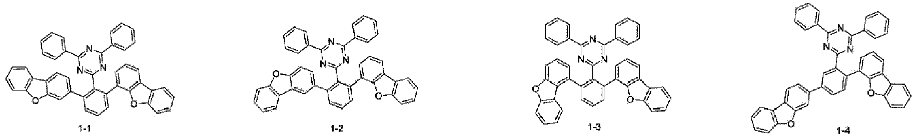

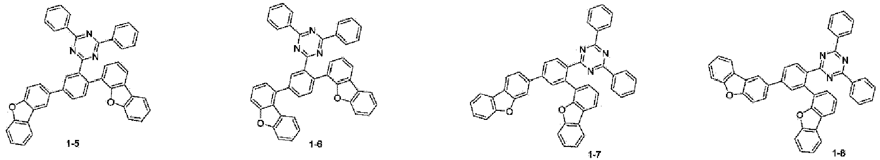

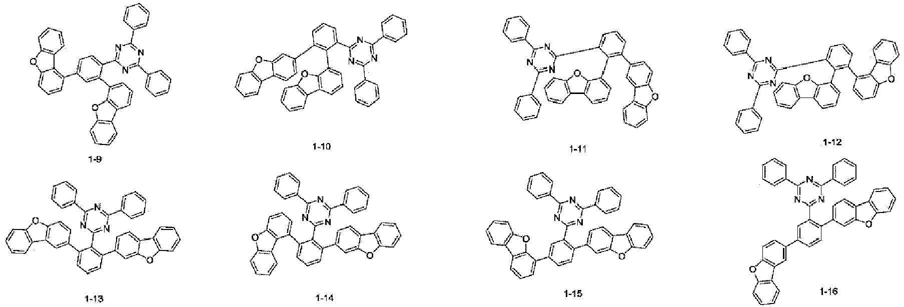

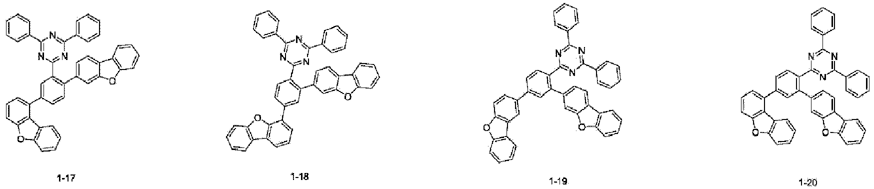

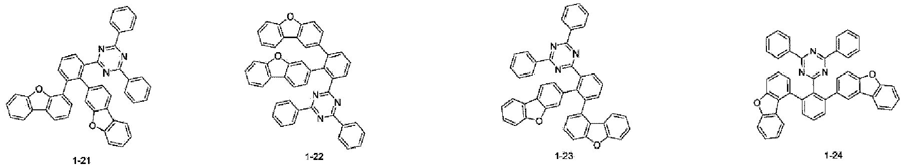

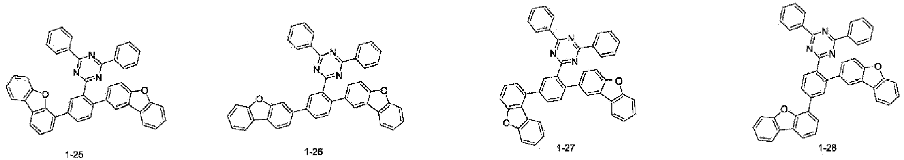

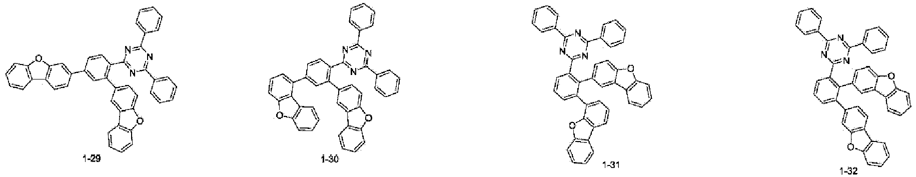

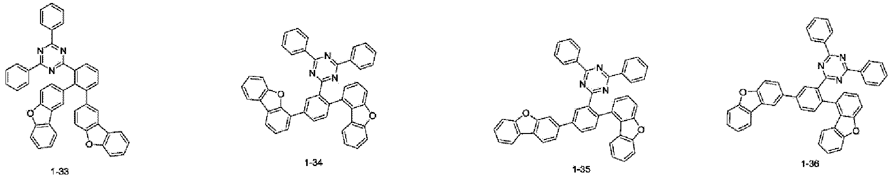

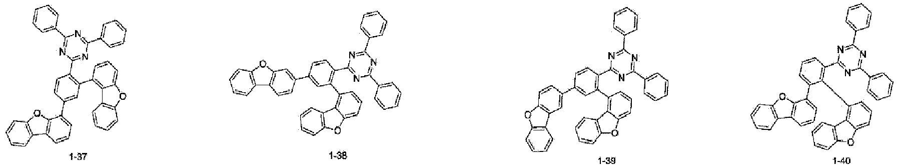

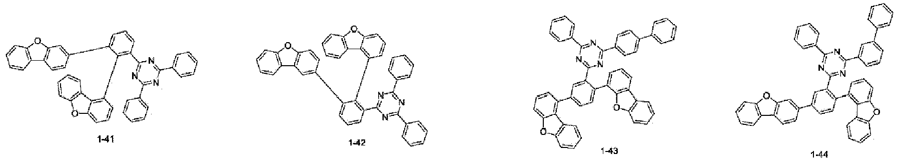

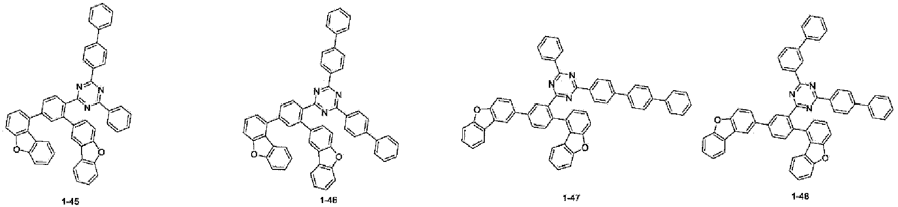

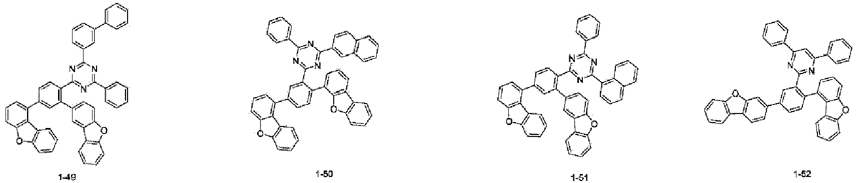

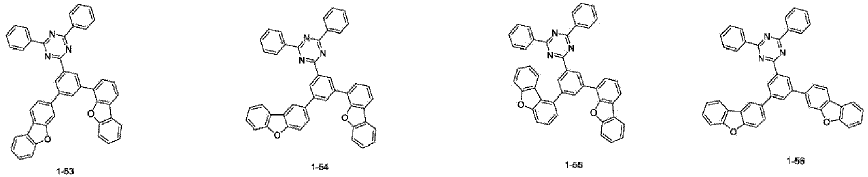

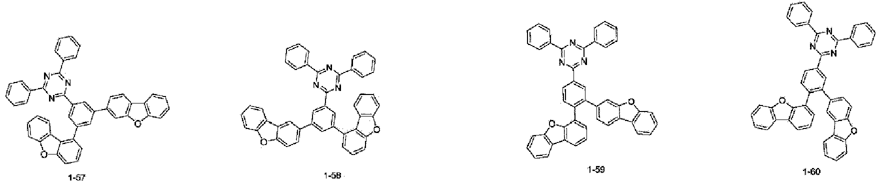

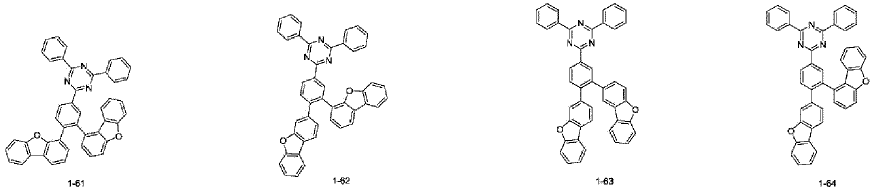

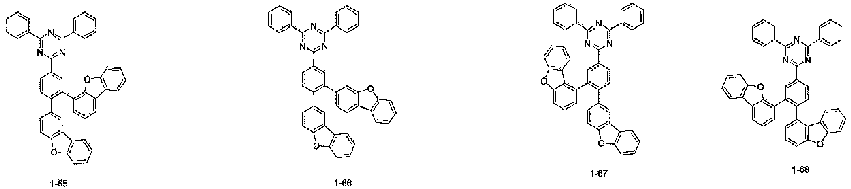

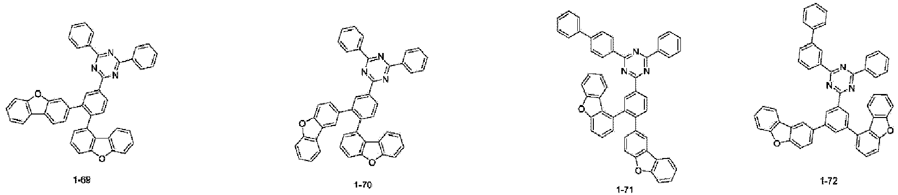

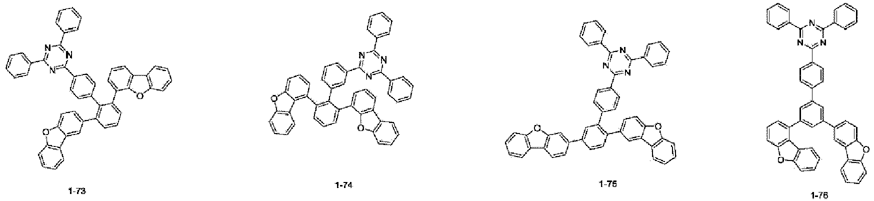

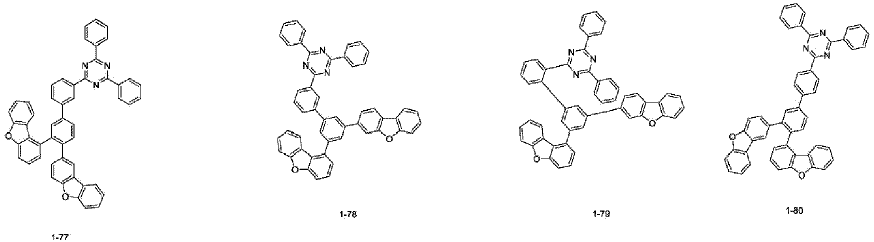

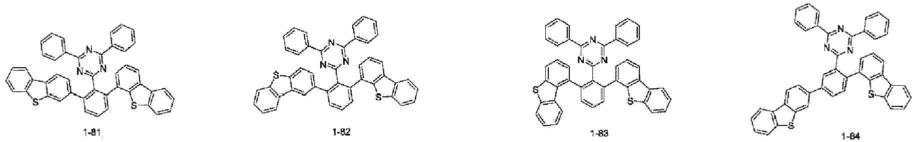

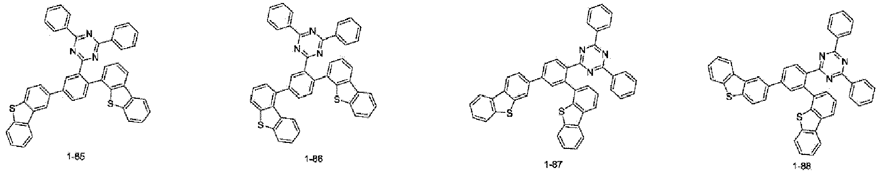

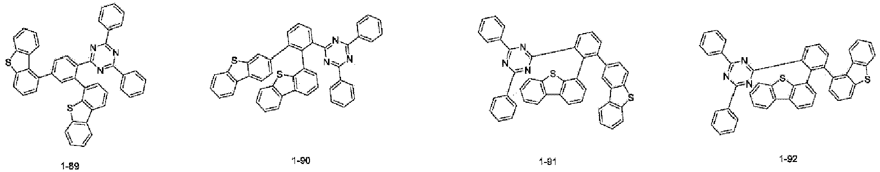

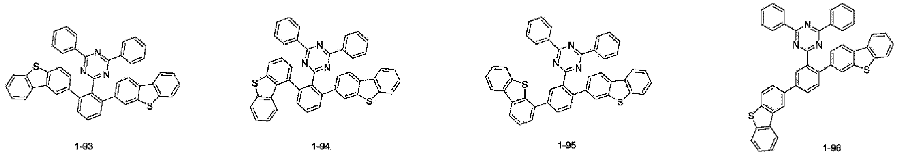

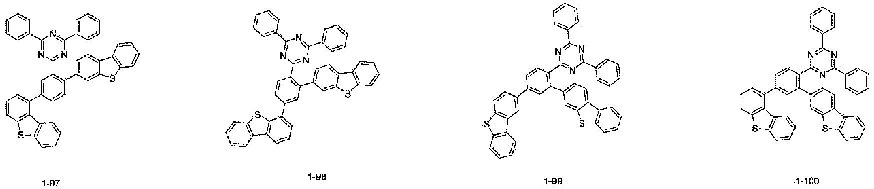

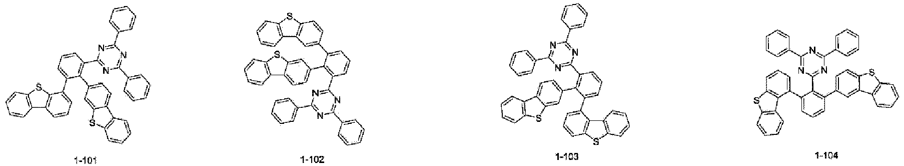

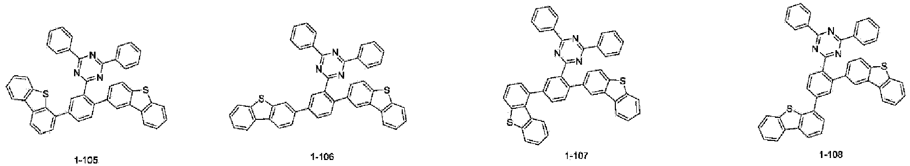

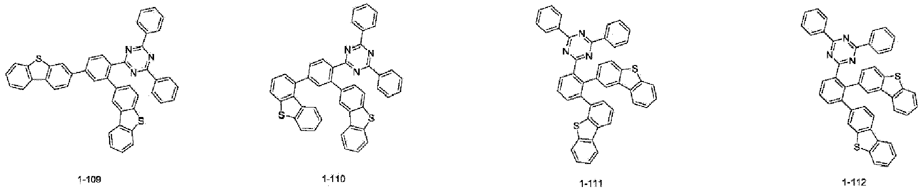

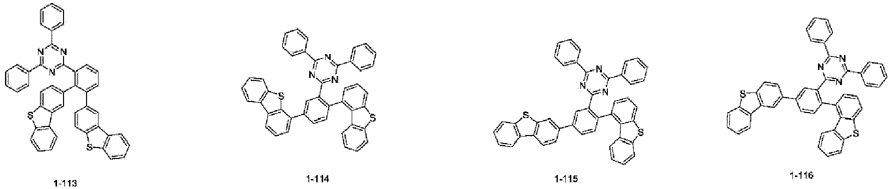

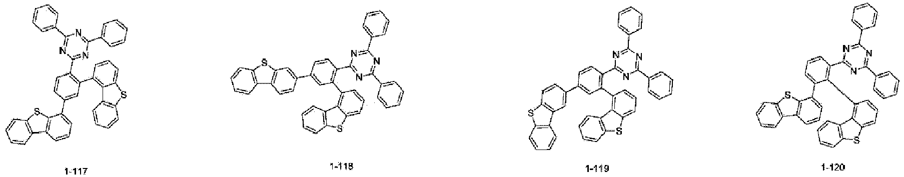

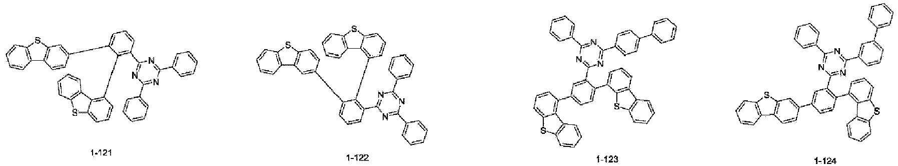

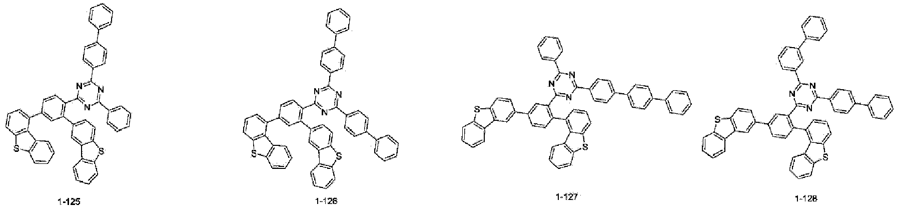

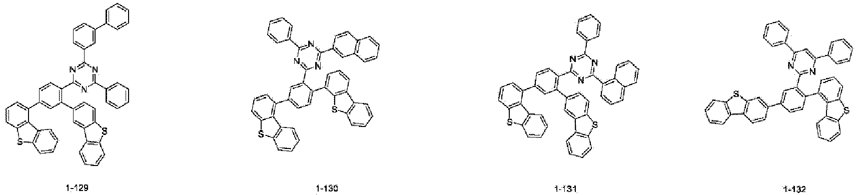

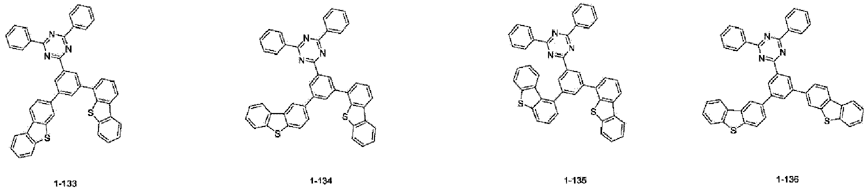

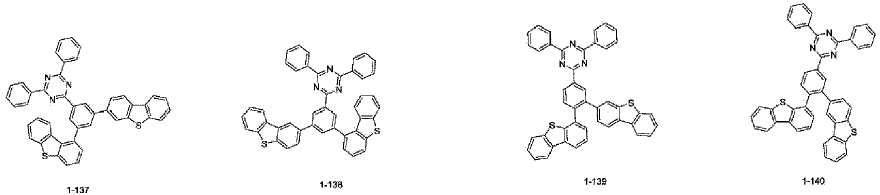

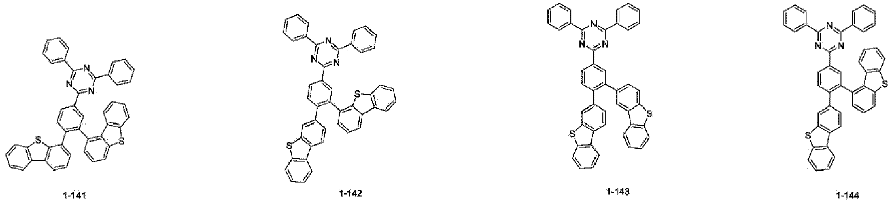

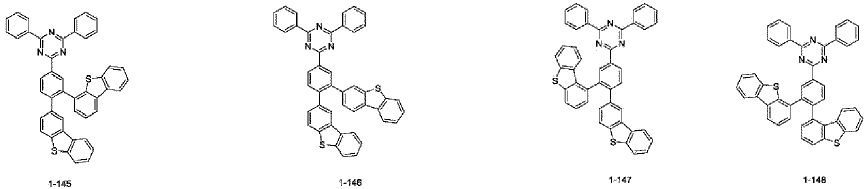

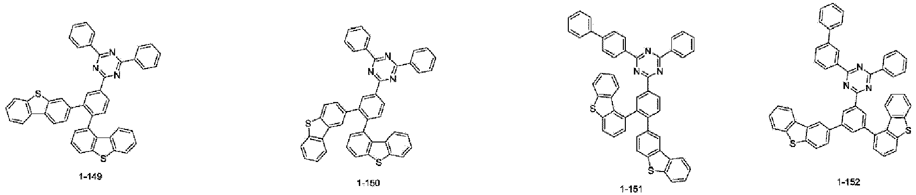

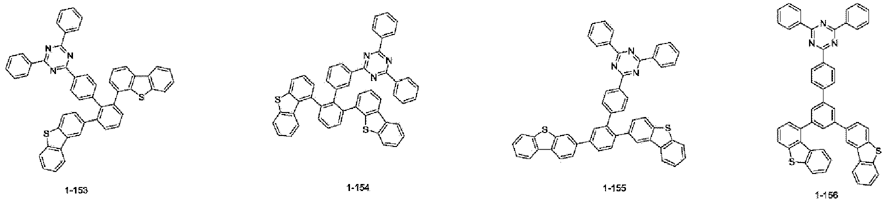

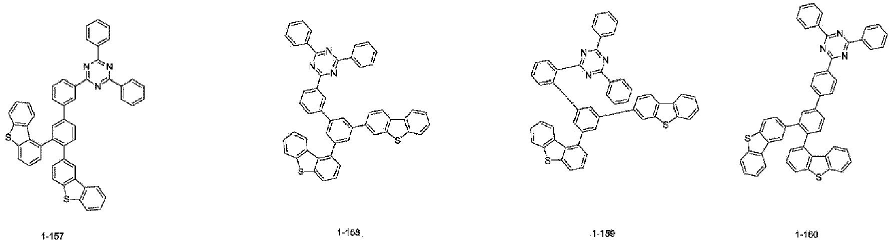

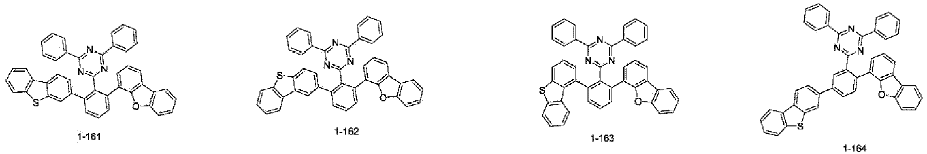

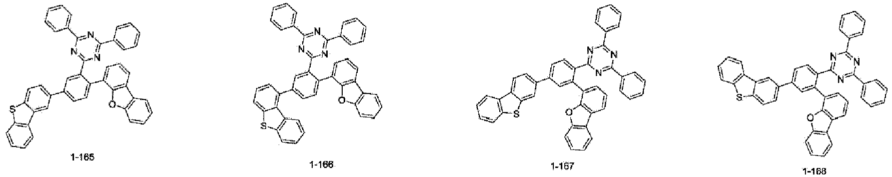

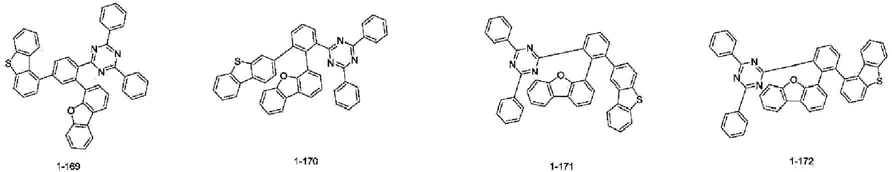

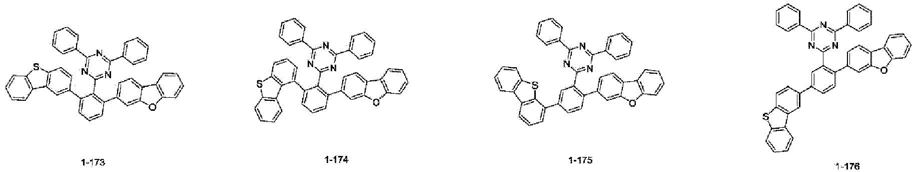

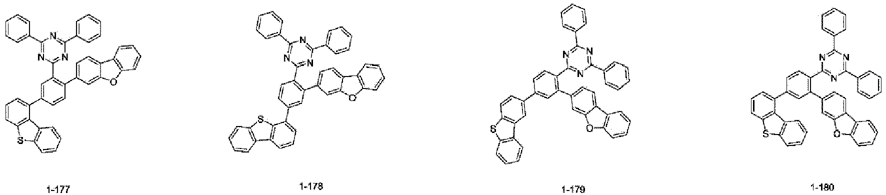

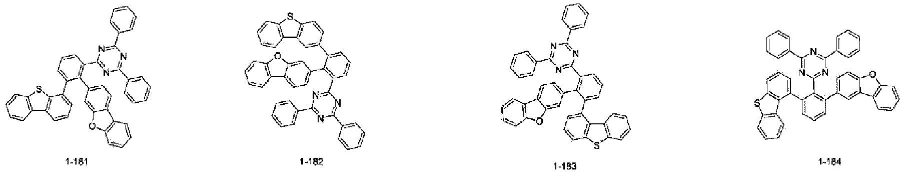

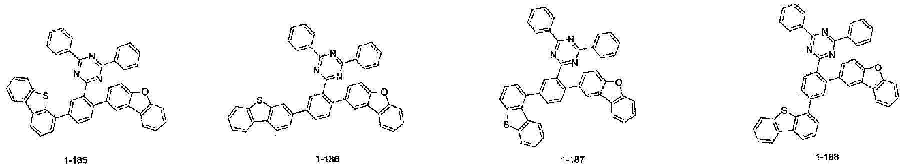

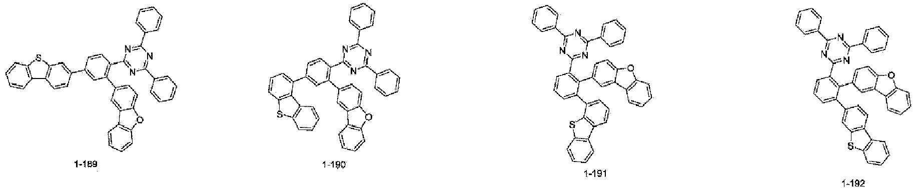

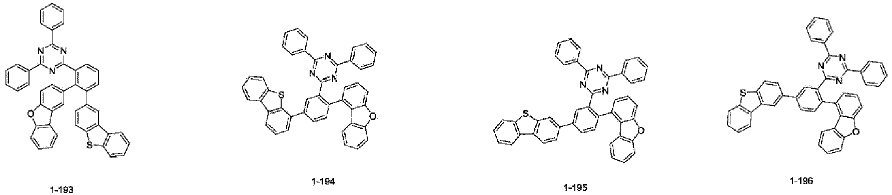

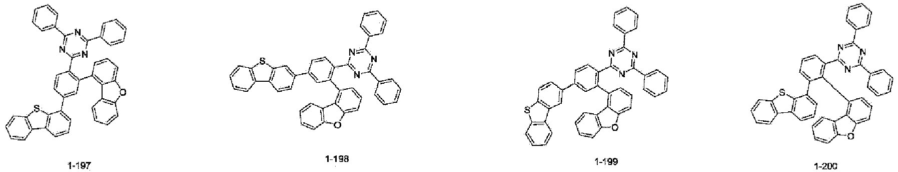

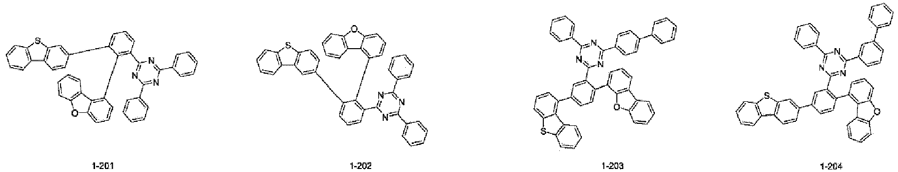

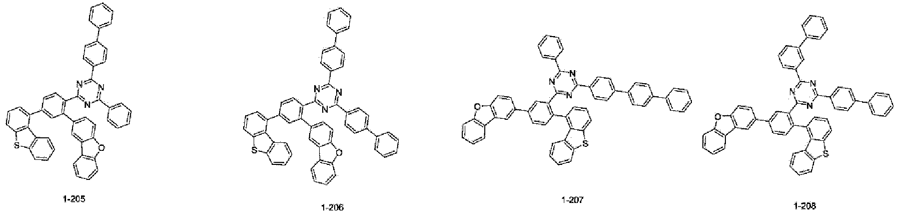

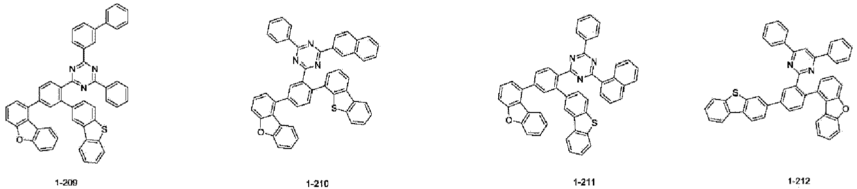

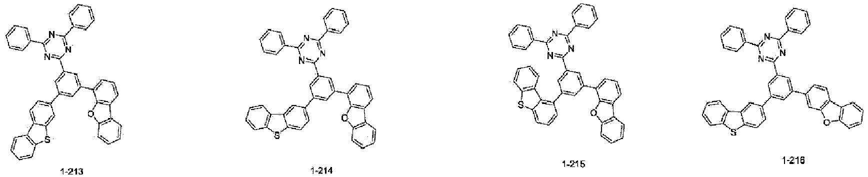

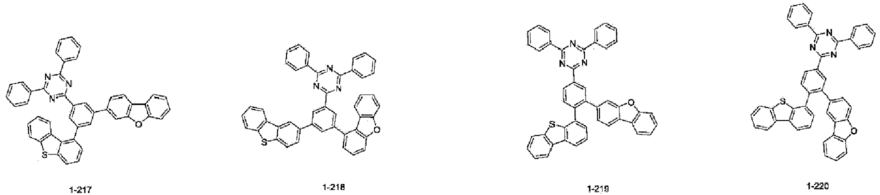

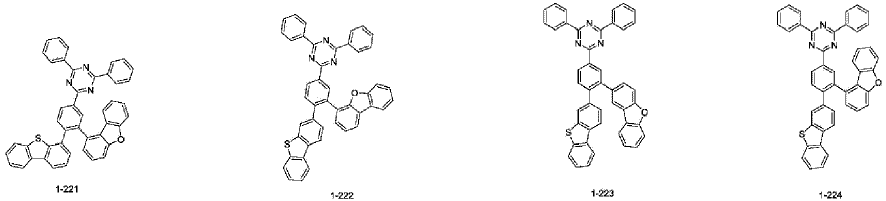

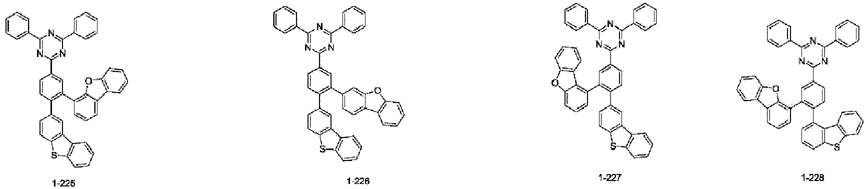

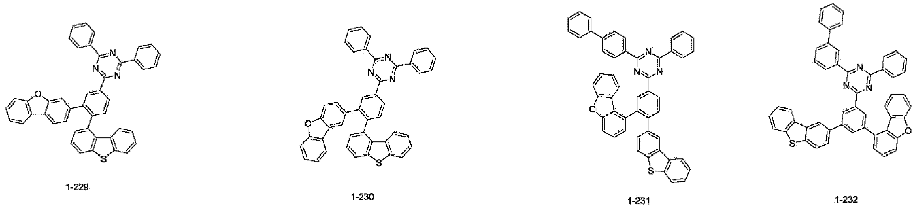

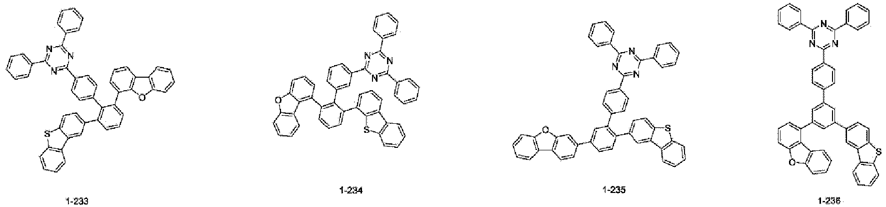

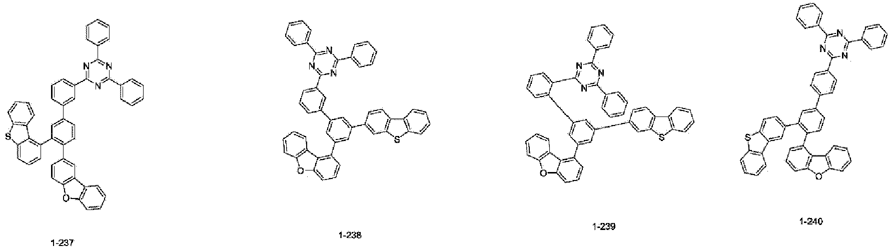

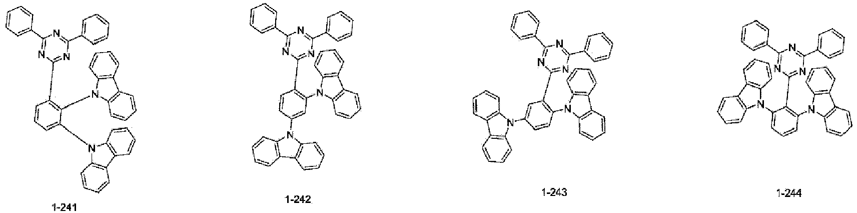

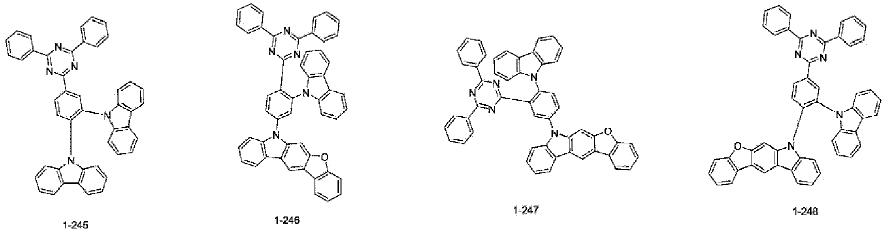

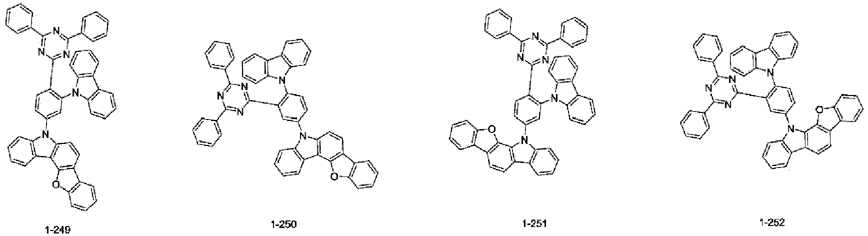

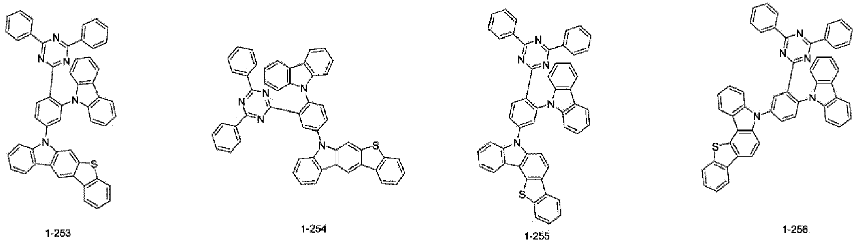

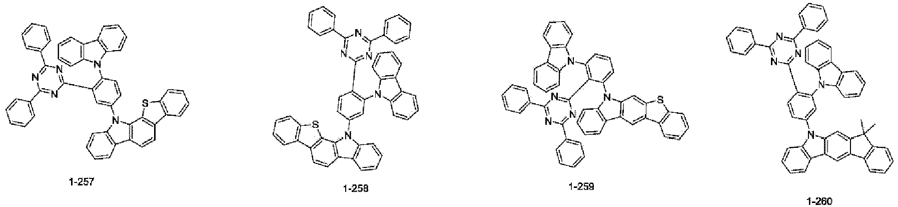

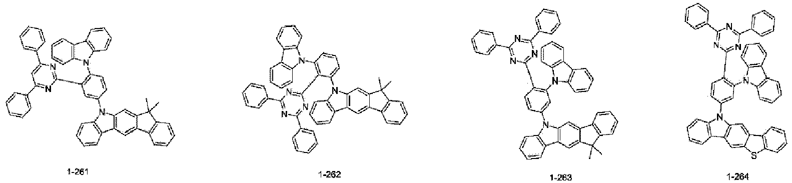

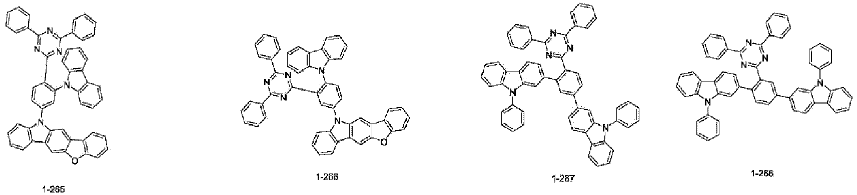

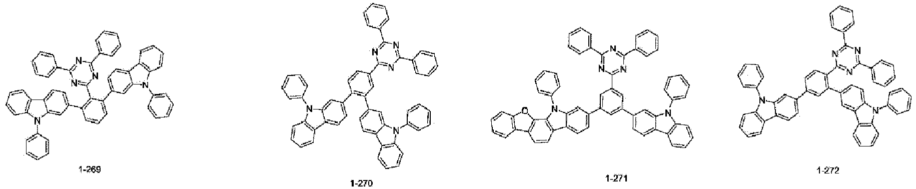

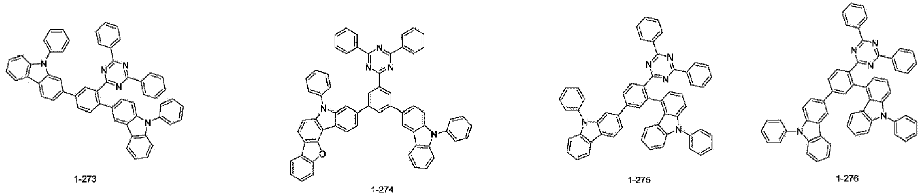

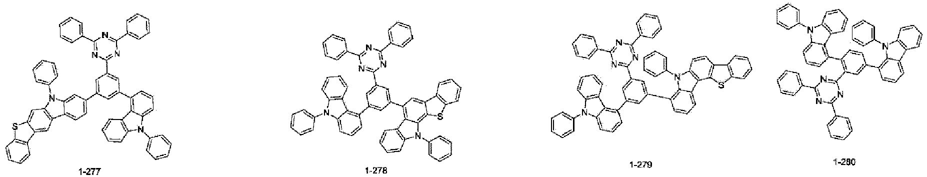

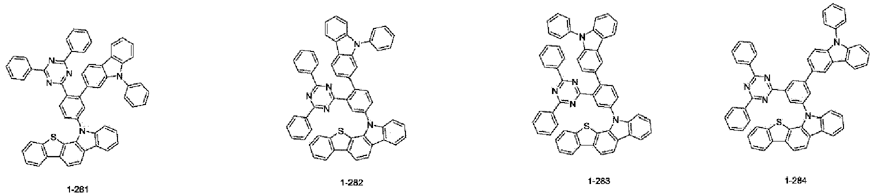

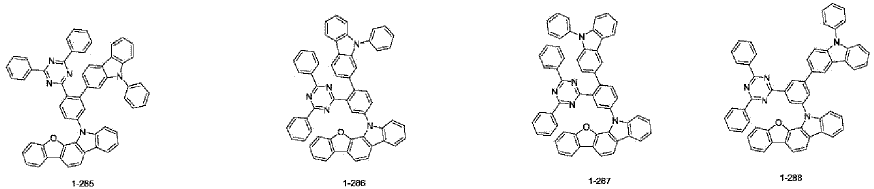

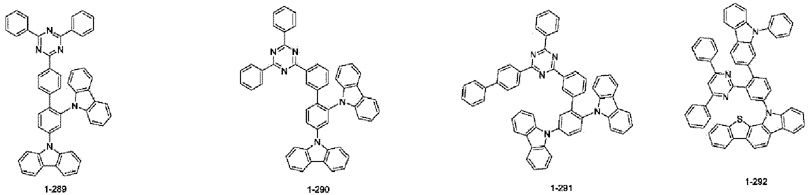

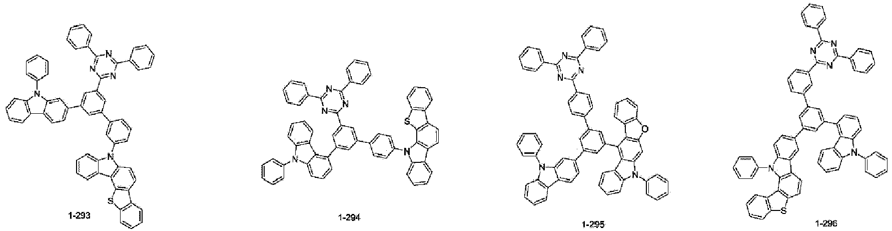

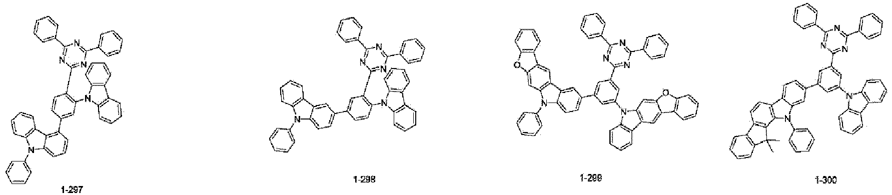

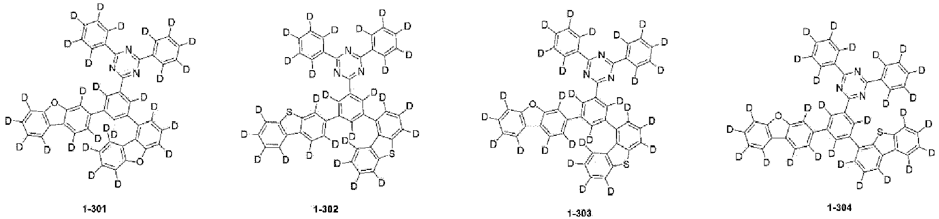

Further, compounds having intrinsic properties of the introduced substituents may be synthesized by introducing various substituents into the structure of Chemical Formula 1 above. For example, substances satisfying the conditions required for each organic material layer may be synthesized by introducing substituents mainly used for a hole injection layer material, a hole transport layer material, a light emitting layer material, an electron transport layer material, and a charge generation layer material used when manufacturing an organic light emitting device into the core structure.

Further, it is possible to finely control the energy band gap by introducing various substituents into the structure of Chemical Formula 1 above, whereas improving the properties at the interface between organic materials and enabling the use of the substances to be diversified.

Meanwhile, the compound is excellent in thermal stability by having a high glass transition temperature (Tg). Such an increase in thermal stability becomes an important factor in providing driving stability to the device.

Further, in an embodiment of the present application, there is provided an organic light emitting device comprising: a first electrode; a second electrode provided opposite to the first electrode; and one or more organic material layers provided between the first electrode and the second electrode, wherein one or more layers of the organic material layers comprise the heterocyclic compound represented by Chemical Formula 1 above.

In an embodiment of the present application, the first electrode may be an anode, and the second electrode may be a cathode.

In another embodiment, the first electrode may be a cathode, and the second electrode may be an anode.

In an embodiment of the present application, the organic light emitting device may be a blue organic light emitting device, and the heterocyclic compound according to Chemical Formula 1 above may be used as a material of the blue organic light emitting device.

In an embodiment of the present application, the organic light emitting device may be a green organic light emitting device, and the heterocyclic compound represented by Chemical Formula 1 above may be used as a material of the green organic light emitting device.

In an embodiment of the present application, the organic light emitting device may be a red organic light emitting device, and the heterocyclic compound represented by Chemical Formula 1 above may be used as a material of the red organic light emitting device.

In an embodiment of the present application, the organic light emitting device may be a blue organic light emitting device, and the heterocyclic compound according to Chemical Formula 1 above may be used as a material for the light emitting layer of the blue organic light emitting device.

In an embodiment of the present application, the organic light emitting device may be a green organic light emitting device, and the heterocyclic compound represented by Chemical Formula 1 above may be used as a material for the light emitting layer of the green organic light emitting device.

In an embodiment of the present application, the organic light emitting device may be a red organic light emitting device, and the heterocyclic compound represented by Chemical Formula 1 above may be used as a material for the light emitting layer of the red organic light emitting device.

Specific details of the heterocyclic compound represented by Chemical Formula 1 above are the same as described above.

The organic light emitting device of the present disclosure may be manufactured by a conventional method and material for manufacturing an organic light emitting device except that one or more organic material layers are formed using the above-described heterocyclic compound.

The heterocyclic compound may be formed into an organic material layer by a solution application method as well as a vacuum deposition method when manufacturing an organic light emitting device. Here, the solution application method refers to spin coating, dip coating, inkjet printing, screen printing, spraying, roll coating, etc., but the present application is not limited thereto.

The organic material layer of the organic light emitting device of the present disclosure may be formed in a single layer structure, but may be formed in a multilayer structure in which an organic material layer with two or more layers is laminated. For example, the organic light emitting device of the present disclosure may have a structure comprising a hole injection layer, a hole transport layer, a light emitting layer, an electron transport layer, an electron injection layer, etc. as an organic material layer. However, the structure of the organic light emitting device is not limited thereto and may comprise an organic material layer with a smaller number of layers.

In the organic light emitting device of the present disclosure, the organic material layer may comprise a light emitting layer, and the light emitting layer may comprise the heterocyclic compound of Chemical Formula 1 above.

In the organic light emitting device of the present disclosure, the organic material layer may comprise a light emitting layer, and the light emitting layer may comprise the heterocyclic compound of Chemical Formula 1 above as a light emitting layer host.

In an organic light emitting device according to an embodiment of the present application, there is provided an organic light emitting device in which the organic material layer comprising the heterocyclic compound represented by Chemical Formula 1 above further comprises a heterocyclic compound represented by Chemical Formula 2 below. 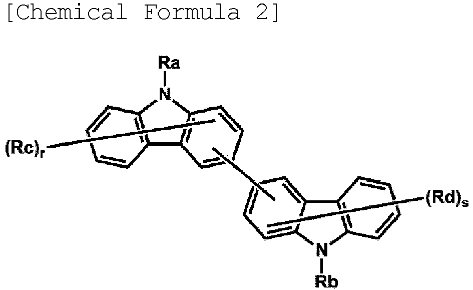
in Chemical Formula 2,
Rc and Rd are the same as or different from each other, and are each independently selected from the group consisting of: hydrogen; deuterium; a halogen group; -CN; a substituted or unsubstituted C₁-C₆₀ alkyl group; a substituted or unsubstituted C₂-C₆₀ alkenyl group; a substituted or unsubstituted C₂-C₆₀ alkynyl group; a substituted or unsubstituted C₁-C₆₀ alkoxy group; a substituted or unsubstituted C₃-C₆₀ cycloalkyl group; a substituted or unsubstituted C₂-C₆₀ heterocycloalkyl group; a substituted or unsubstituted C₆-C₆₀ aryl group; a substituted or unsubstituted C₂-C₆₀ heteroaryl group; -SiR101R102R103; - P(=O)R101R102; and -NR101R102, or two or more groups adjacent to each other are bonded to each other to form a substituted or unsubstituted C₆-C₆₀ aromatic hydrocarbon ring or a substituted or unsubstituted C₂-C₆₀ heteroring,
Ra and Rb are the same as or different from each other, and are each independently a substituted or unsubstituted C₆-C₆₀ aryl group; a substituted or unsubstituted C₂-C₆₀ heteroaryl group; -SiR101R102R103; or -P(=0)R101R102,
R101, R102, and R103 are the same as or different from each other, and are each independently hydrogen; deuterium; -CN; a substituted or unsubstituted C₁-C₆₀ alkyl group; a substituted or unsubstituted C₆-C₆₀ aryl group; or a substituted or unsubstituted C₂-C₆₀ heteroaryl group, and
r and s are integers of 0 to 7, and when r and s are each 2 or more, the substituents in parentheses are the same as or different from each other.

In an embodiment of the present application, Chemical Formula 2 above may be represented by Chemical Formula 2-1 below. 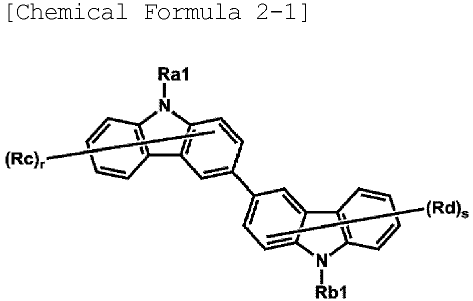
in Chemical Formula 2-1,
the definitions of Rc, Rd, r, and s are the same as those in Chemical Formula 2 above,
Ra1 and Rb1 are the same as or different from each other, and are each independently a substituted or unsubstituted C₆-C₆₀ aryl group.

In an embodiment of the present application, Rc and Rd of Chemical Formula 2 above may be hydrogen; or deuterium.

In an embodiment of the present application, Ra1 and Rb1 of Chemical Formula 2-1 above may be the same as or different from each other, and may be each independently a substituted or unsubstituted C₆-C₆₀ aryl group.

In another embodiment, Ra1 and Rb1 of Chemical Formula 2-1 above may be the same as or different from each other, and may be each independently a substituted or unsubstituted C₆-C₄₀ aryl group.

In another embodiment, Ra1 and Rb1 of Chemical Formula 2-1 above may be the same as or different from each other, and may be each independently a C₆-C₄₀ aryl group substituted or unsubstituted with one or more substituents selected from the group consisting of a C₁-C₄₀ alkyl group, a C₆-C₄₀ aryl group, -CN, and -SiR101R102R103.

In another embodiment, Ra1 and Rb1 of Chemical Formula 2-1 above may be the same as or different from each other, and may be each independently a phenyl group substituted or unsubstituted with a phenyl group, -CN or -SiR101R102R103; a biphenyl group substituted or unsubstituted with a phenyl group; a naphthyl group; a fluorene group substituted or unsubstituted with a methyl group or a phenyl group; a spirobifluorene group; or a triphenylene group.

In an embodiment of the present application, R101, R102, and R103 of Chemical Formula 2-1 above may be a phenyl group.

In an embodiment of the present application, the heterocyclic compound of Chemical Formula 2 above may be represented by any one of compounds below.

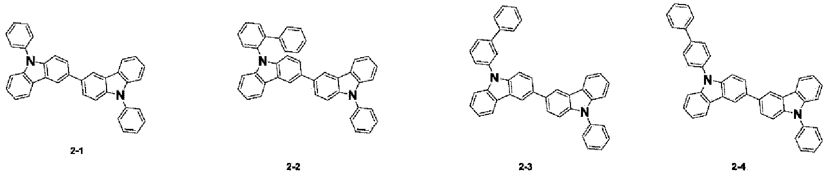

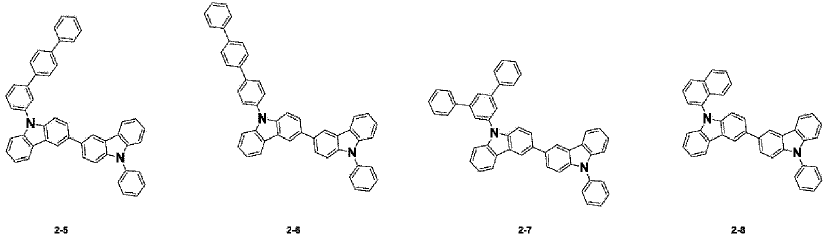

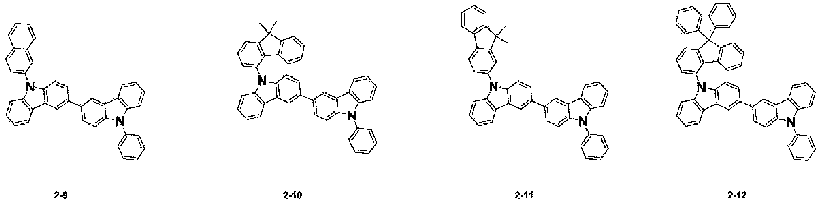

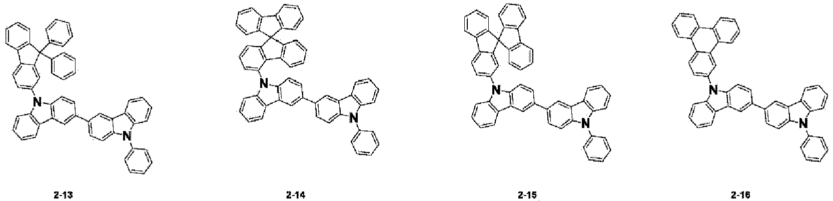

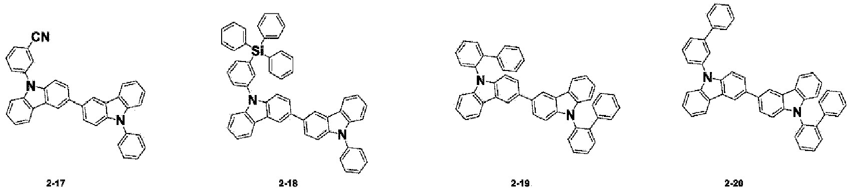

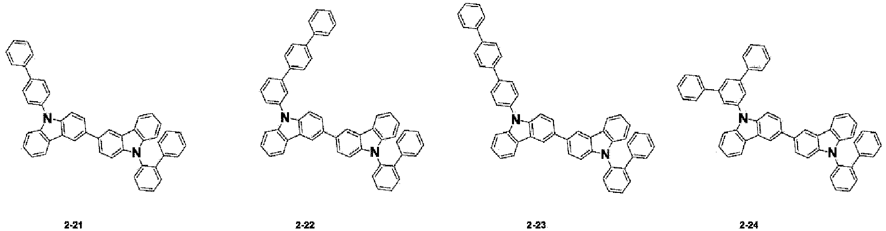

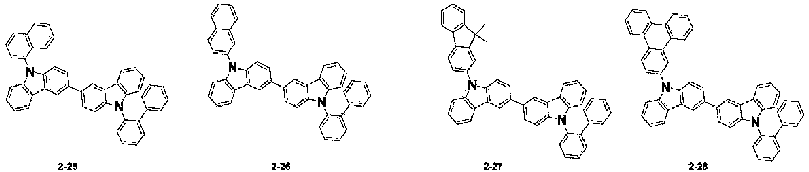

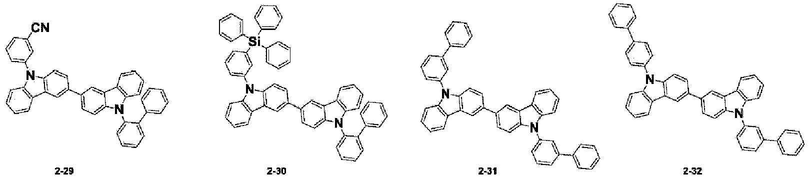

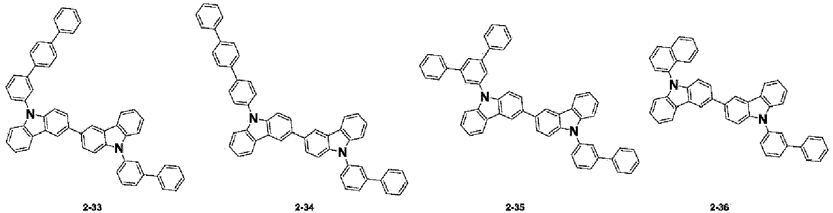

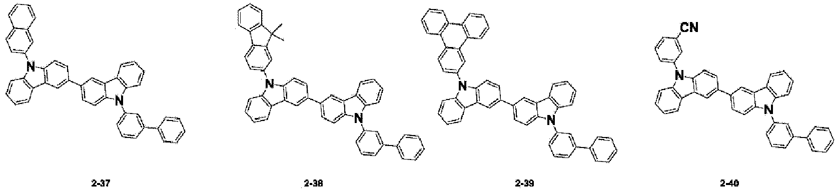

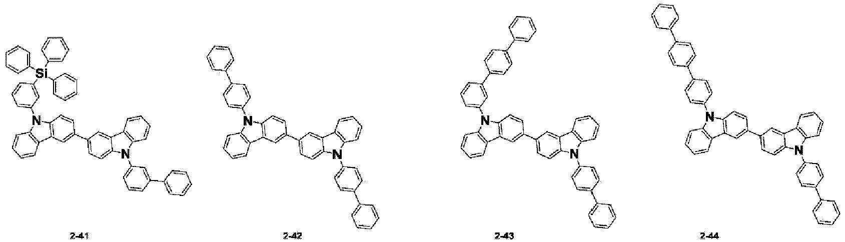

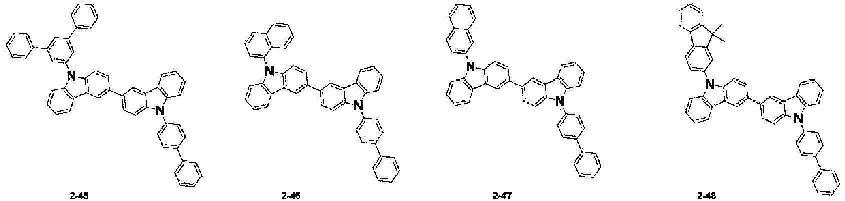

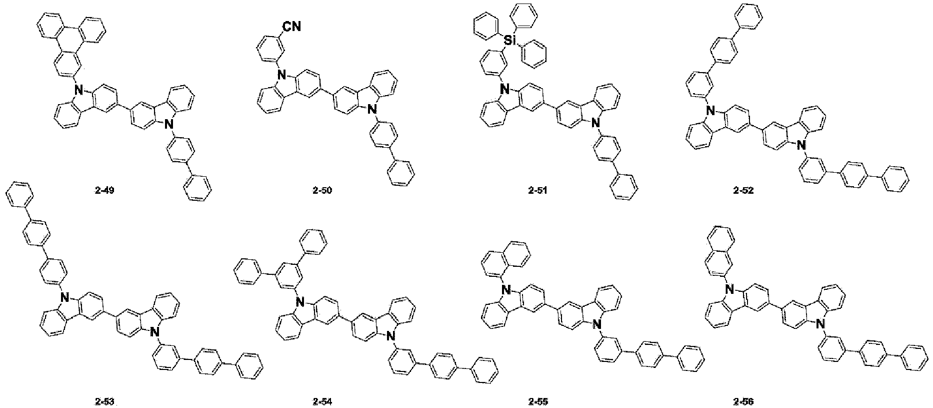

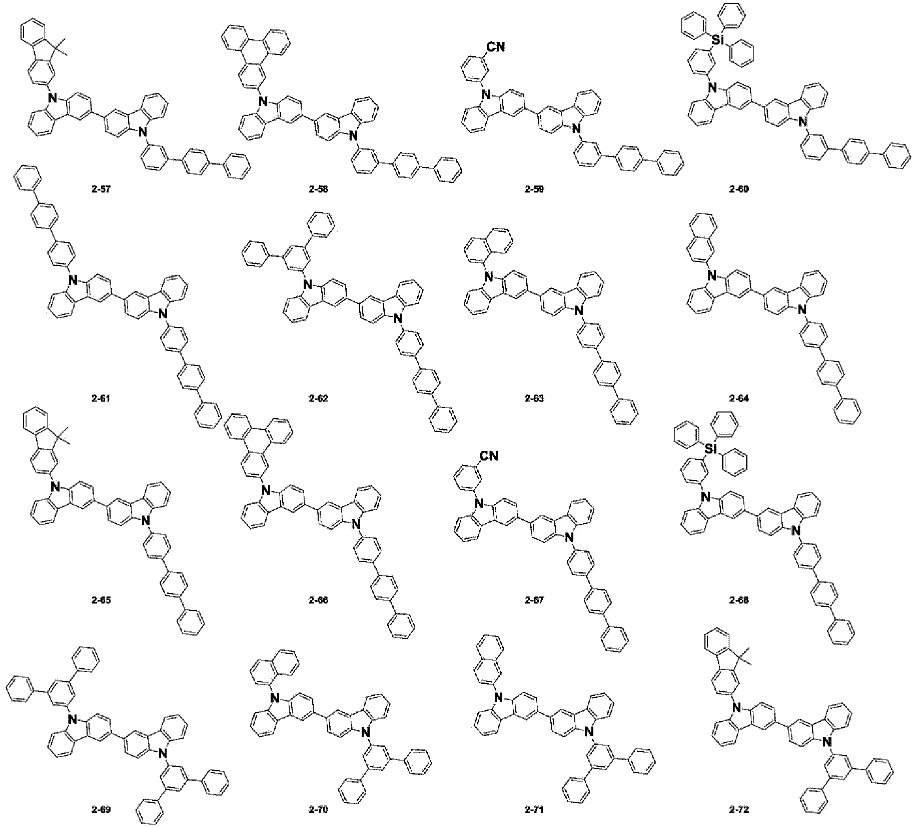

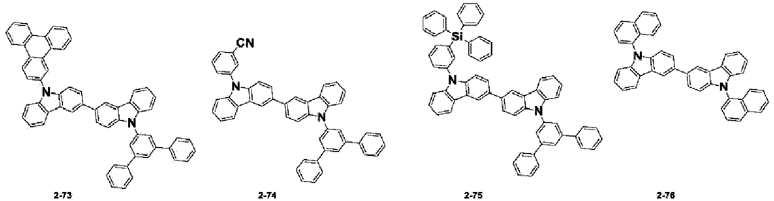

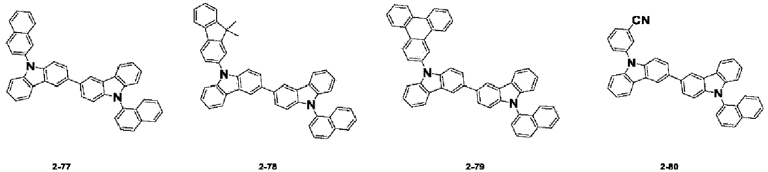

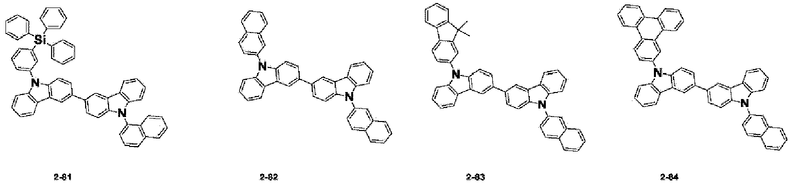

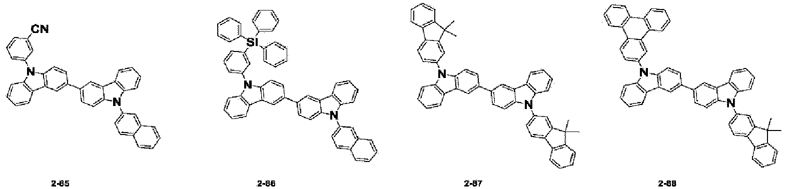

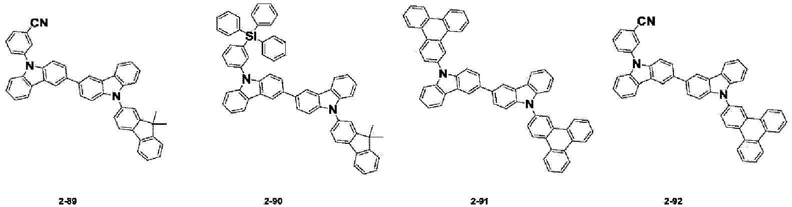

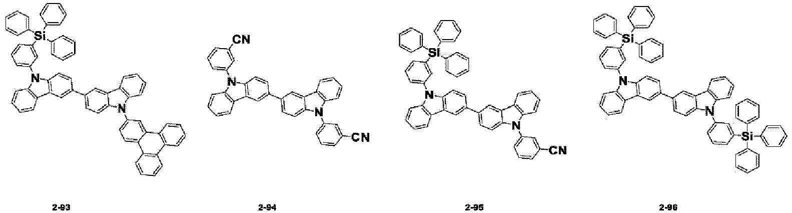

Further, another embodiment of the present application provides an organic material layer composition of an organic light emitting device, comprising the heterocyclic compound represented by Chemical Formula 1 above and the heterocyclic compound represented by Chemical Formula 2 above.

Specific details of the heterocyclic compound represented by Chemical Formula 1 above and the heterocyclic compound represented by Chemical Formula 2 above are the same as described above.

The weight ratio of the heterocyclic compound represented by Chemical Formula 1 above to the heterocyclic compound represented by Chemical Formula 2 above in the composition may be 1:10 to 10:1, may be 1:8 to 8:1, may be 1:5 to 5:1, and may be 1:2 to 2:1, but the present application is not limited thereto.

The composition may be used when forming an organic material of an organic light emitting device, and in particular, it may be more preferably used when forming a host of a light emitting layer.

In an embodiment of the present application, the organic material layer may comprise the heterocyclic compound represented by Chemical Formula 1 above and the heterocyclic compound represented by Chemical Formula 2 above, and may be used together with a phosphorescent dopant.

In an embodiment of the present application, the organic material layer may comprise the heterocyclic compound represented by Chemical Formula 1 above and the heterocyclic compound represented by Chemical Formula 2 above, and may be used together with an iridium-based dopant.

Those known in the art may be used as materials for the phosphorescent dopant.

For example, phosphorescent dopant materials represented by LL'MX', LL'L"M, LMX'X", L2MX', and L3M may be used, but the scope of the present disclosure is not limited by these examples.

Here, L, L', L", X', and X" are different bidentate ligands, and M is a metal forming an octahedral complex.

M may be iridium, platinum, osmium, or the like.

L may be an anionic bidentate ligand coordinated to M with the iridium-based dopant by sp2 carbon and a hetero atom, and X may function to trap electrons or holes. Non-limiting examples of L may comprise 2-(1-naphthyl)benzoxazole, (2-phenylbenzoxazole), (2-phenylbenzothiazole), (7,8-benzoquinoline), (thiophenepyrizine), phenylpyridine, benzothiophenepyrizine, 3-methoxy-2-phenylpyridine, thiophenepyrizine, tolylpyridine, etc. Non-limiting examples of X' and X" may comprise acetylacetonate (acac), hexafluoroacetylacetonate, salicylidene, picolinate, 8-hydroxyquinolinate, etc.

More specific examples are shown below, but the present application is not limited to these examples.

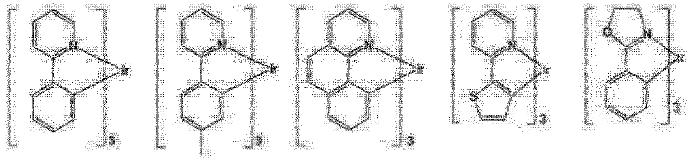

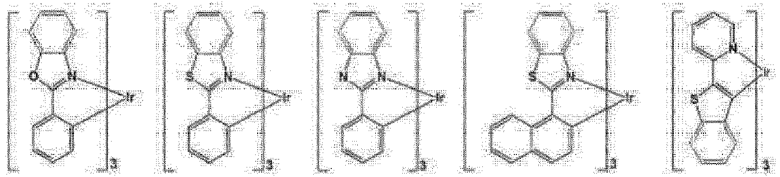

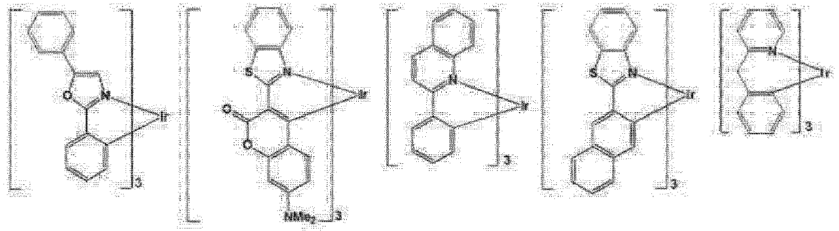

In an embodiment of the present application, Ir(ppy)₃ that is a green phosphorescent dopant may be used as the iridium-based dopant.

In an embodiment of the present application, the dopant may have a content of 1 to 15%, preferably 3 to 10%, and more preferably 5 to 10% based on the total light emitting layer.

In the organic light emitting device of the present disclosure, the organic material layer may comprise an electron injection layer or an electron transport layer, and the electron injection layer or the electron transport layer may comprise the heterocyclic compound.

In another organic light emitting device, the organic material layer may comprise a hole injection layer or a hole transport layer, and the hole injection layer or the hole transport layer may comprise the heterocyclic compound.

In another organic light emitting device, the organic material layer may comprise an electron blocking layer or a hole blocking layer, and the electron blocking layer or the hole blocking layer may comprise the heterocyclic compound.

In another organic light emitting device, the organic material layer may comprise an electron transport layer, a light emitting layer, or a hole blocking layer, and the electron transport layer, the light emitting layer or the hole blocking layer may comprise the heterocyclic compound.

The organic light emitting device of the present disclosure may further comprise one or more layers selected from the group consisting of a light emitting layer, a hole injection layer, a hole transport layer, an electron injection layer, an electron transport layer, an electron blocking layer, and a hole blocking layer.

FIGS. 1 to 3 exemplify the lamination order of the electrodes and the organic material layer of the organic light emitting device according to an embodiment of the present application. However, the scope of the present application is not intended to be limited by these drawings, and the structure of an organic light emitting device known in the art may also be applied to the present application.

According to FIG. 1, an organic light emitting device in which an anode 200, an organic material layer 300, and a cathode 400 are sequentially laminated on a substrate 100 is illustrated. However, it is not limited to such a structure only, and as shown in FIG. 2, an organic light emitting device in which a cathode, an organic material layer, and an anode are sequentially laminated on a substrate may also be implemented.

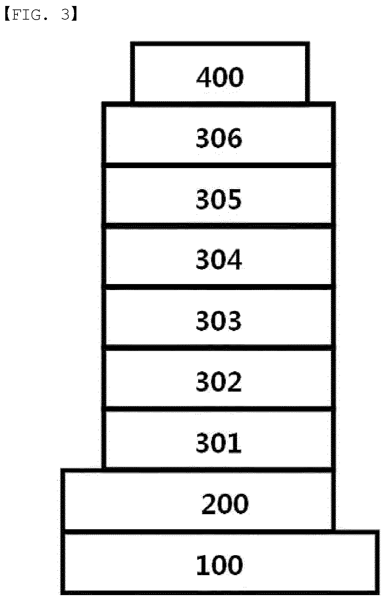

FIG. 3 exemplifies a case in which the organic material layer is multiple layers. The organic light emitting device according to FIG. 3 comprises a hole injection layer 301, a hole transport layer 302, a light emitting layer 303, a hole blocking layer 304, an electron transport layer 305, and an electron injection layer 306. However, the scope of the present application is not limited by such a lamination structure, and as needed, the remaining layers except for the light emitting layer may be omitted, and other necessary functional layers may be further added.

In an embodiment of the present application, there is provided a method for manufacturing an organic light emitting device, the method comprising the steps of: preparing a substrate; forming a first electrode on the substrate; forming one or more organic material layers on the first electrode; and forming a second electrode on the organic material layer, wherein the step of forming the organic material layer comprises a step of forming one or more organic material layers using the organic material layer composition according to an embodiment of the present application.

In an embodiment of the present application, there is provided a method for manufacturing an organic light emitting device, in which the step of forming the organic material layer is forming the organic material layer using a thermal vacuum deposition method by premixing the heterocyclic compound of Chemical Formula 1 above and the heterocyclic compound of Chemical Formula 2 above.

The premixing means first mixing the heterocyclic compound of Chemical Formula 1 and the heterocyclic compound of Chemical Formula 2 in one source of supply before depositing on the organic material layer.

The premixed materials may be referred to as the organic material layer composition according to an embodiment of the present application.

The organic material layer comprising Chemical Formula 1 above may further comprise other materials as needed.

The organic material layer comprising Chemical Formulae 1 and 2 above at the same time may further comprise other materials as needed.

In the organic light emitting device according to an embodiment of the present application, although materials other than the compound of Chemical Formula 1 above or Chemical Formula 2 above are exemplified below, these are for illustration only, not for limiting the scope of the present application, and may be replaced with materials known in the art.

As an anode material, materials having a relatively high work function may be used, and transparent conductive oxides, metals, conductive polymers, or the like may be used. Specific examples of the anode material may comprise: metals such as vanadium, chromium, copper, zinc, and gold, or alloys thereof; metal oxides such as zinc oxide, indium oxide, indium tin oxide (ITO), and indium zinc oxide (IZO); combinations of metals such as ZnO:Al or SnO₂:Sb and oxides; conductive polymers such as poly(3-methylthiophene), poly[3,4-(ethylene-1,2-dioxy)thiophene] (PEDOT), polypyrrole, and polyaniline; etc., but the present application is not limited thereto.

As a cathode material, materials having a relatively low work function may be used, and metals, metal oxides, conductive polymers, or the like may be used. Specific examples of the cathode material may comprise: metals such as magnesium, calcium, sodium, potassium, titanium, indium, yttrium, lithium, gadolinium, aluminum, silver, tin, and lead, or alloys thereof; a multilayered material such as LiF/Al or LiO₂/Al; etc., but the present application is not limited thereto.

As a hole injection material, known hole injection materials may also be used, and for example, phthalocyanine compounds such as copper phthalocyanine, etc. disclosed in US Pat. No. 4,356,429, or starburst-type amine derivatives disclosed in the literature [Advanced Material, 6, p.677 (1994)] such as tris(4-carbazolyl-9-ylphenyl)amine (TCTA), 4,4',4"-tri[phenyl(m-tolyl)amino]triphenylamine (m-MTDATA), 1,3,5-tris[4-(3-methylphenylphenylamino)phenyl]benzene (m-MTDAPB), and a soluble conductive polymer of Polyaniline/Dodecylbenzenesulfonic acid, Poly(3,4-ethylenedioxythiophene)/Poly(4-styrenesulfonate), Polyaniline/Camphor sulfonic acid, Polyaniline/Poly(4-styrene-sulfonate), or the like may be used.

As a hole transport material, pyrazoline derivatives, arylamine-based derivatives, stilbene derivatives, triphenyldiamine derivatives, etc. may be used, and low molecular weight or high molecular weight materials may also be used.

As an electron transport material, metal complexes or the like of oxadiazole derivatives, anthraquinodimethane and its derivatives, benzoquinone and its derivatives, naphthoquinone and its derivatives, anthraquinone and its derivatives, tetracyanoanthraquinodimethane and its derivatives, fluorenone derivatives, diphenyldicyanoethylene and its derivatives, diphenoquinone derivatives, and 8-hydroxyquinoline and its derivatives may be used, and high molecular weight materials as well as low molecular weight materials may also be used.

As an electron injection material, for example, LiF is typically used in the art, but the present application is not limited thereto.

As a light emitting material, a red, green, or blue light emitting material may be used, and if necessary, two or more light emitting materials may be mixed and used. At this time, two or more light emitting materials may be deposited and used as individual sources, or may be premixed to be deposited and used as a single source. Further, a fluorescent material may be used as a light emitting material, but a phosphorescent material may also be used. As the light emitting material, a material that emits light by combining holes and electrons respectively injected from the anode and the cathode may be used alone, but materials in which the host material and the dopant material together involve in light emission may also be used.

When mixing and using the host of the light emitting material, hosts of the same series may be mixed and used, or hosts of different series may also be mixed and used. For example, any two or more types of materials of n-type host materials and p-type host materials may be selected and used as a host material of the light emitting layer.

The organic light emitting device according to an embodiment of the present application may be a top emission type, a back emission type, or a double side emission type depending on materials used.

A heterocyclic compound according to an embodiment of the present application may act on a principle similar to that applied to an organic light emitting device even in an organic electronic device comprising an organic solar cell, an organic photoreceptor, an organic transistor, etc.

Hereinafter, the present specification will be described in more detail through Examples, but these are only for exemplifying the present application and not for limiting the scope of the present application.

### <Preparation Example>

### <Preparation Example 1> Preparation of Compound 1-1 (E)

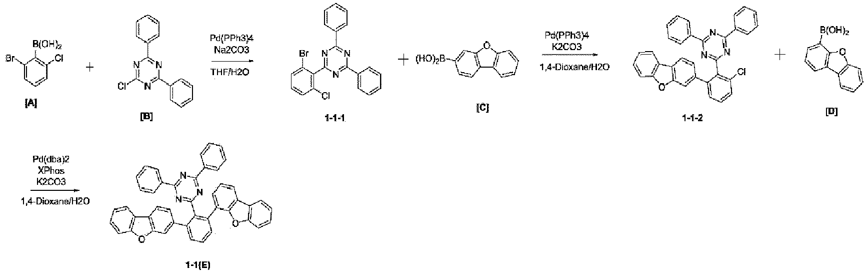

### Preparation of Compound 1-1-1

10 g (0.043 mol) of (2-bromo-6-chlorophenyl)boronic acid, 11.38 g (0.043 mol) of 2-chloro-4,6-diphenyl-1,3,5-triazine, 3.58 g (0.0031 mol) of Pd(PPh₃)₄, 6.78 g (0.064 mol) of sodium carbonate, and a tetrahydrofuran/water (H₂O) mixture (100 mL/30 mL) were refluxed at 100°C in a one neck round bottom flask. After concentrating the reaction product by extracting a reaction product with dichloromethane, the concentrated reaction product was filtered with a silica gel filter. After concentrating the filtrate, the concentrated filtrate was treated with dichloromethane/methanol to obtain 15.09 g (83%) of Compound 1-1-1.

### Preparation of Compound 1-1-2

11.23 g (0.027 mol) of 2-(2-bromo-6-chlorophenyl)-4,6-diphenyl-1,3,5-triazine, 6.36 g (0.030 mol) of dibenzo[b,d]furan-3-ylboronic acid, 1.56 g (0.0014 mol) of Pd(PPh₃)₄, 7.46 g (0.054 mol) of potassium carbonate, and a 1,4-dioxane/water (H₂O) mixture (100 mL/30 mL) were refluxed at 125°C in a one neck round bottom flask. After completing the reaction to obtain a reaction product, a precipitated solid was obtained by filtering the reaction product. The solid thus obtained was dissolved in dichlorobenzene and subjected to filtration with a silica gel filter. After concentrating the filtrate, the concentrated filtrate was treated with methanol to obtain 10.46 g (76%) of Compound 1-1-2.

### Preparation of Compound 1-1 (E)

10.46 g (0.021 mol) of 2-(2-chloro-6-(dibenzo[b,d]furan-3-yl)phenyl)-4,6-diphenyl-1,3,5-triazine, 4.90 g (0.023 mol) of dibenzo[b,d]furan-4-ylboronic acid, 0.063 g (0.0011 mol) of Pd(dba)₂, 1.50 g (0.0032 mol) of XPhos, 5.80 g (0.042 mol) of potassium carbonate, and a 1,4-dioxane/water (H₂O) mixture (100 mL/30 mL) were refluxed at 130°C in a one neck round bottom flask. After completing the reaction to obtain a reaction product, a precipitated solid was obtained by filtering the reaction product. The solid thus obtained was dissolved in dichlorobenzene and subjected to filtration with a silica gel filter. After concentrating the filtrate, the concentrated filtrate was treated with methanol to obtain 7.68 g (57%) of Compound 1-1 (E).

Compound E below was synthesized in the same manner in Preparation Example 1 above except that other intermediates A, B, C, and D of Table 1 below were used.

**[Table 1]**

| Com pou nd | A | B | C | D | E | Yield |
|---|---|---|---|---|---|---|
| 1-1 | 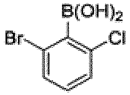 | 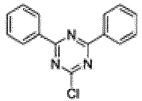 | 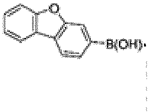 | 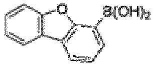 | 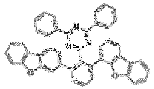 | 57 % |
| 1-3 | 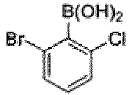 | 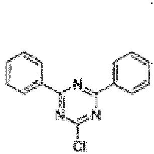 | 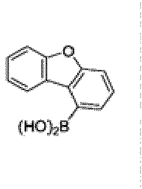 | 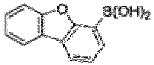 | 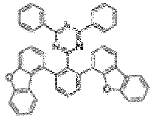 | 83 % |
| 1-4 | 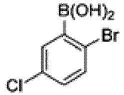 | 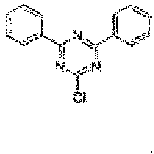 | 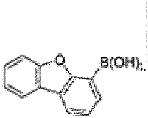 | 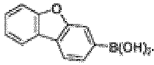 | 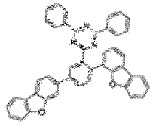 | 70 % |
| 1-7 | 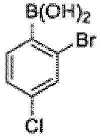 | 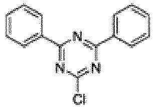 | 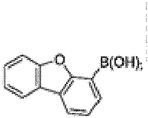 | 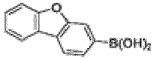 | 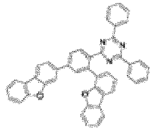 | 71 % |
| 1-9 | 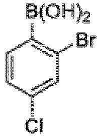 | 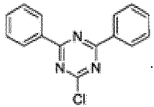 | 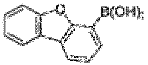 | 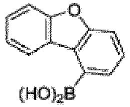 | 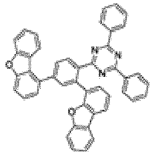 | 63 % |
| 1-15 | 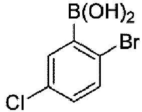 | 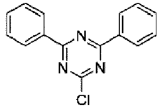 | 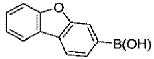 | 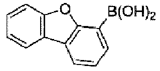 | 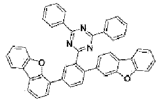 | 60 % |
| 1-18 | 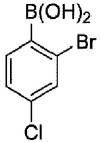 | 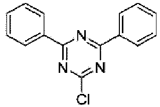 | 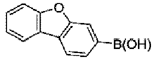 | 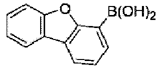 | 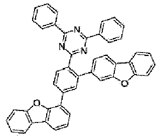 | 77 % |
| 1-20 | 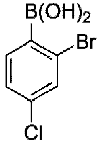 | 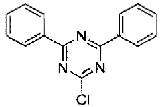 | 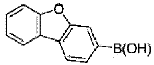 | 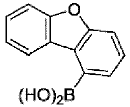 | 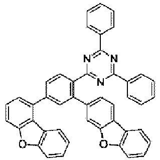 | 83 % |
| 1-22 | 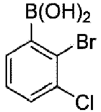 | 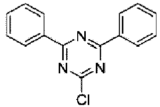 | 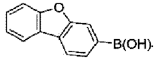 | 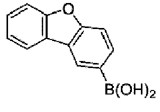 | 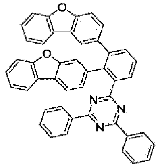 | 79 % |
| 1-26 | 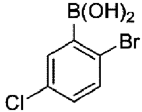 | 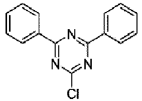 | 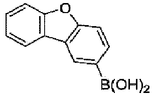 | 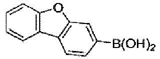 | 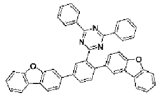 | 66 % |
| 1-28 | 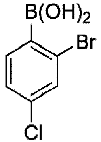 | 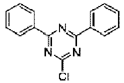 | 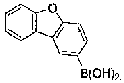 | 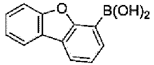 | 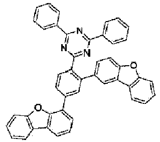 | 65 % |
| 1-30 | 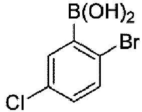 | 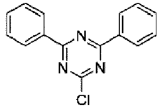 | 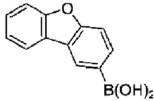 | 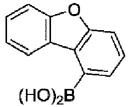 | 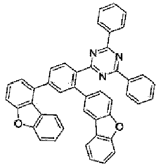 | 87 % |
| 1-34 | 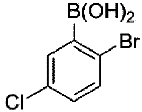 | 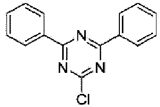 | 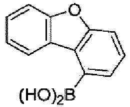 | 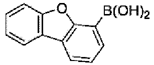 | 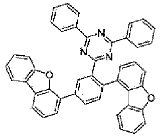 | 60 % |
| 1-35 | 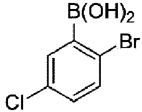 | 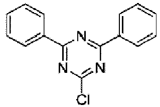 | 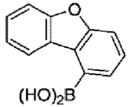 | 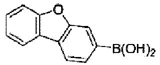 | 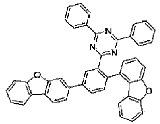 | 81 % |
| 1-40 | 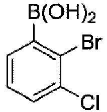 | 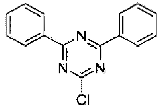 | 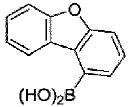 | 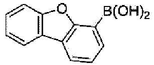 | 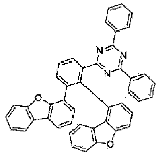 | 73 % |
| 1-44 | 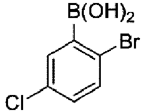 | 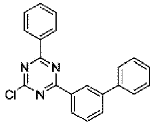 | 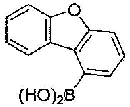 | 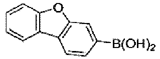 | 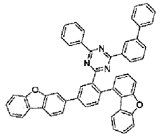 | 70 % |
| 1-45 | 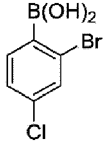 | 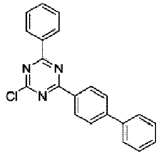 | 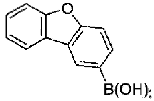 | 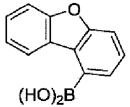 | 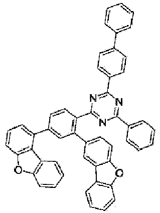 | 64 % |
| 1-46 | 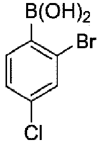 | 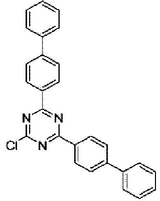 | 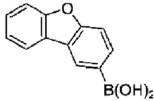 | 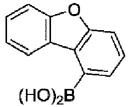 | 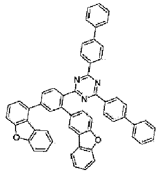 | 87 % |
| 1-52 | 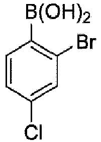 | 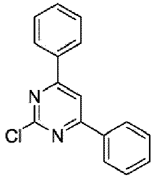 | 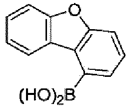 | 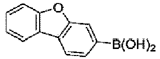 | 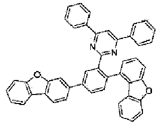 | 66 % |
| 1-53 | 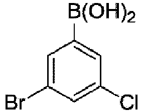 | 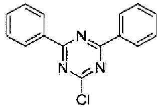 | 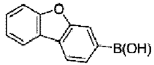 | 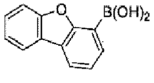 | 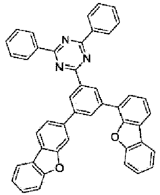 | 72 % |
| 1-54 | 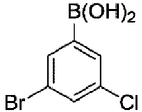 | 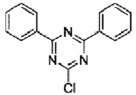 | 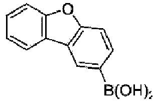 | 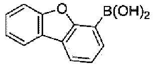 | 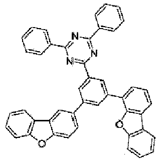 | 73 % |
| 1-55 | 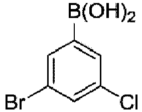 | 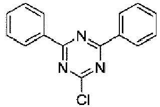 | 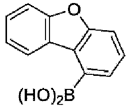 | 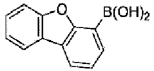 | 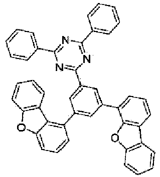 | 71 % |
| 1-56 | 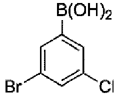 | 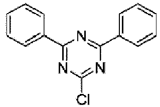 | 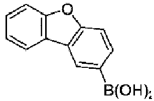 | 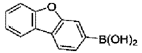 | 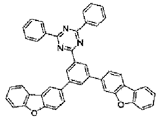 | 65 % |
| 1-60 | 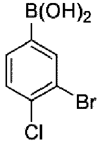 | 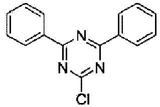 | 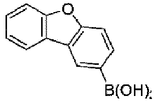 | 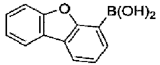 | 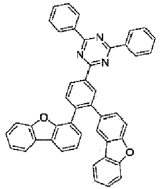 | 60 % |
| 1-64 | 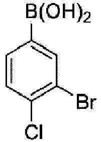 | 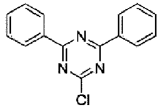 | 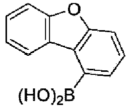 | 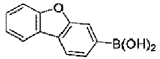 | 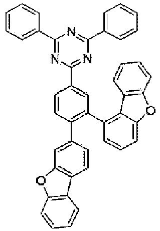 | 67 % |
| 1-71 | 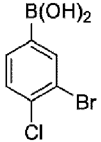 | 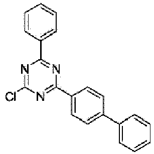 | 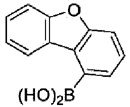 | 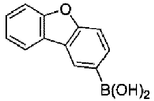 | 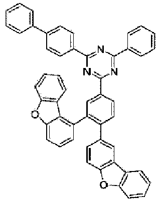 | 69 % |
| 1-73 | 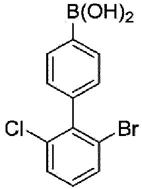 | 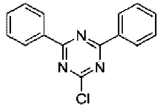 | 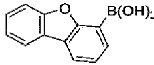 | 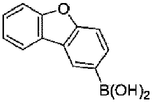 | 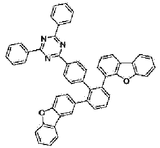 | 51 % |
| 1-75 | 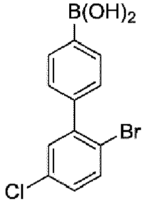 | 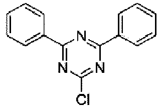 | 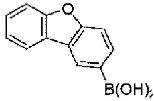 | 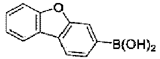 | 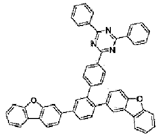 | 76 % |
| 1-77 | 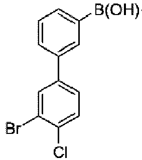 | 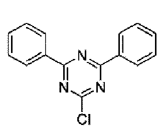 | 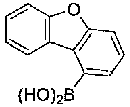 | 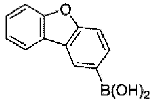 | 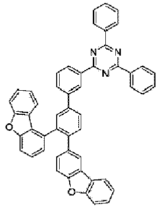 | 63 % |
| 1-78 | 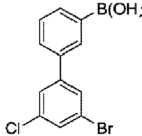 | 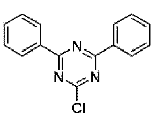 | 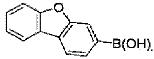 | 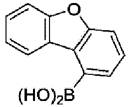 | 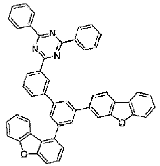 | 61 % |
| 1-83 | 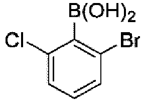 | 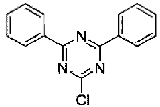 | 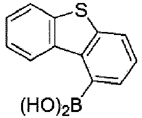 | 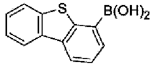 | 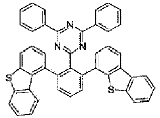 | 77 % |
| 1-84 | 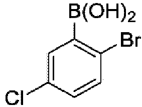 | 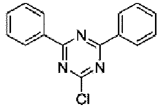 | 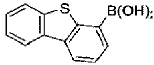 | 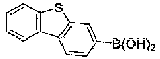 | 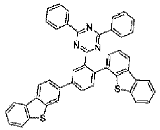 | 63 % |
| 1-87 | 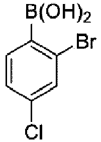 | 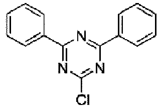 | 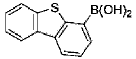 | 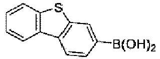 | 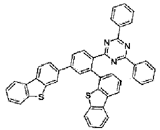 | 55 % |
| 1-89 | 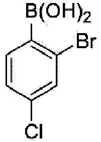 | 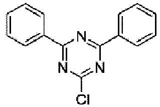 | 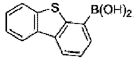 | 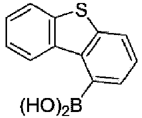 | 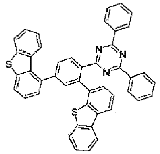 | 58 % |
| 1-98 | 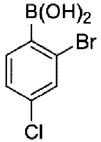 | 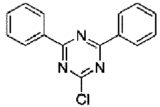 | 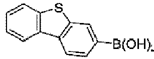 | 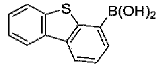 | 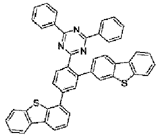 | 51 % |
| 1-99 | 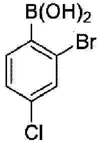 | 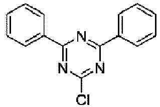 | 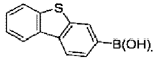 | 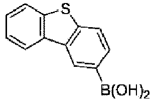 | 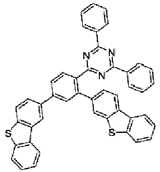 | 60 % |
| 1-10 0 | 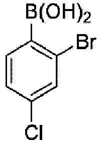 | 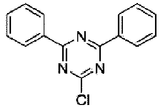 | 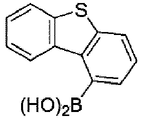 | 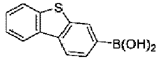 | 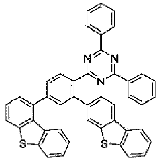 | 62 % |
| 1-10 2 | 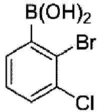 | 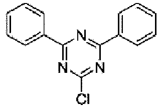 | 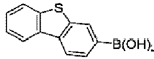 | 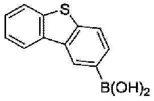 | 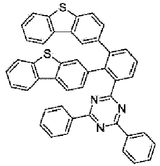 | 39 % |
| 1-11 0 | 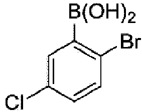 | 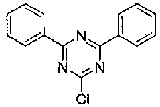 | 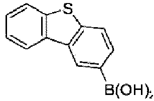 | 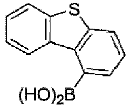 | 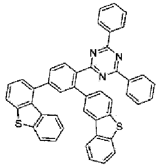 | 68 % |
| 1-12 5 | 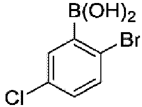 | 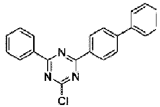 | 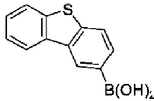 | 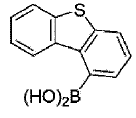 | 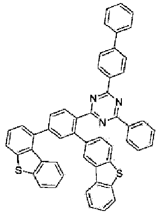 | 70 % |
| 1-12 8 | 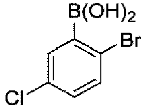 | 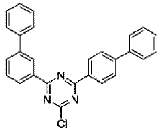 | 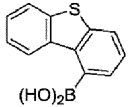 | 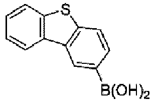 | 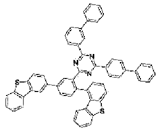 | 63 % |
| 1-13 5 | 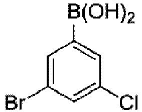 | 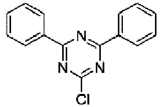 | 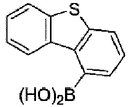 | 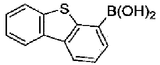 | 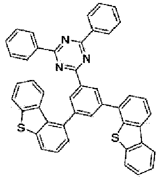 | 58 % |
| 1-13 6 | 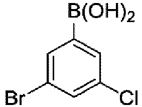 | 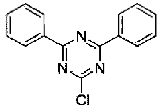 | 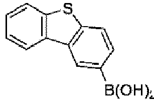 | 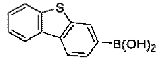 | 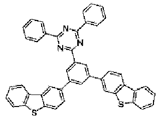 | 74 % |
| 1-13 7 | 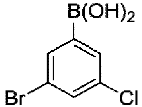 | 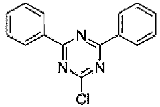 | 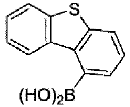 | 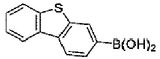 | 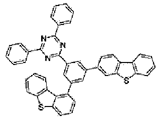 | 81 % |
| 1-13 8 | 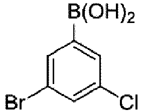 | 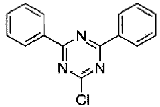 | 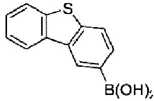 | 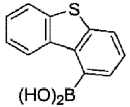 | 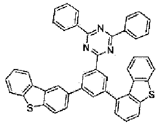 | 79 % |
| 1-15 5 | 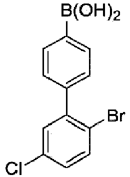 | 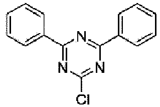 | 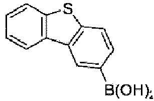 | 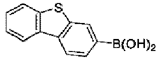 | 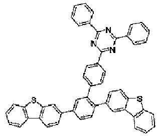 | 71 % |
| 1-15 8 | 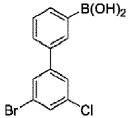 | 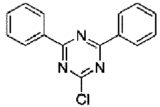 | 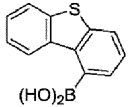 | 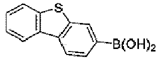 | 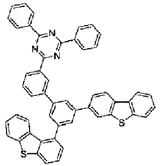 | 52 % |
| 1-16 3 | 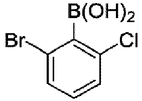 | 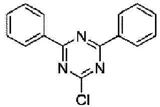 | 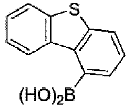 | 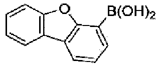 | 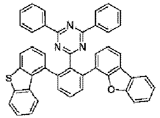 | 69 % |
| 1-16 5 | 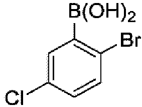 | 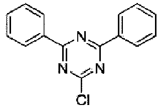 | 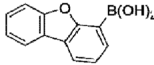 | 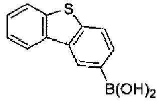 | 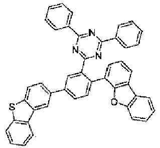 | 63 % |
| 1-17 8 | 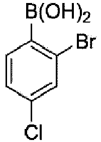 | 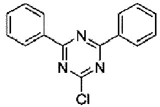 | 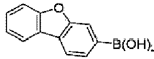 | 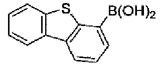 | 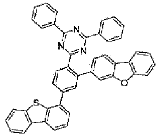 | 78 % |
| 1-18 0 | 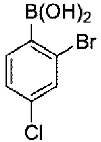 | 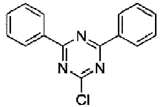 | 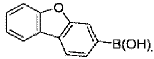 | 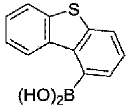 | 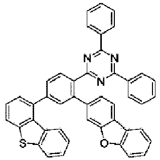 | 56 % |
| 1-18 6 | 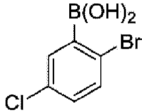 | 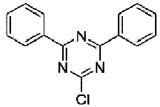 | 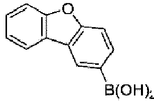 | 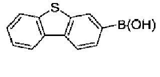 | 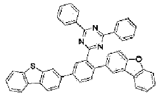 | 50 % |
| 1-19 0 | 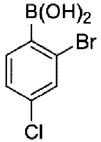 | 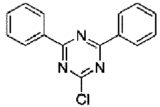 | 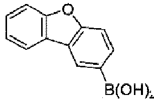 | 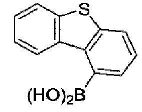 | 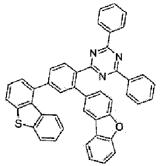 | 65 % |
| 1-19 9 | 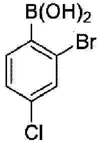 | 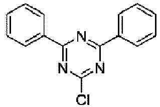 | 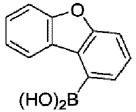 | 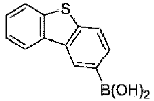 | 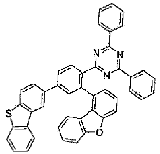 | 62 % |
| 1-20 0 | 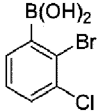 | 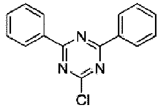 | 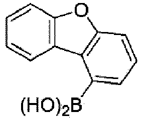 | 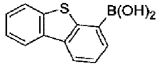 | 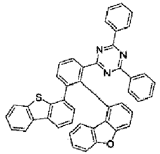 | 75 % |
| 1-20 4 | 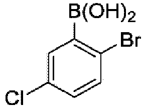 | 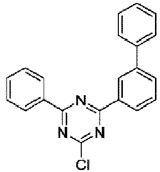 | 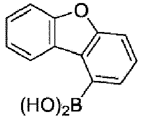 | 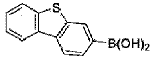 | 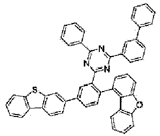 | 88 % |
| 1-20 9 | 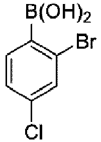 | 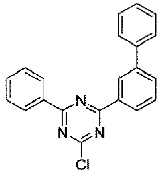 | 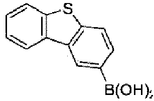 | 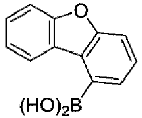 | 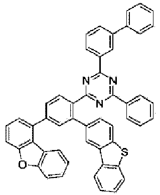 | 74 % |
| 1-21 3 | 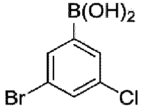 | 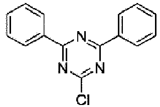 | 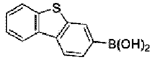 | 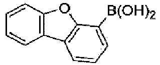 | 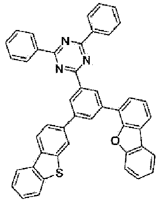 | 71 % |
| 1-21 4 | 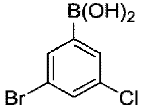 | 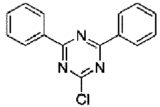 | 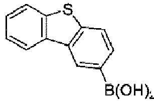 | 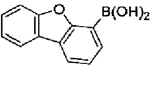 | 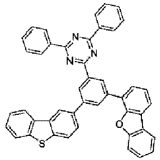 | 62 % |
| 1-21 5 | 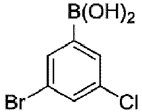 | 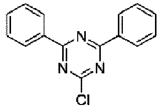 | 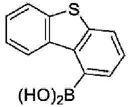 | 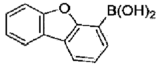 | 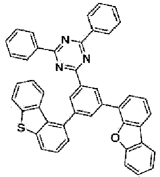 | 67 % |
| 1-21 6 | 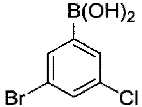 | 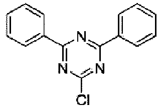 | 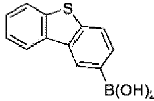 | 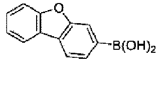 | 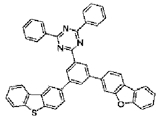 | 78 % |
| 1-21 9 | 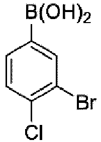 | 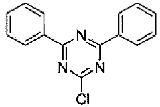 | 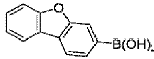 | 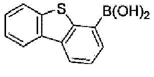 | 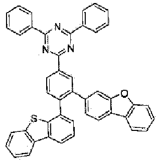 | 85 % |
| 1-23 2 | 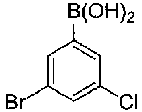 | 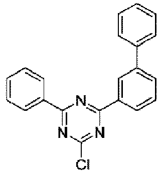 | 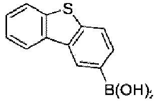 | 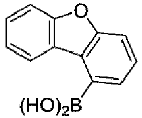 | 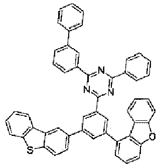 | 74 % |
| 1-23 6 | 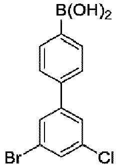 | 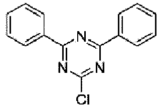 | 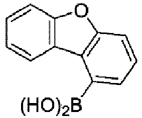 | 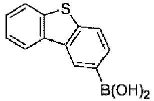 | 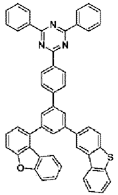 | 70 % |
| 1-23 8 | 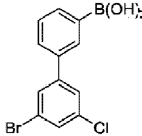 | 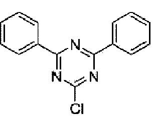 | 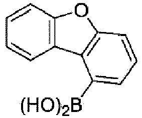 | 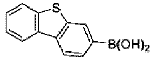 | 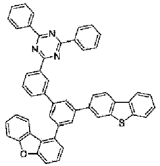 | 62 % |
| 1-24 0 | 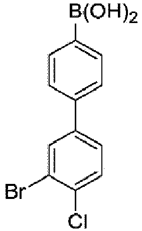 | 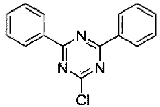 | 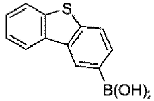 | 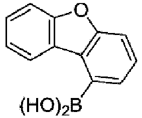 | 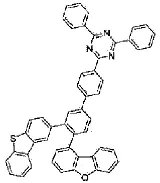 | 60 % |
| 1-26 7 | 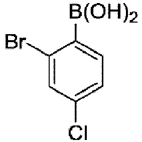 | 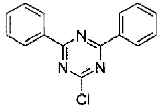 | 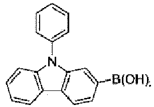 | 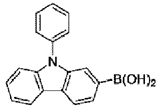 | 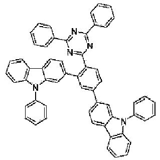 | 71 % |
| 1-27 1 | 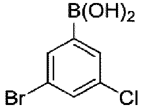 | 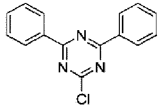 | 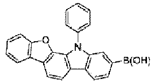 | 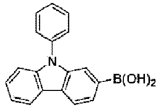 | 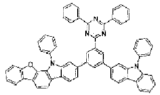 | 80 % |
| 1-27 2 | 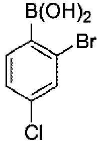 | 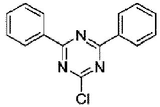 | 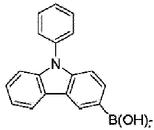 | 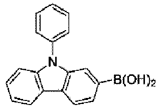 | 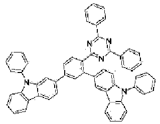 | 64 % |
| 1-27 4 | 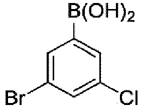 | 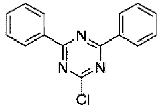 | 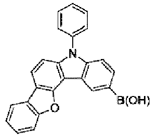 | 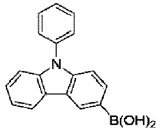 | 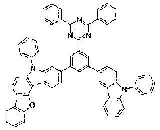 | 72 % |
| 1-27 8 | 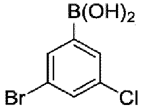 | 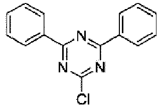 | 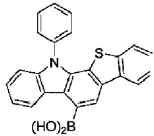 | 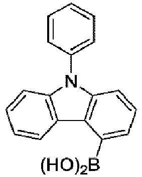 | 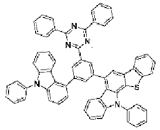 | 60 % |
| 1-28 0 | 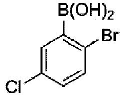 | 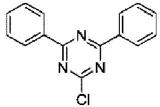 | 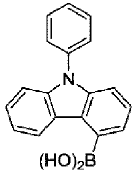 | 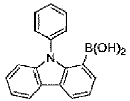 | 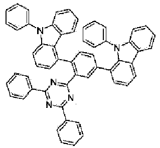 | 58 % |

### <Preparation Example 2> Preparation of Compound 1-242 (F)

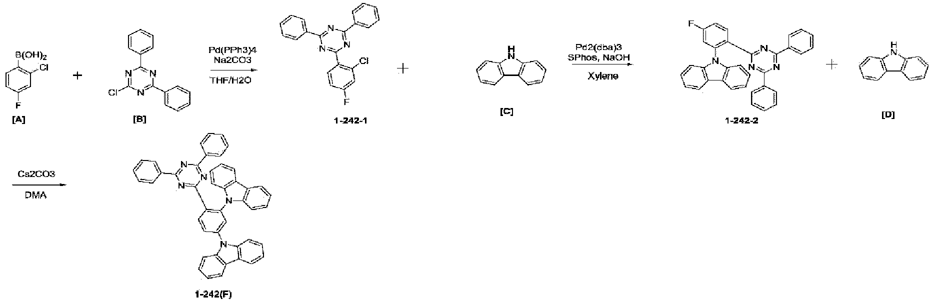

### Preparation of Compound 1-242-1

10 g (0.057 mol) of (2-chloro-4-fluorophenyl)boronic acid, 15.35 g (0.057 mol) of 2-chloro-4,6-diphenyl-1,3,5-triazine, 3.29 g (0.0029 mol) of Pd(PPh₃)₄, 12.08 g (0.11 mol) of sodium carbonate, and a tetrahydrofuran/water (H₂O) mixture (100 mL/30 mL) were refluxed at 100°C in a one neck round bottom flask. After concentrating the reaction product by extracting a reaction product with dichloromethane, the concentrated reaction product was filtered with a silica gel filter. After concentrating the filtrate, the concentrated filtrate was treated with dichloromethane/methanol to obtain 16.91 g (82%) of Compound 1-242-1.

### Preparation of Compound 1-242-2

A mixture of 16.91 g (0.047 mol) of 2-(2-chloro-4-fluorophenyl)-4,6-diphenyl-1,3,5-triazine, 7.82 g (0.047 mol) of 9H-carbazole, 2.15 g (0.0024 mol) of Pd₂(dba)₃, 38.59 g (0.094 mol) of SPhos, 3.76 g (0.094 mol) of sodium hydroxide, and 160 mL of xylene was refluxed at 180°C in a one neck round bottom flask. After completing the reaction to obtain a reaction product, a precipitated solid was obtained by filtering the reaction product. The solid was dissolved, filtered with a silica gel filter, and concentrated to obtain 13.66 g (59%) of Compound 1-242-2.

### Preparation of Compound 1-242 (F)

A mixture of 13.66 g (0.028 mol) of 9-(2-(4,6-diphenyl-1,3,5-triazin-2-yl)-5-fluorophenyl)-9H-carbazole, 4.68 g (0.028 mol) of 9H-carbazole, 18.25 g (0.056 mol) of Cs₂CO₃, and 130 mL of dimethylacetamide was refluxed at 180°C in a one neck round bottom flask. After completing the reaction to obtain a reaction product, a precipitated solid was obtained by filtering the reaction product. The solid was dissolved, filtered with a silica gel filter, and concentrated to obtain 12.36 g (69%) of Compound 1-242 (F).

Compound F below was synthesized in the same manner in Preparation Example 2 above except that other intermediates A, B, C, and D of Table 2 below were used.

**[Table 2]**

| Com pou nd | A | B | C | D | F | Yie Id |
|---|---|---|---|---|---|---|
| 1 24 2 | 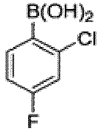 | 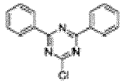 | 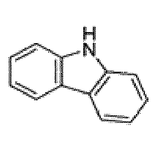 | 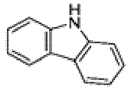 | 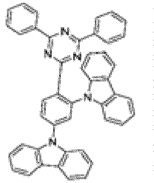 | 6 9 % |
| 1-24 3 | 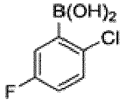 | 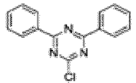 | 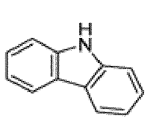 | 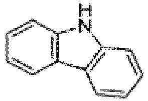 | 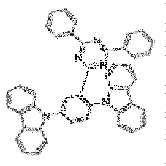 | 4 7 % |
| 1-24 4 | 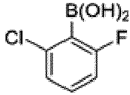 | 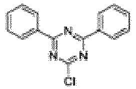 | 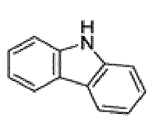 | 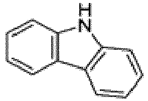 | 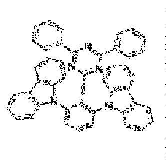 | 5 3 % |
| 1-24 9 | 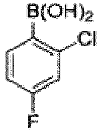 | 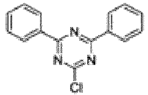 | 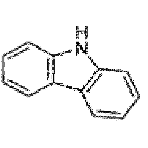 | 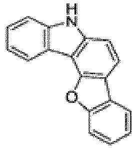 | 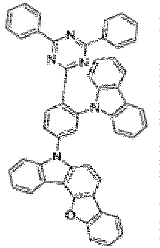 | 7 3 % |
| 1-25 3 | 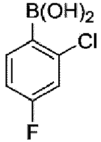 | 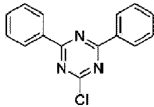 | 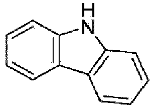 | 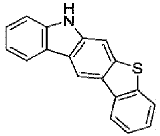 | 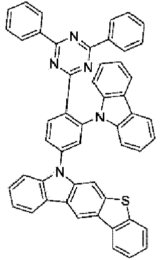 | 6 5 % |
| 1-25 5 | 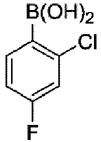 | 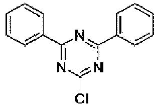 | 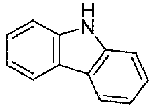 | 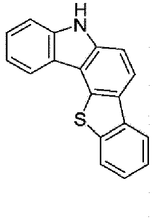 | 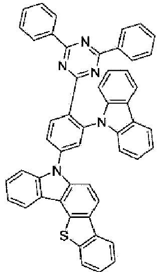 | 6 1 % |
| 1-25 8 | 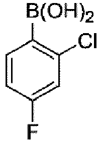 | 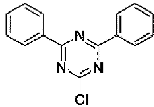 | 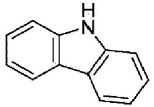 | 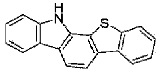 | 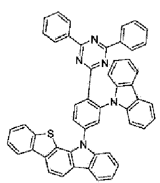 | 7 8 % |
| 1-25 9 | 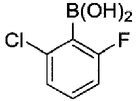 | 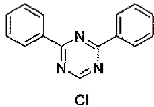 | 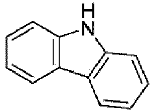 | 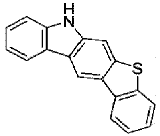 | 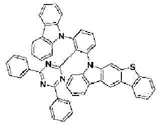 | 7 7 % |
| 1-26 0 | 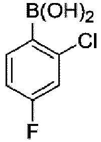 | 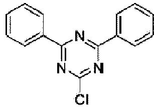 | 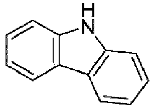 | 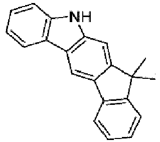 | 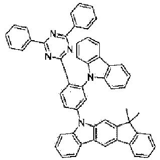 | 7 4 % |
| 1-26 1 | 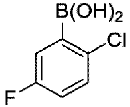 | 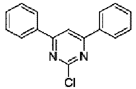 | 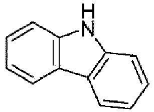 | 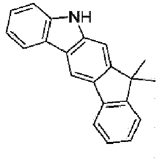 | 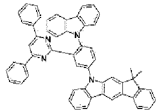 | 6 2 % |
| 1-26 2 | 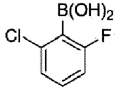 | 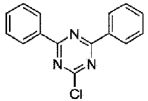 | 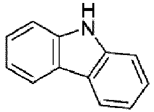 | 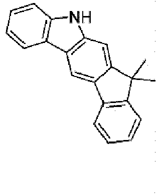 | 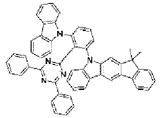 | 5 2 % |
| 1-26 3 | 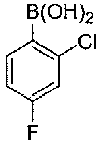 | 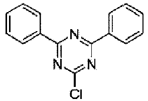 | 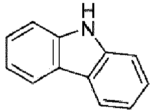 | 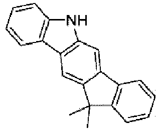 | 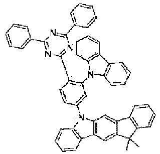 | 6 1 % |
| 1-26 5 | 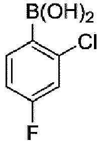 | 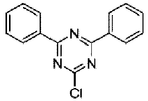 | 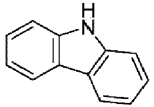 | 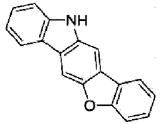 | 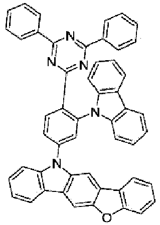 | 6 6 % |
| 1-28 9 | 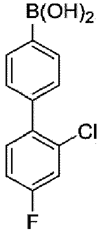 | 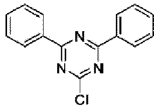 | 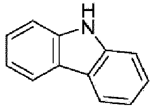 | 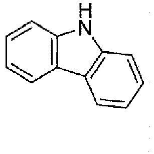 | 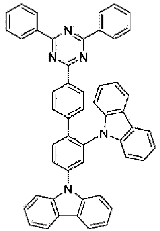 | 8 5 % |
| 1-29 1 | 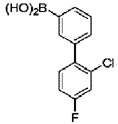 | 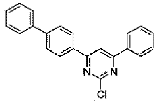 | 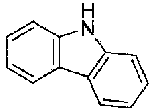 | 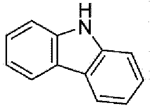 | 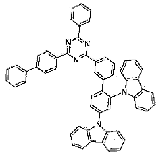 | 7 0 % |

### <Preparation Example 3> Preparation of Compound 1-281 (G)

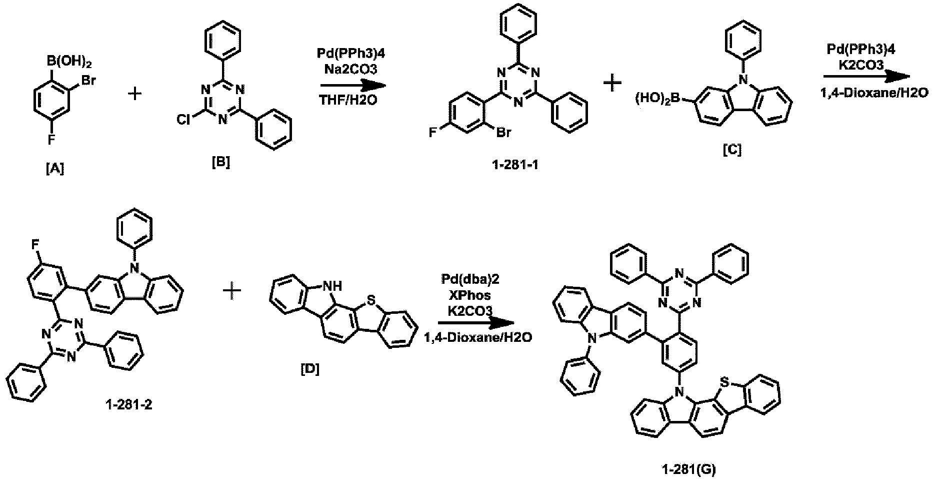

### Preparation of Compound 1-281-1

10 g (0.046 mol) of (2-bromo-4-fluorophenyl)boronic acid, 12.23 g (0.046 mol) of 2-chloro-4,6-diphenyl-1,3,5-triazine, 2.66 g (0.0023 mol) of Pd(PPh₃)₄, 9.75 g (0.092 mol) of sodium carbonate, and a tetrahydrofuran/water (H₂O) mixture (100 mL/30 mL) were refluxed at 100°C in a one neck round bottom flask. After concentrating the reaction product by extracting a reaction product with dichloromethane, the concentrated reaction product was filtered with a silica gel filter. After concentrating the filtrate, the concentrated filtrate was treated with dichloromethane/methanol to obtain 14.95 g (80%) of Compound 1-281-1.

### Preparation of Compound 1-281-2

14.95 g (0.037 mol) of 2-(2-bromo-4-fluorophenyl)-4,6-diphenyl-1,3,5-triazine, 10.62 g (0.037 mol) of (9-phenyl-9H-carbazol-2-yl)boronic acid, 2.14 g (0.0019 mol) of Pd(PPh₃)₄, 10.23 g (0.074 mol) of potassium carbonate, and a 1,4-dioxane/water (H₂O) mixture (140 mL/40 mL) were refluxed at 125°C in a one neck round bottom flask. After concentrating the reaction product by extracting a reaction product with dichloromethane, the concentrated reaction product was filtered with a silica gel filter. After concentrating the filtrate, the concentrated filtrate was treated with dichloromethane/methanol to obtain 15.15 g (72%) of Compound 1-281-2.

### Preparation of Compound 1-281 (G)

A mixture of 15.15 g (0.027 mol) of 2-(2-(4,6-diphenyl-1,3,5-triazin-2-yl)-5-fluorophenyl)-9-phenyl-9H-carbazole, 7.38 g (0.027 mol) of 12H-benzo[4,5]thieno[2,3-a]carbazole, 17.59 g (0.054 mol) of Cs₂CO₃, and 150 mL of dimethylacetamide was refluxed at 180°C in a one neck round bottom flask. After completing the reaction to obtain a reaction product, a precipitated solid was obtained by filtering the reaction product. The solid was dissolved, filtered with a silica gel filter, and concentrated to obtain 13.54 g (61%) of Compound 1-281 (G).

Compound G below was synthesized in the same manner in Preparation Example 3 above except that other intermediates A, B, C, and D of Table 3 below were used.

**[Table 3]**

| Compound | A | β | C | D | G | Yie Id |
|---|---|---|---|---|---|---|
| 1-281 | 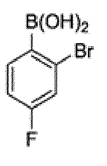 | 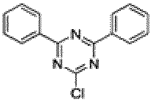 | 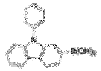 | 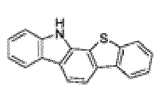 | 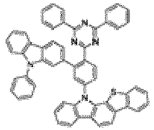 | 6 1 % |
| 1-284 | 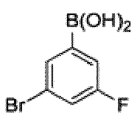 | 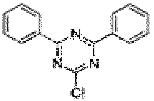 | 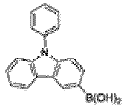 | 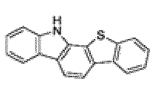 | 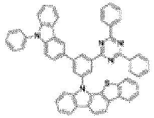 | 7 6 % |
| 1-288 | 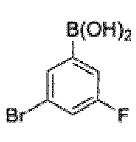 | 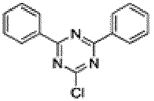 | 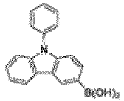 | 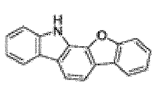 | 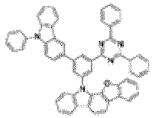 | 7 4 % |
| 1-292 | 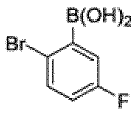 | 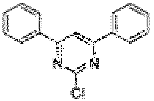 | 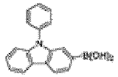 | 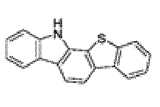 | 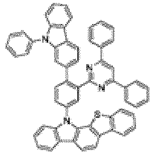 | 5 2 % |
| 1-294 | 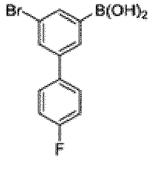 | 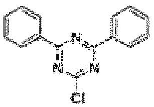 | 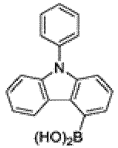 | 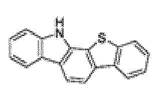 | 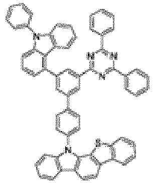 | 6 9 % |

### <Preparation Example 4> Synthesis of Compound 2-3 (H)

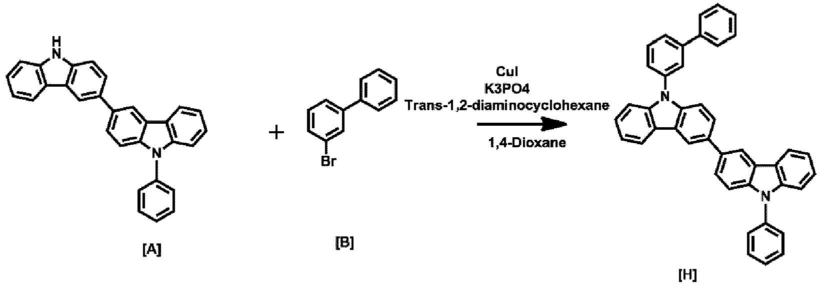

10 g (0.024 mol) of 9-phenyl-9H,9'H-3,3'-bicarbazole, 6.15 g (0.026 mol) of 3-bromo-1,1'-biphenyl, 4.57 g (0.024 mol) of CuI, 2.74 g (0.024 mol) of trans-1,4-diaminocyclohexane, and 10.19 g (0.048 mol) of K₃PO₄ were dissolved in 100 mL of 1,4-oxane, and then refluxed at 125°C for 8 hours in a one neck round bottom flask. After completing the reaction, distilled water and DCM were put at room temperature for extraction, the organic layer was dried with MgSO₄, and the solvent was removed by a rotary evaporator. The reaction product was purified by column chromatography (DCM:Hex=1:3) and recrystallized with methanol to obtain 11.84 g (85%) of Compound 2-3[H].

Compound H below was synthesized in the same manner in Preparation Example 4 above except that other intermediates A and B of Table 4 below were used.

**[Table 4]**

| Compound | A | B | H | Yield |
|---|---|---|---|---|
| 2-3 | 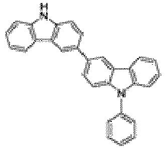 | 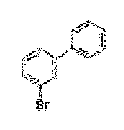 | 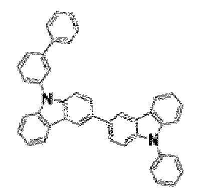 | 88% |
| 2-6 | 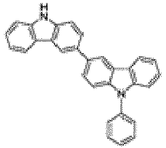 | 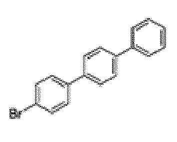 | 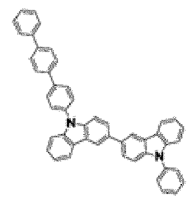 | 87% |
| 2-8 | 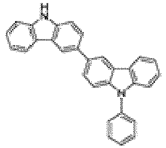 | 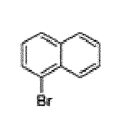 | 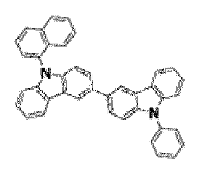 | 96% |
| 2-16 | 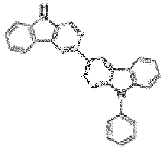 | 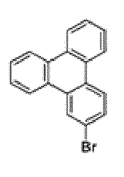 | 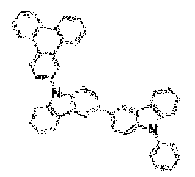 | 68% |
| 2-34 | 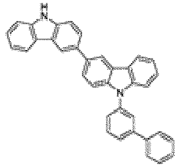 | 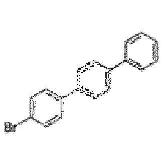 | 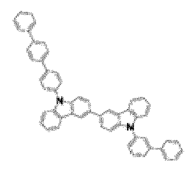 | 69% |
| 2-44 | 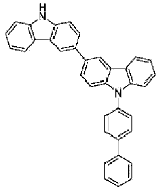 | 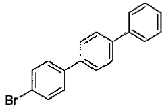 | 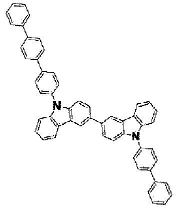 | 68% |
| 2-52 | 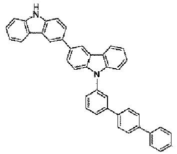 | 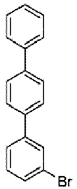 | 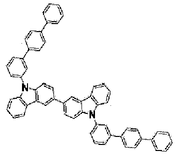 | 58% |
| 2-61 | 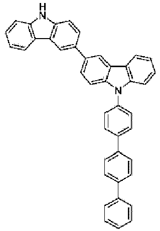 | 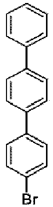 | 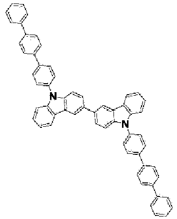 | 92% |
| 2-73 | 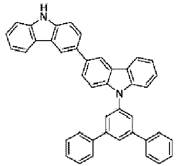 | 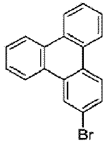 | 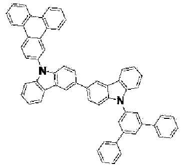 | 71% |

The heterocyclic compounds of Chemical Formulae 1 and 2 above that are the rest other than the compounds described in Preparation Examples 1 to 4 and Tables 1 to 4 above were also prepared in the same manner as described in the aforementioned Preparation Examples.

Tables 5 and 6 below are ¹H NMR data and FD-MS data of the synthesized compounds, and it can be confirmed that the desired compounds were synthesized through the data below.

**[Table 5]**

| Compound | ¹H NMR(DMSO, 300Mz) |
|---|---|
| 1-1 | δ = 8.36 (4H, m), 7.98-8.08 (7H, m), 7.76-7.83 (3H, m), 7.50-7.54 (9H, m), 7.31-7.39 (4H, m) |
| 1-3 | δ = 8.36 (4H, m), 7.98-8.08 (6H, m), 7.82-7.83 (2H, m), 7.69 (1H, d), 7.50-7.57 (10H, m), 7.31-7.39 (4H, m) |
| 1-4 | δ = 8.36 (4H, m), 7.98-8.15 (7H, m), 7.76-7.82 (2H, m), 7.50-7.54 (9H, m), 7.31-7.39 (5H, m) |
| 1-7 | δ = 8.36 (4H, m), 7.98-8.15 (7H, m), 7.76-7.82 (2H, m), 7.50-7.54 (9H, m), 7.31-7.39 (5H, m) |
| 1-9 | δ = 8.36 (4H, m), 7.98-8.15 (6H, m), 7.82 (1H, d), 7.69 (1H, d), 7.50-7.57 (10H, m), 7.31-7.39 (4H, m) |
| 1-15 | δ = 8.36 (4H, m), 7.98-8.15 (7H, m), 7.76-7.82 (2H, m), 7.50-7.54 (9H, m), 7.31-7.39 (4H, m) |
| 1-18 | δ = 8.36 (4H, m), 7.98-8.15 (7H, m), 7.76-7.82 (2H, m), 7.50-7.54 (9H, m), 7.31-7.39 (5H, m) |
| 1-20 | δ = 8.36 (4H, m), 8.13-8.15 (2H, m), 7.98-8.03 (3H, m), 7.76-7.82 (3H, m), 7.69 (1H, d), 7.50-7.57 (9H, m), 7.31-7.39 (5H, m) |
| 1-22 | δ = 8.36 (4H, m), 7.98-8.06 (5H, m), 7.79-7.88 (6H, m), 7.50-7.54 (8H, m), 7.31-7.39 (4H, m) |
| 1-26 | δ = 8.36 (4H, m), 8.13-8.15 (2H, m), 7.98-8.03 (3H, m), 7.76-7.88 (5H, m), 7.50-7.54 (8H, m), 7.31-7.39 (5H, m) |
| 1-28 | δ = 8.36 (4H, m), 7.98-8.15 (6H, m), 7.79-7.88 (3H, m), 7.50-7.54 (9H, m), 7.31-7.39 (5H, m) |
| 1-30 | δ = 8.36 (4H, m), 8.13-8.15 (2H, m), 7.98 (2H, d), 7.79-7.88 (4H, m), 7.69 (1H, d), 7.50-7.57 (9H, m), 7.31-7.39 (5H, m) |
| 1-34 | δ = 8.36 (4H, m), 7.98-8.15 (6H, m), 7.82 (1H, d), 7.69 (1H, d), 7.50-7.57 (10H, m), 7.31-7.39 (5H, m) |
| 1-35 | δ = 8.36 (4H, m), 8.13-8.15 (2H, m), 7.98-8.03 (3H, m), 7.76-7.82 (3H, m), 7.69 (1H, d), 7.50-7.57 (9H, m), 7.31-7.39 (5H, m) |
| 1-40 | δ = 8.36 (4H, m), 7.98-8.08 (6H, m), 7.82-7.83 (2H, m), 7.69 (1H, d), 7.50-7.54 (10H, m), 7.31-7.39 (4H, m) |
| 1-44 | δ = 8.36-8.38 (3H, m), 8.13-8.15 (2H, m), 7.94-7.98 (4H, m), 7.73-7.82 (7H, m), 7.31-7.61 (15H, m) |
| 1-45 | δ = 8.36 (2H, m), 8.13-8.15 (2H, m), 7.69-7.98 (11H, m), 7.31-7.57 (16H, m) |
| 1-46 | δ = 8.13-8.15 (2H, m), 7.69-7.98 (15H, m), 7.25-7.57 (18H, m) |
| 1-52 | δ = 8.23 (1H, s), 8.13-8.15 (2H, m), 7.94-8.03 (7H, m), 7.76-7.82 (3H, m), 7.69 (1H, d), 7.31-7.57 (14H, m) |
| 1-53 | δ = 8.36 (4H, m), 7.98-8.04 (8H, m), 7.76-7.82 (2H, m), 7.50-7.54 (9H, m), 7.31-7.39 (4H, m) |
| 1-54 | δ = 8.36 (4H, m), 7.98-8.04 (7H, m), 7.79-7.88 (3H, m), 7.50-7.54 (9H, m), 7.31-7.39 (4H, m) |
| 1-55 | δ = 8.36 (4H, m), 7.98-8.08 (7H, m), 7.82 (1H, d), 7.69 (1H, d), 7.50-7.57 (10H, m), 7.31-7.39 (4H, m) |
| 1-56 | δ = 8.36 (4H, m), 7.98-8.04 (6H, m), 7.76-7.83 (5H, m), 7.50-7.54 (8H, m), 7.31-7.39 (4H, m) |
| 1-60 | δ = 8.36 (4H, m), 7.98-8.15 (7H, m), 7.79-7.88 (3H, m), 7.50-7.54 (9H, m), 7.31-7.39 (4H, m) |
| 1-64 | δ = 8.36 (4H, m), 8.13-8.15 (2H, m), 7.98-8.06 (4H, m), 7.76-7.82 (3H, m), 7.69 (1H, d), 7.50-7.57 (9H, m), 7.31-7.39 (4H, m) |
| 1-71 | δ = 8.36 (2H, m), 8.13-8.15 (2H, m), 7.69-8.06 (12H, m), 7.25-7.57 (15H, m) |
| 1-73 | δ = 8.36 (4H, m), 7.96-8.08 (8H, m), 7.79-7.88 (4H, m), 7.50-7.54 (9H, m), 7.25-7.39 (6H, m) |
| 1-75 | δ = 8.36 (4H, m), 8.13-8.15 (2H, m), 7.96-7.98 (5H, m), 7.76-7.88 (5H, m), 7.50-7.54 (8H, m), 7.31-7.39 (7H, m) |
| 1-77 | δ = 8.36-8.38 (5H, m), 8.13-8.15 (2H, m), 7.69-7.98 (9H, m), 7.50-7.57 (10H, m), 7.31-7.39 (5H, m) |
| 1-78 | δ = 8.36-8.38 (5H, m), 7.94-8.04 (7H, m), 7.69-7.82 (5H, m), 7.50-7.57 (10H, m), 7.31-7.39 (4H, m) |
| 1-83 | δ = 8.55 (1H, d), 8.45 (2H, d), 8.32-8.36 (5H, m), 8.03-8.06 (3H, m), 7.93 (2H, m), 7.83 (1H, t), 7.68-7.70 (2H, t), 7.49-7.56 (10H, m) |
| 1-84 | δ = 8.55 (1H, d), 8.45 (2H, d), 8.32-8.36 (5H, m), 8.13-8.24 (5H, m), 7.93 (2H, d), 7.70 (1H, t), 7.49-7.56 (10H, m), 7.35 (1H, d) |
| 1-87 | δ = 8.55 (1H, d), 8.45 (2H, d), 8.32-8.36 (5H, m), 8.13-8.24 (5H, m), 7.93 (2H, d), 7.70 (1H, t), 7.49-7.56 (10H, m), 7.35 (1H, d) |
| 1-89 | δ = 8.55 (1H, d), 8.45 (2H, d), 8.32-8.36 (5H, m), 8.13-8.15 (2H, m), 8.03 (1H, d), 7.93-7.94 (3H, m), 7.68-7.70 (2H, m), 7.49-7.56 (10H, m), 7.35 (1H, d) |
| 1-98 | δ = 8.55 (1H, d), 8.45 (2H, d), 8.32-8.36 (5H, m), 8.15-8.24 (5H, m), 7.93 (2H, d), 7.70 (1H, t), 7.49-7.56 (10H, m), 7.35 (1H, d) |
| 1-99 | δ = 8.45 (2H, d), 8.36 (4H, m), 8.12-8.24 (7H, m), 7.93-7.99 (3H, m), 7.49-7.56 (10H, m), 7.35 (1H, d) |
| 1-100 | δ = 8.45 (2H, d), 8.36 (4H, m), 8.13-8.24 (5H, m), 8.03 (1H, d), 7.93-7.94 (3H, m), 7.68 (1H, t), 7.49-7.56 (10H, m), 7.35 (1H, d) |
| 1-102 | δ = 8.45 (2H, d), 8.36 (4H, m), 7.93-8.24 (10H, m), 7.83 (1H, t), 7.49-7.56 (10H, m) |
| 1-110 | δ = 8.45 (2H, d), 8.36 (4H, m), 8.12-8.15 (4H, m), 7.94-8.03 (5H, m), 7.69 (1H, t), 7.49-7.56 (10H, m), 7.35 (1H, d) |
| 1-125 | δ = 8.45 (2H, d), 8.36 (2H, m), 8.12-8.15 (4H, m), 7.93-8.03 (7H, m), 7.68-7.75 (3H, m), 7.35-7.56 (11H, m), 7.25 (2H, d) |
| 1-128 | δ = 8.45 (2H, d), 8.12-8.15 (4H, m), 7.93-7.99 (8H, m), 7.41-7.75 (18H, m), 7.25 (2H, d) |
| 1-135 | δ = 8.55 (1H, d), 8.45 (2H, d), 8.32-8.36 (5H, m), 8.04 (3H, m), 7.93-7.94 (3H, m), 7.68-7.70 (2H, m), 7.49-7.56 (10H, m) |
| 1-136 | δ = 8.45 (2H, d), 8.36 (4H, m), 8.12-8.24 (5H, m), 7.93-8.04 (6H, m), 7.49-7.56 (10H, m) |
| 1-137 | δ = 8.45 (2H, d), 8.36 (4H, m), 8.12-8.24 (5H, m), 7.93-8.04 (6H, m), 7.49-7.56 (10H, m) |
| 1-138 | δ = 8.45 (2H, d), 8.36 (4H, m), 8.12 (2H, m), 7.93-8.0 (8H, m), 7.68 (1H, t), 7.49-7.56 (10H, m) |
| 1-155 | δ = 8.45 (1H, d), 8.36 (4H, m), 8.12-8.24 (7H, m), 7.93-7.99 (5H, m), 7.49-7.56 (10H, m), 7.35 (1H, d), 7.25 (2H, d) |
| 1-158 | δ = 8.36-8.45 (7H, m), 8.17-8.24 (3H, m), 8.03-8.04 (4H, m), 7.93-7.94 (4H, m), 7.49-7.68 (13H, m) |
| 1-163 | δ = 8.45 (1H, d), 8.36 (4H, m), 7.93-8.08 (8H, m), 7.83 (1H, t), 7.68 (1H, t), 7.49-7.56 (10H, m), 7.31-7.39 (2H, m) |
| 1-165 | δ = 8.45 (1H, d), 8.36 (4H, m), 7.93-8.12 (9H, m), 7.49-7.56 (10H, m), 7.31-7.39 (3H, m) |
| 1-178 | δ = 8.55 (1H, d), 8.45 (1H, d), 8.32-8.36 (5H, m), 8.13-8.15 (2H, m), 7.93-8.03 (3H, m), 7.70-7.82 (3H, m), 7.49-7.56 (10H, m), 7.31-7.39 (3H, m) |
| 1-180 | δ = 8.45 (1H, d), 8.36 (4H, m), 8.13-8.15 (2H, m), 7.93-8.03 (5H, m), 7.76-7.82 (2H, m), 7.68 (1H, t), 7 .49-7 .54 (9H, m), 7 .31-7 .39 (3H, m) |
| 1-186 | δ = 8.45 (1H, d), 8.36 (4H, m), 8.13-8.24 (5H, m), 7.79-7.98 (5H, m), 7.49-7.56 (9H, m), 7.31-7.39 (3H, m) |
| 1-190 | δ = 8.45 (1H, d), 8.36 (4H, m), 8.13-8.15 (2H, m), 7.79-8.03(6H, m), 7.68 (1H, t), 7.49-7.54 (9H, m), 7.31-7.39 (3H, m) |
| 1-199 | δ = 8.45 (1H, d), 8.36 (4H, m), 8.12-8.15 (4H, m), 7.93-7.99 (3H, m), 7.82 (1H, d), 7.69 (1H, d), 7 .49-7 .57 (10H, m), 7 .31-7 .39 (3H, m) |
| 1-200 | δ = 8.55 (1H, d), 8.45 (1H, d), 8.32-8.36 (5H, m), 8.06 (2H, d), 7.93-7.98 (2H, m), 7.82-7.83 (2H, m), 7.69-7.70 (2H, m), 7.49-7.54 (10H, m), 7.31-7.39 (2H, m) |
| 1-204 | δ = 8.36-8.45 (3H, m), 8.13-8.24 (5H, m), 7.93-7.98 (3H, m), 7.69-7.82 (5H, m), 7.31-7.61 (13H, m) |
| 1-209 | δ = 8.36-8.45 (3H, m), 8.12-8.15 (4H, m), 7.93-7.99 (4H, m), 7.69-7.82 (5H, m), 7.31-7.61 (14H, m) |
| 1-213 | δ = 8.45 (1H, d), 8.36 (4H, m), 8.17-8.24 (3H, m), 7.93-8.04 (7H, m), 7.50-7.56 (10H, m), 7.31-7.39 (2H, m) |
| 1-215 | δ = 8.45 (1H, d), 8.36 (4H, m), 7.93-8.08 (9H, m), 7.68 (1H, t), 7.49-7.56 (10H, m), 7.31-7.39 (2H, m) |
| 1-216 | δ = 8.45 (1H, d), 8.36 (4H, m), 8.12 (2H, m), 7.93-8.04 (7H, m), 7.76-7.82 (2H, m), 7.49-7.56 (9H, m), 7.31-7.39 (2H, m) |
| 1-219 | δ = 8.55 (1H, d), 8.45 (1H, d), 8.32-8.36 (5H, m), 8.13-8.15 (2H, m), 7.93-8.06 (4H, m), 7.76-7.82 (3H, m), 7.49-7.56 (9H, m), 7.31-7.39 (2H, m) |
| 1-232 | δ = 8.36-8.45 (4H, m), 8.12 (2H, m), 7.93-8.04 (7H, m), 7.73-7.82 (5H, m), 7.31-7.57 (12H, m) |
| 1-236 | δ = 8.45 (1H, d), 8.36 (4H, m), 8.12 (2H, m), 7.93-8.04 (8H, m), 7.82 (1H, d), 7.69 (1H, d), 7.49-7.57 (10H, m), 7.25-7.39 (4H, m) |
| 1-238 | δ = 8.36-8.45 (6H, m), 8.17-8.24 (3H, m), 7.93-8.04 (6H, m), 7.82 (1H, d), 7.69-7.73 (2H, m), 7.50-7.61 (11H, m), 7.31-7.39 (2H, m) |
| 1-240 | δ = 8.45 (1H, d), 8.36 (4H, m), 8.12-8.15 (4H, m), 7.93-7.99 (5H, m), 7.82 (1H, d), 7.69 (1H, d), 7.49-7.57 (10H, m), 7.25-7.39 (5H, m) |
| 1-242 | δ = 8.55 (2H, d), 8.36 (4H, m), 8.19 (2H, d), 8.05 (1H, d), 7.88-7.94 (3H, m), 7.69 (1H, s), 7.50-7.58 (10H, m), 7.35 (2H, t), 7.16-7.20 (4H, m) |
| 1-243 | δ = 8.55 (2H, d), 8.16-8.36 (5H, m), 8.19 (2H, d), 8.06 (1H, d), 7.94-7.97 (3H, m), 7.50-7.58 (10H, m), 7.35 (2H, t), 7.16-8.36 (4H, m) |
| 1-244 | δ = 8.55 (2H, d), 8.36 (4H, m), 8.19 (2H, d), 7.88-7.94 (4H, m), 7.73 (1H, t), 7.50-7.58 (10H, m), 7.35 (2H, t), 7.16-7.20 (3H, m) |
| 1-249 | δ = 8.55 (2H, d), 8.36 (4H, m), 8.19 (1H, d), 7.84-7.98 (6H, m), 7.69 (1H, s), 7.50-7.58 (9H, m), 7.31-7.39 (4H, m), 7.13-7.20 (4H, m) |
| 1-253 | δ = 8.55 (1H, d), 8.45 (1H, d), 8.36 (4H, m0, 8.19 (1H, d), 8.05 (1H, d), 7.86-7.94 (5H, m), 7.70 (1H, s), 7.69 (1H, s), 7.49-7.58 (10H, m), 7.35 (2H, t), 7.16-7.20 (3H, m) |
| 1-255 | δ = 8.55 (2H, d), 8.45 (1H, d), 8.36 (4H, m), 8.19 (1H, d), 8.05 (2H, d), 7.88-7.94 (4H, m), 7.69 (1H, s), 7.49-7.58 (10H, m), 7.33-7.35 (3H, m), 7.16-7.20 (3H, m) |
| 1-258 | δ = 8.55 (2H, d), 8.45 (1H, d), 8.36 (4H, m), 8.19 (1H, d), 8.05 (2H, d), 7.88-7.94 (4H, m), 7.69 (1H, s), 7.49-7.60 (11H, m), 7.35 (2H, t), 7.16-7.20 (3H, m) |
| 1-259 | δ = 8.55 (2H, d), 8.45 (1H, d), 8.36 (4H, m), 8.19 (1H, d), 7.86-7.94 (6H, m), 7.73-7.78 (2H, m), 7.50-7.58 (10H, m), 7.35 (2H, t), 7.16-7.20 (3H, m) |
| 1-260 | δ = 8.55 (2H, d), 8.36 (4H, m), 8.19-8.24 (2H, m), 8.05 (1H, d), 7.88-7.94 (4H, m), 7.69-7.74 (2H, m), 7.49-7.57 (9H, m), 7.35-7.38 (3H, t), 7.16-7.20 (3H, t), 1.69 (6H, s) |
| 1-261 | δ = 8.55 (2H, d), 8.35 (1H, s), 8.19-8.24 (3H, m), 8.06 (1H, d), 7.88-7.97 (8H, m), 7.74 (1H, d), 7.49-7.58 (10H, m), 7.35-7.38 (3H, t), 7.16-7.20 (3H, t), 1.69 (6H, s) |
| 1-262 | δ = 8.55 (2H, d), 8.36 (4H, m), 8.19-8.24 (2H, t), 7.88-7.94 (5H, m), 7.73-7.74 (2H, m), 7.49-7.58 (9H, m), 7.16-7.38 (3H, m), 7.16-7.20 (2H, m), 1.69 (6H, s) |
| 1-263 | δ = 8.55 (2H, d), 8.36 (4H, m), 8.19-8.24 (3H, m), 8.05 (1H, d), 7.88-7.94 (3H, m), 7.69-7.74 (3H, m), 7.50-7.58 (9H, m), 7.35-7.38 (2H, m), 7.16-7.20 (2H, m), 1.69 (6H, s) |
| 1-265 | δ = 8.55 (2H, d), 8.36 (4H, m), 8.19 (1H, d), 8.05 (1H, d), 7.88-7.98 (4H, m), 7.69 (1H, s), 7.49-7.58 (10H, m), 7.31-7.42 (5H, m), 7.16-7.20 (3H, m) |
| 1-267 | δ = 8.62 (2H, d), 8.36 (4H, m), 8.13-8.22 (6H, m), 7.74 (2H, s), 7 .50-7 .62 (19H, m), 7.35 (1H, d), 7.20 (2H, t) |
| 1-271 | δ = 8.62 (2H, d), 8.36 (4H, m), 8.19-8.22 (3H, m), 7.98-8.04 (4H, m), 7.84 (1H, d), 7.74 (2H, s), 7.50-7.62 (19H, m), 7.31-7.39 (2H, m), 7.20 (1H, t) |
| 1-272 | δ = 8.62 (1H, d), 8.30-8.36 (5H, m), 8.13-8.22 (5H, m), 7.89 (1H, s), 7.7 (1H, s), 7 .50-7 .62 (20H, m), 7.35 (1H, d), 7.20 (2H, t) |
| 1-274 | δ = 8.62 (1H, d), 8.30-8.36 (5H, m), 8.13-8.22 (3H, m), 7.98-8.04 (4H, m), 7.84-7.89 (2H, m), 7.74 (1H, s), 7.50-7.62 (19H, m), 7.31-7.39 (2H, m), 7.13-7.20 (2H, m) |
| 1-278 | δ = 8.62 (1H, d), 8.36 (4H, m), 8.13-8.22 (3H, m), 8.04 (3H, m), 7.84-7.89 (2H, m), 7.74 (1H, s), 7.50-7.62 (19H, m), 7.31-7.39 (2H, m), 7.13-7.20 (2H, m) |
| 1-280 | δ = 8.65 (1H, d), 8.36-8.42 (5H, m), 8.36-8.42 (5H, m), 8.13-8.22 (5H, m), 8.04 (1H, d), 7.50-7.62 (22H, m), 7.35 (1H, d), 7.20 (2H, t) |
| 1-281 | δ = 8.62 (1H, d), 8.55 (1H, d), 8.36-8.45 (6H, m), 8.19-8.22 (2H, m), 8.02-8.10 (3H, m), 7.93-7.94 (2H, m), 7.74 (1H, s), 7.49-77.62 (16H, m), 7.35 (1H, t), 7.16-7.20 (2H, m) |
| 1-284 | δ = 8.55 (1H, d), 8.45 (1H, d), 8.30-8.36 (6H, m), 8.13-8.19 (2H, m), 8.05 (1H, d), 7.89-7.94 (4H, m), 7.49-7.62 (16H, m), 7.35 (1H, t), 7.20 (1H, m) |
| 1-288 | δ = 8.55 (1H, d), 8.30-8.36 (7H, m), 8.13-8.19 (2H, m), 7.84-7.94 (5H, m), 7.48-7.62 (15H, m), 7.31-7.39 (3H, m), 7.16-7.20 (2H, m) |
| 1-289 | δ = 8.55 (2H, d), 8.36 (4H, m), 8.19 (2H, d), 8.05 (1H, d), 7.94-7.96 (5H, m), 7.69 (1H, s), 7.50-7.58 (10H, m), 7.35 (2H, t), 7.16-7.25 (5H, m) |
| 1-291 | δ = 8.55 (1H, d), 8.35-8.38 (4H, m), 8.19 (2H, d), 8.06 (1H, d), 7.94-7.97 (6H, m), 7.50-7.75 (3H, m), 7.35-7.61 (13H, m), 7.16-7.25 (6H, m) |
| 1-292 | δ = 8.55-8.62 (2H, m), 8.40-8.45 (2H, m), 8.19-8.23 (3H, m), 7.93-8.10 (9H, m), 7.74 (1H, s), 7.49-7.62 (16H, m), 7.35 (1H, t), 7.16-7.20 (2H, m) |
| 1-294 | δ = 8.55 (1H, d), 8.45 (1H, d), 8.36 (4H, m), 8.19-8.22 (2H, m), 8.04-8.05 (5H, m), 7.91-7.94 (6H, m), 7.49-7.62 (17H, m), 7.35 (1H, t), 7.16-7.20 (2H, m) |
| 1-301 | No ¹H NMR peak with 100% deuterium content |
| 1-303 | No ¹H NMR peak with 100% deuterium content |
| 2-3 | δ = 8.55 (1H, d), 8.30 (1H, d), 8.13-8.21 (3H, m), 7.89-7.99 (4H, m), 7.35-7.68 (17H, m), 7.16-7.20 (2H, m) |
| 2-6 | δ = 8.55 (1H, d), 8.30 (1H, d), 8.13-8.19 (2H, m), 7.89-7.99 (8H, m), 7.75-7.77 (3H, m), 7.35-7.62 (11H, m), 7.16-7.25 (6H, m) |
| 2-8 | δ = 8.55 (1H, d), 8.30 (1H, d), 8.11-8.19 (5H, m), 7.89-7.99 (4H, m), 7.77-7.79 (2H, m), 7.50-7.62 (10H, m), 7.35 (1H, t), 7.16-7.20 (2H, m) |
| 2-16 | δ = 9.05 (1H, s), 8.55 (1H, d), 8.13-8.33 (7H, m), 7.89-7.99 (5H, m), 7.50-7.77 (13H, m), 7.35 (1H, t), 7.16-7.20 (2H, m) |
| 2-34 | δ = 8.55 (1H, d), 8.30 (1H, d), 8.13-8.21 (3H, m), 7.89-7.99 (8H, m), 7.35-7.77 (17H, m), 7.16-7.25 (6H, m) |
| 2-44 | δ = 8.55 (1H, d), 8.30 (1H, d), 8.13-8.19 (2H, m), 7.89-7.99 (12H, m), 7.75-7.77 (5H, m), 7.58 (1H, d), 7.35-7.50 (8H, m), 7.16-7.25 (6H, m) |
| 2-52 | δ = 8.55 (1H, d), 8.30 (1H, d), 8.13-8.21 (4H, m), 7.89-7.99 (4H, m), 7.35-7.77 (20H, m), 7.16-7.52 (10H, m) |
| 2-61 | δ = 8.55 (1H, d), 8.30 (1H, d), 8.13-8.19 (2H, m), 7.89-7.99 (12H, m), 7.75-7.77 (5H, m), 7.58 (1H, d), 7.35-7.50 (8H, m), 7.16-7.25 (10H, m) |
| 2-73 | δ = 9.05 (1H, s), 8.55 (1H, d), 8.19-8.33 (9H, m), 7.89-7.99 (6H, m), 7.35-7.77 (19H, m), 7.16-7.20 (2H, m) |

**[Table 6]**

| Compound | FD-Mass | Compound | FD-Mass |
|---|---|---|---|
| 1-1 | m/z= 641.73 (C45H27N302=641.21) | 1-3 | m/z= 641.73 (C45H27N3O2=641.21) |
| 1-4 | m/z= 641.73 (C45H27N3O2=641.21) | 1-7 | m/z= 641.73 (C45H27N3O2=641.21) |
| 1-9 | m/z= 641.73 (C45H27N3O2=641.21) | 1-15 | m/z= 641.73 (C45H27N3O2=641.21) |
| 1-18 | m/z= 641.73 (C45H27N3O2=641.21) | 1-20 | m/z= 641.73 (C45H27N3O2=641.21) |
| 1-22 | m/z= 641.73 (C45H27N3O2=641.21) | 1-26 | m/z= 641.73 (C45H27N3O2=641.21) |
| 1-28 | m/z= 641.73 (C45H27N3O2=641.21) | 1-30 | m/z= 641.73 (C45H27N3O2=641.21) |
| 1-34 | m/z= 641.73 (C45H27N3O2=641.21) | 1-35 | m/z= 641.73 (C45H27N3O2=641.21) |
| 1-40 | m/z= 641.73 (C45H27N3O2=641.21) | 1-44 | m/z= 717.83 (C51H31N3O2=712.24) |
| 1-45 | m/z= 717.83 (C51H31N3O2=712.24) | 1-46 | m/z= 793.93 (C57H35N3O2=793.27) |
| 1-52 | m/z= 640.84 (C46H28N2O2=640.22) | 1-53 | m/z= 641.73 (C45H27N3O2=641.21) |
| 1-54 | m/z= 641.73 (C45H27N3O2=641.21) | 1-55 | m/z= 641.73 (C45H27N3O2=641.21) |
| 1-60 | m/z= 641.73 (C45H27N3O2=641.21) | 1-64 | m/z= 641.73 (C45H27N3O2=641.21) |
| 1-71 | m/z= 717.83 (C51H31N3O2=717.24) | 1-73 | m/z= 717.83 (C51H31N3O2=717.24) |
| 1-75 | m/z= 717.83 (C51H31N3O2=717.24) | 1-77 | m/z= 717.83 (C51H31N3O2=717.24) |
| 1-78 | m/z= 717.83 (C51H31N3O2=717.24) | 1-83 | m/z= 673.85 (C45H27N3S2=673.16) |
| 1-84 | m/z= 673.85 (C45H27N3S2=673.16) | 1-87 | m/z= 673.85 (C45H27N3S2=673.16) |
| 1-89 | m/z= 673.85 (C45H27N3S2=673.16) | 1-98 | m/z= 673.85 (C45H27N3S2=673.16) |
| 1-99 | m/z= 673.85 (C45H27N3S2=673.16) | 1-100 | m/z= 673.85 (C45H27N3S2=673.16) |
| 1-102 | m/z= 673.85 (C45H27N3S2=673.16) | 1-110 | m/z= 673.85 (C45H27N3S2=673.16) |
| 1-125 | m/z= 749.95 (C51H31N3S2=749.20) | 1-128 | m/z= 826.05 (C57H35N3S2=825.23) |
| 1-135 | m/z= 673.85 (C45H27N3S2=673.16) | 1-136 | m/z= 673.85 (C45H27N3S2=673.16) |
| 1-137 | m/z= 673.85 (C45H27N3S2=673.16) | 1-138 | m/z= 673.85 (C45H27N3S2=673.16) |
| 1-155 | m/z= 749.95 (C51H31N3S2=749.20) | 1-158 | m/z= 749.95 (C51H31N3S2=749.20) |
| 1-163 | m/z= 657.79 (C45H27N3OS=657.19) | 1-165 | m/z= 657.79 (C45H27N30S=657.19) |
| 1-178 | m/z= 657.79 (C45H27N30S=657.19) | 1-180 | m/z= 657.79 (C45H27N30S=657.19) |
| 1-186 | m/z= 657.79 (C45H27N30S=657.19) | 1-190 | m/z= 657.79 (C45H27N30S=657.19) |
| 1-199 | m/z= 657.79 (C45H27N30S=657.19) | 1-200 | m/z= 657.79 (C45H27N30S=657.19) |
| 1-204 | m/z= 733.89 (C51H31N3OSS=733.22) | 1-209 | m/z= 733.89 (C51H31N3OSS=733.22) |
| 1-213 | m/z= 657.79 (C45H27N30S=657.19) | 1-214 | m/z= 657.79 (C45H27N30S=657.19) |
| 1-215 | m/z= 657.79 (C45H27N30S=657.19) | 1-216 | m/z= 657.79 (C45H27N30S=657.19) |
| 1-219 | m/z= 657.79 (C45H27N30S=657.19) | 1-232 | m/z= 733.89 (C51H31N3OSS=733.22) |
| 1-236 | m/z= 733.89 (C51H31N3OSS=733.22) | 1-238 | m/z= 733.89 (C51H31N3OSS=733.22) |
| 1-240 | m/z= 733.89 (C51H31N3OSS=733.22) | 1-242 | m/z= 639.76 (C45H29N5=639.24) |
| 1-243 | m/z= 639.76 (C45H29N5=639.24) | 1-244 | m/z= 639.76 (C45H29N5=639.24) |
| 1-249 | m/z= 729.84 (C51H31N5O=729.25) | 1-253 | m/z= 745.90 (C51H31N5S-745.23) |
| 1-255 | m/z= 745.90 (C51H31N5S-745.23) | 1-258 | m/z= 745.90 (C51H31N5S-745.23) |
| 1-259 | m/z= 745.90 (C51H31N5S=745.23) | 1-260 | m/z= 755.93 (C54H37N5=755.30) |
| 1-261 | m/z= 754.94 (C55H38N4=754.31) | 1-262 | m/z= 755.93 (C54H37N5=755.30) |
| 1-263 | m/z= 755.93 (C54H37N55-755.30) | 1-265 | m/z= 729.84 (C51H31N5O=729.25) |
| 1-267 | m/z= 791.96 (C57H37N5=791.30) | 1-271 | m/z= 882.04 (C63H39N50=881.32) |
| 1-272 | m/z= 791.96 (C57H7N5=791.30) | 1-274 | m/z= 882.04 (C63H39N50=881.32) |
| 1-278 | m/z= 791.96 (C57H37N5=791.30) | 1-280 | m/z= 791.96 (C57H37N5=791.30) |
| 1-281 | m/z= 822.00 (C57H35N5S=821.26) | 1-284 | m/z= 822.00 (C57H35N5S=821.26) |
| 1-288 | m/z= 805.94 (C57H35N5O=805.28) | 1-289 | m/z= 715.86 (C51H33N5=715.27) |
| 1-291 | m/z= 791.96 (C57H37N5=791.30) | 1-292 | m/z= 821.01 (C58H36N4S=820.27) |
| 1-294 | m/z= 898.10 (C63H39N5S=897.29) | 1-301 | m/z= 668.89 (C45D27N3O2=668.38) |
| 1-303 | m/z= 684.96 (C45D27N3OS=684.36) | 2-3 | m/z= 560.70 (C42H28N2=560.23) |
| 2-6 | m/z= 636.80 (C48H32N2=636.26) | 2-8 | m/z= 534.66 (C40H26N2=534.21) |
| 2-16 | m/z= 634.78 (C48H30N2=634.24) | 2-34 | m/z= 712.90 (C54H36N2=712.29) |
| 2-44 | m/z= 712.90 (C54H36N2=712.29) | 2-52 | m/z= 788.99 (C60H40N2=788.32) |
| 2-61 | m/z= 788.99 (C60H40N2=788.32) | 2-73 | m/z= 786.98 (C60H38N2=786.30) |

### <Experimental Example 1> - Fabrication of Organic Light Emitting Devices

Glass substrates coated with a thin film of ITO to a thickness of 1,500 Å were washed with distilled water and ultrasonic waves. After washing the substrates with distilled water, ultrasonic cleaning was performed on the washed substrates with solvents such as acetone, methanol, isopropyl alcohol, etc., the ultrasonic cleaned substrates were dried, and UVO-treated for 5 minutes using UV in a UV cleaner. Thereafter, after transferring the substrates to a plasma cleaner (PT), plasma treatment was performed in order to remove the ITO work function and residual film in a vacuum state, and then the substrates were transferred to thermal deposition equipment for organic deposition.

As common layers on the ITO transparent electrodes (anodes), a hole injection layer 2-TNATA (4,4',4"-Tris[2-naphthyl(phenyl)amino]triphenylamine) and a hole transport layer NPB (N,N'-Di (1-naphthyl) -N,N'-diphenyl- (1, 1'-biphenyl) -4,4'-diamine) were formed.

A light emitting layer was deposited thereon by thermal vacuum deposition as follows. The light emitting layer was deposited to a thickness of 400 Å with the compounds shown in Table 7 below as a host, and the green phosphorescent dopant was deposited by doping Ir(ppy)₃ to a thickness corresponding to 7% of the deposition thickness of the light emitting layer. Thereafter, BCP was deposited to a thickness of 60 Å as a hole blocking layer, and Alq₃ was deposited to a thickness of 200 Å thereon as an electron transport layer. Finally, after depositing lithium fluoride (LiF) to a thickness of 10 Å on the electron transport layer to form an electron injection layer, organic electroluminescent devices were manufactured by depositing an aluminum (Al) cathode to a thickness of 1,200 Å on the electron injection layer to form cathodes.

Meanwhile, all organic compounds required for fabricating OLED devices were vacuum sublimated and purified under 10⁻⁶ to 10⁻⁸ torr for each material respectively, and used for OLED fabrication.

Electroluminescence (EL) characteristics were measured for the organic electroluminescent devices fabricated as described above with M7000 of McScience, and T₉₀ values were measured with the measurement results when the reference luminance was 6,000 cd/m² through the device lifespan measuring system (M6000) manufactured by McScience. The results were as shown in Table 7 below.

**[Table 7]**

| | Compound | Driving voltage (V) | Efficiency (cd/A) | Color coordinates (x, y) | Lifespan (T₉₀) |
|---|---|---|---|---|---|
| Comparative Example 1 | A | 6.13 | 43.2 | (0.250, 0.681) | 57 |
| Comparative Example 2 | B | 5.58 | 45.0 | (0.244, 0.672) | 70 |
| Comparative Example 3 | C | 5.62 | 44.8 | (0.242, 0.675) | 68 |
| Comparative Example 4 | D | 5.83 | 44.2 | (0.275, 0.680) | 62 |
| Comparative Example 5 | E | 6.43 | 42.5 | (0.244, 0.675) | 59 |
| Comparative Example 6 | F | 6.23 | 43.9 | (0.245, 0.676) | 56 |
| Example 1 | 1-1 | 3.95 | 73.6 | (0.275, 0.680) | 119 |
| Example 2 | 1-3 | 3.62 | 74.8 | (0.280, 0.685) | 115 |
| Example 3 | 1-4 | 3.67 | 73.9 | (0.274, 0.674) | 106 |
| Example 4 | 1-7 | 3.68 | 73.5 | (0.259, 0.682) | 121 |
| Example 5 | 1-9 | 3.55 | 70.8 | (0.245, 0.657) | 98 |
| Example 6 | 1-15 | 3.82 | 75.9 | (0.244, 0.675) | 117 |
| Example 7 | 1-18 | 3.92 | 76.3 | (0.238, 0.687) | 113 |
| Example 8 | 1-20 | 3.97 | 73.5 | (0.270, 0.656) | 99 |
| Example 9 | 1-22 | 3.93 | 74.6 | (0.248, 0.674) | 105 |
| Example 10 | 1-26 | 4.17 | 75.0 | (0.274, 0.676) | 107 |
| Example 11 | 1-28 | 4.26 | 78.2 | (0.244, 0.670) | 122 |
| Example 12 | 1-30 | 4.28 | 73.8 | (0.243, 0.674) | 101 |
| Example 13 | 1-34 | 4.25 | 71.8 | (0.286, 0.675) | 123 |
| Example 14 | 1-35 | 4.13 | 76.3 | (0.240, 0.652) | 104 |
| Example 15 | 1-40 | 4.10 | 72.8 | (0.263, 0.643) | 109 |
| Example 16 | 1-44 | 3.75 | 76.5 | (0.238, 0.663) | 118 |
| Example 17 | 1-45 | 3.72 | 70.6 | (0.246, 0.684) | 106 |
| Example 18 | 1-46 | 3.70 | 72.0 | (0.275, 0.665) | 113 |
| Example 19 | 1-52 | 3.68 | 76.5 | (0.240, 0.656) | 120 |
| Example 20 | 1-53 | 3.62 | 77.2 | (0.245, 0.680) | 109 |
| Example 21 | 1-54 | 4.12 | 78.0 | (0.285, 0.665) | 100 |
| Example 22 | 1-55 | 4.15 | 79.3 | (0.274, 0.670) | 105 |
| Example 23 | 1-56 | 3.77 | 74.5 | (0.258, 0.682) | 121 |
| Example 24 | 1-60 | 4.26 | 79.3 | (0.240, 0.667) | 113 |
| Example 25 | 1-64 | 4.12 | 74.6 | (0.258, 0.670) | 109 |
| Example 26 | 1-71 | 3.94 | 75.6 | (0.244, 0.656) | 115 |
| Example 27 | 1-73 | 3.58 | 71.3 | (0.244, 0.674) | 120 |
| Example 28 | 1-75 | 3.87 | 72.5 | (0.245, 0.664) | 108 |
| Example 29 | 1-77 | 4.13 | 80.2 | (0.276, 0.670) | 103 |
| Example 30 | 1-78 | 4.30 | 83.5 | (0.245, 0.655) | 110 |
| Example 31 | 1-83 | 5.55 | 51.3 | (0.263, 0.643) | 87 |
| Example 32 | 1-84 | 5.39 | 52.2 | (0.254, 0.576) | 91 |
| Example 33 | 1-87 | 5.27 | 54.3 | (0.254, 0.660) | 90 |
| Example34 | 1-89 | 5.38 | 58.2 | (0.240, 0.774) | 86 |
| Example 35 | 1-98 | 5.22 | 50.9 | (0.280, 0.655) | 74 |
| Example 36 | 1-99 | 5.23 | 60.3 | (0.242, 0.652) | 86 |
| Example 37 | 1-100 | 5.13 | 58.3 | (0.262, 0.643) | 88 |
| Example 38 | 1-102 | 5.59 | 54.9 | (0.275, 0.682) | 73 |
| Example 39 | 1-110 | 5.08 | 53.6 | (0.254, 0.670) | 76 |
| Example 40 | 1-125 | 5.19 | 55.1 | (0.244, 0.675) | 78 |
| Example 41 | 1-128 | 5.33 | 52.4 | (0.272, 0.687) | 91 |
| Example 42 | 1-135 | 5.26 | 57.6 | (0.284, 0.675) | 75 |
| Example 43 | 1-136 | 5.18 | 54.6 | (0.270, 0.671) | 76 |
| Example 44 | 1-137 | 5.15 | 55.3 | (0.260, 0.643) | 82 |
| Example 45 | 1-138 | 5.67 | 52.7 | (0.275, 0.681) | 77 |
| Example 46 | 1-155 | 5.58 | 58.6 | (0.254, 0.670) | 86 |
| Example 47 | 1-158 | 5.61 | 60.2 | (0.240, 0.675) | 92 |
| Example 48 | 1-163 | 4.56 | 67.2 | (0.272, 0.685) | 76 |
| Example 49 | 1-165 | 4.28 | 68.5 | (0.286, 0.672) | 77 |
| Example 50 | 1-178 | 4.58 | 64.4 | (0.250, 0.671) | 78 |
| Example 51 | 1-180 | 4.29 | 65.2 | (0.241, 0.660) | 91 |
| Example 52 | 1-186 | 4.76 | 66.4 | (0.257, 0.650) | 95 |
| Example 53 | 1-190 | 4.80 | 62.3 | (0.240, 0.654) | 92 |
| Example 54 | 1-199 | 4.79 | 61.7 | (0.244, 0.670) | 86 |
| Example 55 | 1-200 | 4.68 | 72.3 | (0.240, 0.654) | 88 |
| Example 56 | 1-204 | 4.38 | 71.6 | (0.276, 0.650) | 83 |
| Example 57 | 1-209 | 4.25 | 64.5 | (0.245, 0.657) | 99 |
| Example 58 | 1-213 | 4.18 | 60.9 | (0.253, 0.643) | 92 |
| Example 59 | 1-214 | 4.99 | 67.1 | (0.254, 0.566) | 94 |
| Example 60 | 1-215 | 4.69 | 63.5 | (0.262, 0.687) | 96 |
| Example 61 | 1-216 | 4.58 | 65.7 | (0.284, 0.655) | 90 |
| Example 62 | 1-219 | 4.49 | 62.6 | (0.271, 0.673) | 89 |
| Example 63 | 1-232 | 4.77 | 64.9 | (0.262, 0.648) | 87 |
| Example 64 | 1-236 | 4.85 | 68.7 | (0.273, 0.671) | 90 |
| Example 65 | 1-238 | 4.29 | 71.0 | (0.243, 0.664) | 94 |
| Example 66 | 1-240 | 5.02 | 72.6 | (0.245, 0.674) | 98 |
| Example 67 | 1-242 | 2.46 | 98.4 | (0.242, 0.664) | 144 |
| Example 68 | 1-243 | 2.37 | 99.5 | (0.275, 0.651) | 140 |
| Example 69 | 1-244 | 2.68 | 103.4 | (0.255, 0.667) | 142 |
| Example 70 | 1-249 | 2.58 | 101.3 | (0.258, 0.643) | 135 |
| Example 71 | 1-253 | 2.59 | 97.3 | (0.255, 0.576) | 130 |
| Example 72 | 1-255 | 2.48 | 96.8 | (0.254, 0.662) | 136 |
| Example 73 | 1-258 | 2.65 | 95.5 | (0.242, 0.764) | 137 |
| Example 74 | 1-259 | 2.67 | 94.7 | (0.248, 0.655) | 140 |
| Example 75 | 1-260 | 2.59 | 93.7 | (0.245, 0.650) | 143 |
| Example 76 | 1-261 | 2.35 | 95.4 | (0.262, 0.643) | 141 |
| Example 77 | 1-262 | 2.88 | 96.8 | (0.275, 0.682) | 136 |
| Example 78 | 1-263 | 2.78 | 98.2 | (0.270, 0.671) | 138 |
| Example 79 | 1-265 | 3.02 | 100.4 | (0.253, 0.674) | 144 |
| Example 80 | 1-267 | 3.29 | 93.8 | (0.245, 0.672) | 130 |
| Example 81 | 1-271 | 3.27 | 91.7 | (0.254, 0.654) | 122 |
| Example 82 | 1-272 | 3.16 | 97.6 | (0.258, 0.662) | 126 |
| Example 83 | 1-274 | 3.25 | 93.7 | (0.242, 0.664) | 128 |
| Example 84 | 1-278 | 3.17 | 94.8 | (0.248, 0.665) | 134 |
| Example 85 | 1-280 | 3.08 | 95.1 | (0.265, 0.655) | 131 |
| Example 86 | 1-281 | 3.32 | 92.3 | (0.262, 0.643) | 122 |
| Example 87 | 1-284 | 3.48 | 87.0 | (0.270, 0.672) | 120 |
| Example 88 | 1-288 | 3.65 | 84.6 | (0.265, 0.644) | 126 |
| Example 89 | 1-289 | 3.67 | 88.0 | (0.273, 0.673) | 125 |
| Example 90 | 1-291 | 3.54 | 87.6 | (0.253, 0.662) | 128 |
| Example 91 | 1-292 | 3.60 | 94.8 | (0.245, 0.675) | 130 |
| Example 92 | 1-294 | 3.58 | 95.2 | (0.240, 0.654) | 135 |
| Example 93 | 1-301 | 2.11 | 105.3 | (0.246, 0.665) | 139 |
| Example 94 | 1-303 | 2.23 | 102.3 | (0.244, 0.653) | 141 |

Compounds A to F used in Comparative Examples 1 to 6 above are as follows.

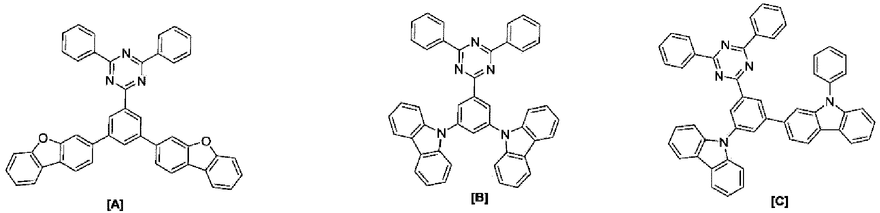

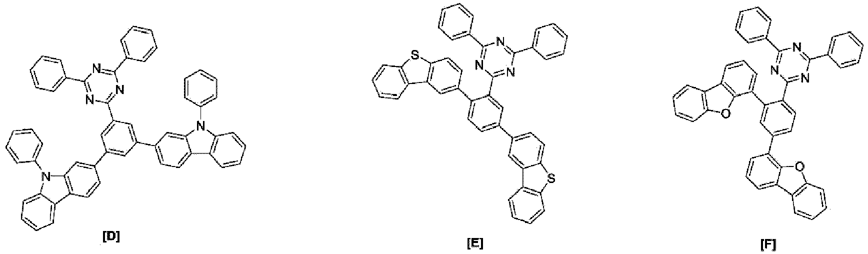

### <Experimental Example 2> - Fabrication of Organic Light Emitting Devices

Glass substrates coated with a thin film of ITO to a thickness of 1,500 Å were washed with distilled water and ultrasonic waves. After washing the substrates with distilled water, ultrasonic cleaning was performed on the washed substrates with solvents such as acetone, methanol, isopropyl alcohol, etc., the ultrasonic cleaned substrates were dried, and UVO-treated for 5 minutes using UV in a UV cleaner. Thereafter, after transferring the substrates to a plasma cleaner (PT), plasma treatment was performed in order to remove the ITO work function and residual film in a vacuum state, and then the substrates were transferred to thermal deposition equipment for organic deposition.

As common layers on the ITO transparent electrodes (anodes), a hole injection layer 2-TNATA (4,4',4"-Tris[2-naphthyl(phenyl)amino]triphenylamine) and a hole transport layer NPB (N,N'-Di (1-naphthyl) -N,N'-diphenyl- (1,1'-biphenyl) -4,4'-diamine) were formed.

A light emitting layer was thermal vacuum deposited thereon as follows. The light emitting layer was deposited to a thickness of 400 Å after premixing one compound described in Chemical Formula 1 and one compound described in Chemical Formula 2 in Table 8 below as a host in one source of supply, and the green phosphorescent dopant was deposited by doping Ir(ppy)₃ to a thickness corresponding to 7% of the deposition thickness of the light emitting layer. The ratio in Table 8 below means the weight ratio. Thereafter, BCP was deposited to a thickness of 60 Å as a hole blocking layer, and Alq₃ was deposited to a thickness of 200 Å thereon as an electron transport layer. Finally, after depositing lithium fluoride (LiF) to a thickness of 10 Å on the electron transport layer to form an electron injection layer, organic electroluminescent devices were manufactured by depositing an aluminum (Al) cathode to a thickness of 1,200 Å on the electron injection layer to form cathodes.

Meanwhile, all organic compounds required for fabricating OLED devices were vacuum sublimated and purified under 10⁻⁶ to 10⁻⁸ torr for each material respectively, and used for OLED fabrication.

Electroluminescence (EL) characteristics were measured for the organic electroluminescent devices fabricated as described above with M7000 of McScience, and T₉₀ values were measured with the measurement results when the reference luminance was 6,000 cd/m² through the device lifespan measuring system (M6000) manufactured by McScience.

The results of measuring driving voltage values, luminous efficiency values, color coordinate (CIE) values, and lifespan values of the organic light emitting devices manufactured according to the present disclosure were as shown in Table 8 below.

**[Table 8]**

| | Light emitting layer compound | Ratio | Driving voltage (V) | Efficiency (cd/A) | Color coordinates (x, y) | Lifespan (T₉₀) |
|---|---|---|---|---|---|---|
| Example 93 | 1-1 : 2-6 | 1 : 1 | 3.48 | 94.9 | (0.254, 0.652) | 241 |
| Example 94 | | 1: 2 | 3.45 | 94.6 | (0.246, 0.670) | 245 |
| Example 95 | | 1 : 2.5 | 3.32 | 93.5 | (0.267, 0.679) | 249 |
| Example 96 | 1-9 : 2-44 | 1 : 1 | 3.31 | 94.0 | (0.245, 0.704) | 237 |
| Example 97 | | 1: 2 | 3.23 | 93.7 | (0.241, 0.639) | 246 |
| Example 98 | | 1 : 2.5 | 3.18 | 92.3 | (0.243, 0.612) | 249 |
| Example 99 | 1-15 : 2-44 | 1 : 1 | 3.41 | 89.1 | (0.252, 0.704) | 237 |
| Example 100 | | 1 : 2 | 3.34 | 88.5 | (0.230, 0.712) | 243 |
| Example 101 | | 1 : 2.5 | 3.20 | 87.9 | (0.241, 0.613) | 244 |
| Example 102 | 1-22 : 2-61 | 1 : 1 | 3.51 | 89.3 | (0.242, 0.724) | 230 |
| Example 103 | | 1 : 2 | 3.49 | 87.3 | (0.246, 0.657) | 235 |
| Example 104 | | 1 : 2.5 | 3.35 | 85.2 | (0.251, 0.721) | 239 |
| Example 105 | 1-44 : 2-34 | 1 : 1 | 3.39 | 91.5 | (0.241, 0.695) | 238 |
| Example 106 | | 1 : 2 | 3.25 | 86.7 | (0.231, 0.712) | 243 |
| Example 107 | | 1 : 2.5 | 3.10 | 85.3 | (0.233, 0.714) | 245 |
| Example 108 | 1-53 : 2-6 | 1 : 1 | 3.39 | 95.2 | (0.235, 0.682) | 240 |
| Example 109 | | 1 : 2 | 3.25 | 94.5 | (0.232, 0.625) | 248 |
| Example 110 | | 1 : 2.5 | 3.03 | 93.2 | (0.241, 0.713) | 250 |
| Example 111 | 1-73 : 2-16 | 1 : 1 | 3.40 | 89.0 | (0.247, 0.715) | 238 |
| Example 112 | | 1 : 2 | 3.22 | 87.9 | (0.231, 0.710) | 240 |
| Example 113 | | 1 : 2.5 | 3.12 | 86.7 | (0.252, 0.614) | 243 |
| Example 114 | 1-83 : 2-16 | 1 : 1 | 4.72 | 71.9 | (0.260, 0.715) | 197 |
| Example 115 | | 1 : 2 | 4.66 | 71.3 | (0.241, 0.615) | 200 |
| Example 116 | | 1 : 2.5 | 4.53 | 70.2 | (0.243, 0.713) | 213 |
| Example 117 | 1-89 : 2-34 | 1 : 1 | 4.83 | 76.3 | (0.250, 0.627) | 200 |
| Example 118 | | 1 : 2 | 4.75 | 75.5 | (0.233, 0.710) | 206 |
| Example 119 | | 1 : 2.5 | 4.72 | 72.9 | (0.239, 0.745) | 210 |
| Example 120 | 1-128 : 2-44 | 1 : 1 | 6.69 | 77.6 | (0.236, 0.664) | 198 |
| Example 121 | | 1 : 2 | 6.68 | 75.8 | (0.231, 0.645) | 205 |
| Example 122 | | 1 : 2.5 | 4.61 | 74.3 | (0.248, 0.721) | 213 |
| Example 123 | 1-135 : 2-52 | 1 : 1 | 4.59 | 78.6 | (0.235, 0.711) | 210 |
| Example 124 | | 1 : 2 | 4.56 | 77.5 | (0.252, 0.619) | 213 |
| Example 125 | | 1 : 2.5 | 4.50 | 75.9 | (0.263, 0.715) | 215 |
| Example 126 | 1-163 : 2-34 | 1 : 1 | 3.99 | 77.6 | (0.258, 0.662) | 208 |
| Example 127 | | 1 : 2 | 3.91 | 76.5 | (0.244, 0.659) | 210 |
| Example 128 | | 1 : 2.5 | 3.88 | 76.1 | (0.248, 0.675) | 219 |
| Example 129 | 1-178 : 2-61 | 1 : 1 | 3.79 | 84.9 | (0.265, 0.755) | 213 |
| Example 130 | | 1 : 2 | 3.73 | 84.4 | (0.262, 0.649) | 219 |
| Example 131 | | 1 : 2.5 | 3.66 | 83.6 | (0.271, 0.662) | 221 |
| Example 132 | 1-200 : 2-61 | 1 : 1 | 4.01 | 75.5 | (0.230, 0.644) | 210 |
| Example 133 | | 1 : 2 | 3.81 | 75.3 | (0.248, 0.720) | 213 |
| Example 134 | | 1 : 2.5 | 3.85 | 75.0 | (0.235, 0.701) | 215 |
| Example 135 | 1-209 : 2-52 | 1 : 1 | 3.69 | 78.5 | (0.250, 0.619) | 218 |
| Example 136 | | 1 : 2 | 3.60 | 77.9 | (0.263, 0.725) | 220 |
| Example 137 | | 1 : 2.5 | 3.54 | 77.3 | (0.258, 0.662) | 225 |
| Example 138 | 1-242 : 2-61 | 1 : 1 | 2.29 | 120.5 | (0.235, 0.710) | 338 |
| Example 139 | | 1 : 2 | 2.25 | 119.6 | (0.239, 0.735) | 341 |
| Example 140 | | 1 : 2.5 | 2.15 | 119.2 | (0.236, 0.654) | 344 |
| Example 141 | 1-244 : 2-34 | 1 : 1 | 2.23 | 116.7 | (0.232, 0.640) | 305 |
| Example 142 | | 1 : 2 | 2.20 | 113.6 | (0.245, 0.721) | 310 |
| Example 143 | | 1 : 2.5 | 2.19 | 113.0 | (0.252, 0.615) | 313 |
| Example 144 | 1-249 : 2-52 | 1 : 1 | 1.89 | 120.2 | (0.233, 0.705) | 340 |
| Example 145 | | 1 : 2 | 1.85 | 118.9 | (0.258, 0.662) | 342 |
| Example 146 | | 1 : 2.5 | 1.80 | 118.7 | (0.247, 0.650) | 345 |
| Example 147 | 1-259 : 2-34 | 1 : 1 | 2.30 | 111.0 | (0.248, 0.675) | 283 |
| Example 148 | | 1 : 2 | 2.28 | 110.9 | (0.265, 0.745) | 297 |
| Example 149 | | 1 : 2.5 | 2.25 | 110.3 | (0.259, 0.735) | 300 |
| Example 150 | 1-267 : 2-44 | 1 : 1 | 3.15 | 105.0 | (0.246, 0.654) | 270 |
| Example 151 | | 1 : 2 | 3.13 | 104.8 | (0.239, 0.740) | 276 |
| Example 152 | | 1 : 2.5 | 3.01 | 104.3 | (0.265, 0.721) | 279 |
| Example 153 | 1-271 : 2-52 | 1 : 1 | 2.66 | 101.6 | (0.252, 0.665) | 277 |
| Example 154 | | 1 : 2 | 2.63 | 100.3 | (0.234, 0.731) | 280 |
| Example 155 | | 1 : 2.5 | 2.59 | 99.5 | (0.250, 0.659) | 283 |
| Example 156 | 1-278 : 2-16 | 1 : 1 | 2.60 | 105.2 | (0.261, 0.615) | 287 |
| Example 157 | | 1 : 2 | 2.55 | 104.9 | (0.257, 0.632) | 290 |
| Example 158 | | 1 : 2.5 | 2.53 | 104.3 | (0.245, 0.669) | 295 |
| Example 159 | 1-281 : 2-52 | 1 : 1 | 2.83 | 100.8 | (0.254, 0.637) | 280 |
| Example 160 | | 1 : 2 | 2.79 | 99.7 | (0.235, 0.690) | 285 |
| Example 161 | | 1 : 2.5 | 2.76 | 98.6 | (0.246, 0.740) | 286 |
| Example 162 | 1-294 : 2-44 | 1 : 1 | 2.66 | 103.0 | (0.236, 0.654) | 275 |
| Example 163 | | 1 : 2 | 2.65 | 101.2 | (0.239, 0.645) | 276 |
| Example 164 | | 1 : 2.5 | 2.61 | 100.3 | (0.244, 0.722) | 280 |
| Example 165 | 1-301 : 2-6 | 1 : 1 | 2.09 | 119.2 | (0.244, 0.665) | 270 |
| Example 165 | | 1 : 2 | 2.05 | 113.2 | (0.245, 0.657) | 273 |
| Example 166 | 1-303 : 2-6 | 1 : 1 | 2.15 | 120.2 | (0.246, 0.653) | 283 |
| Example 167 | | 1 : 2 | 2.06 | 118.7 | (0.247, 0.655) | 285 |

As can be seen from the results in Table 7 above, the organic electroluminescent device using the light emitting layer material of the organic electroluminescent device of the present disclosure not only has a low driving voltage and improved luminous efficiency, but also has remarkably improved lifespan compared to those of Comparative Examples 1 to 6.

Looking at the results of Tables 7 and 8, when the compound of Chemical Formula 1 and the compound of Chemical Formula 2 are comprised at the same time, better efficiency and lifespan effects are exhibited. An exciplex phenomenon may be expected to occur from these results when both compounds are comprised at the same time. The exciplex phenomenon is a phenomenon in which energy having a size of a HOMO level of a donor (p-host) and a LUMO level of an acceptor (n-host) is emitted through electron exchange between two molecules. Since holes are injected into the p-host and electrons are injected into the n-host when a donor (p-host) with good hole transport ability and an acceptor (n-host) with good electron transport ability are used as the host of the light emitting layer, the driving voltage may be lowered, thereby helping to improve the lifespan. In the present disclosure, it could be confirmed that the device exhibits excellent device properties, when the compound of Chemical Formula 2 above as a donor, and the compound of Chemical Formula 1 above as an acceptor are used as a host for the light emitting layer.
in Chemical Formula 1 above, two ortho sites on both sides of triazine are relatively rich in electrons due to the resonance structure. Meanwhile, two meta sites based on triazine are sites with relatively few electrons due to the resonance structure. Therefore, in the case of a symmetric structure in which carbazole containing nitrogen with high electronegativity is directly bonded to two places where electrons are relatively scarce, electron balance is broken and electron transfer within a molecule is not easy. Since the symmetric structure in which carbazole is directly bonded to the ortho site is bonded to the electron-rich site, electron transfer is better than that of Comparative Example Material B so that the driving voltage is low, and the efficiency and lifespan are good.

However, when a cyclic compound fused to at least one of the two meta sites comes as a substituent, there is an effect that the HOMO orbital spreads widely, and the number of electrons possessed by the substituent itself increases so that the electron balance is well matched. Since the electron balance in the molecule is better matched than that of Comparative Example Material B, the molecular structure is more stable, and since the HOMO orbital is widely spread, the electron transfer between molecules also occurs well so that the driving voltage tends to be lowed.

Dibenzofuran and dibenzothiophene are relatively rich in electrons at sites 2 and 4 and relatively lack of electrons at sites 1 and 3 due to their resonance structure. Accordingly, when a substituent is bonded to a site having the same number as in Comparative Example Materials A, E, and F, the electron balance is not well matched. It could be confirmed that the driving voltage, efficiency, and lifespan were improved when the electron-deficient site and the electron-abundant site were combined with a substituent in a balanced manner.

## Claims

1. A heterocyclic compound represented by the following Chemical Formula 1: 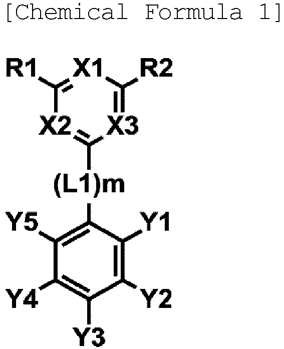
in Chemical Formula 1,
R1 and R2 are the same as or different from each other, and are each independently hydrogen; deuterium; a substituted or unsubstituted C₁-C₆₀ alkyl group; a substituted or unsubstituted C₆-C₆₀ aryl group; or a substituted or unsubstituted C₂-C₆₀ heteroaryl group,
X1 to X3 are the same as or different from each other and are each independently CRx; or N, and at least two thereof are N,
L1 is a direct bond; a substituted or unsubstituted C₆-C₆₀ arylene group; or a substituted or unsubstituted C₂-C₆₀ heteroarylene group,
Rx is hydrogen; deuterium; a substituted or unsubstituted C₆-C₆₀ aryl group; or a substituted or unsubstituted C₂-C₆₀ heteroaryl group,
two of Y1 to Y5 are represented by Chemical Formula A or Chemical Formula B below, and the remainder is hydrogen; or deuterium, 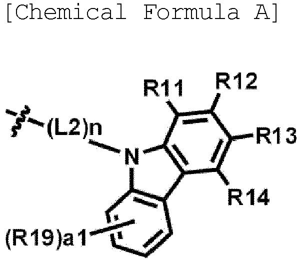 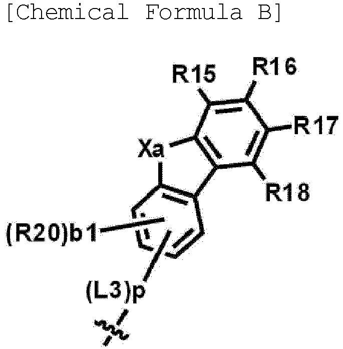
in Chemical Formula A and Chemical Formula B,
L2 and L3 are the same as or different from each other, and are each independently a direct bond; a substituted or unsubstituted C₆-C₆₀ arylene group; or a substituted or unsubstituted C₂-C₆₀ heteroarylene group,
Xa is O; S; or NRy,
R11 to R20 are the same as or different from each other, and are each independently hydrogen; or deuterium, or two or more groups adjacent to each other are bonded to each other to form a substituted or unsubstituted C₆-C₆₀ aromatic hydrocarbon ring or a substituted or unsubstituted C₂-C₆₀ hetero ring,
Ry is a substituted or unsubstituted C₆-C₆₀ aryl group; or a substituted or unsubstituted C₂-C₆₀ heteroaryl group,
m, n, a1, and p are integers of 0 to 4,
b1 is an integer of 0 to 3,
when m, n, a1, b1 and p are each 2 or more, the substituents in parentheses are the same as or different from each other,
when two of Y1 to Y5 are represented by Chemical Formula B above, and Xa is O; or S, the two Chemical Formulae B are different from each other,
when two of Y1 to Y5 are represented by Chemical Formula A above, and Chemical Formula A above is substituted with Y2, the remaining Chemical Formula A is substituted with one of Y1, Y3 and Y5,
when two of Y1 to Y5 are Chemical Formula B in which Xa is NRy, and Chemical Formula B in which Xa is NRy is substituted with Y2 and Y4, at least two among R15 to R18 of Chemical Formula B in which Xa is NRy, which is substituted with Y2, are bonded to each other to form a substituted or unsubstituted C₆-C₆₀ aromatic hydrocarbon ring or a substituted or unsubstituted C₂-C₆₀ heteroring, and
when two of Y1 to Y5 are Chemical Formula B in which Xa is NRy, and Chemical Formula A, Chemical Formula A above is substituted with Y2, and Chemical Formula B in which Xa is NRy is substituted with Y4, at least two among R11 to R14 of Chemical Formula A above substituted with Y2 are bonded to each other to form a substituted or unsubstituted C₆-C₆₀ aromatic hydrocarbon ring or a substituted or unsubstituted C₂-C₆₀ heteroring.

2. The heterocyclic compound of claim 1, wherein Chemical Formula 1 above is represented by any one of the following Chemical Formulae 3 to 6: 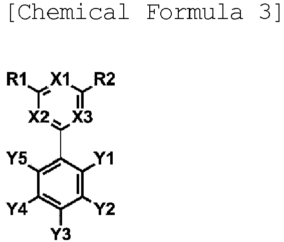 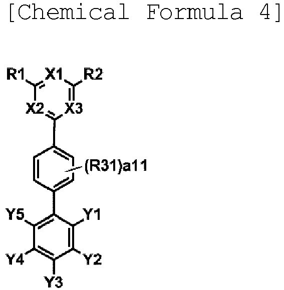 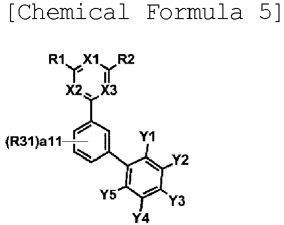 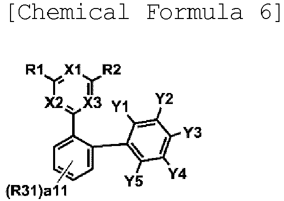
in Chemical Formulae 3 to 6,
the definition of each substituent is the same as that in Chemical Formula 1 above,
R31 is hydrogen; or deuterium, and
a11 is an integer of 0 to 4, and when a11 is 2 or more, the substituents in parentheses are the same as or different from each other.

3. The heterocyclic compound of claim 1, wherein Chemical Formula A above is represented by the following Chemical Formula A-1-1 or A-1-2: 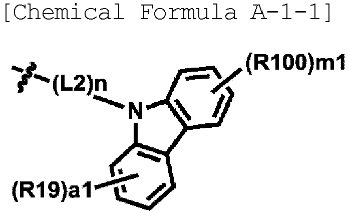 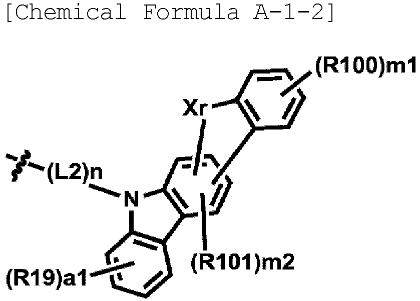
in Chemical Formulae A-1-1 and A-1-2,
the definitions of L2, R19, a1 and n are the same as those in Chemical Formula A above,
Xr is O; S; CRR'; or NR,
R100 and R101 are the same as or different from each other, and are each independently hydrogen; or deuterium,
m1 is an integer of 0 to 4, m2 is an integer of 0 to 2,
when m1 is an integer of 2 or more, or m2 is 2, the substituents in parentheses are the same as or different from each other, and
R and R' are the same as or different from each other, and are each independently a substituted or unsubstituted C₁-C₆₀ alkyl group; or a substituted or unsubstituted C₆-C₆₀ aryl group.

4. The heterocyclic compound of claim 1, wherein Chemical Formula B above is represented by the following Chemical Formula B-1-1 or B-1-2: 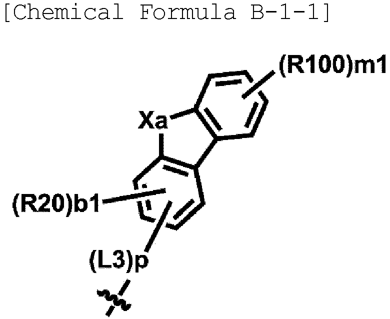 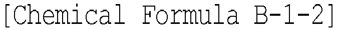 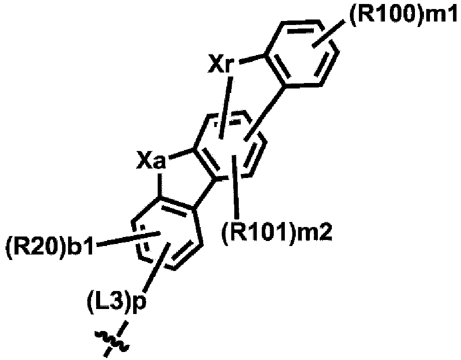
in Chemical Formulae B-1-1 and B-1-2,
the definitions of L3, Xa, R20, b1 and p are the same as those in Chemical Formula B above,
Xr is O; S; CRR'; or NR,
R and R' are the same as or different from each other, and are each independently a substituted or unsubstituted C₁-C₆₀ alkyl group; or a substituted or unsubstituted C₆-C₆₀ aryl group,
R100 and R101 are the same as or different from each other, and are each independently hydrogen; or deuterium,
m1 is an integer of 0 to 4, m2 is an integer of 0 to 2, and
when m1 is an integer of 2 or more, or m2 is 2, the substituents in parentheses are the same as or different from each other.

5. The heterocyclic compound of claim 1, wherein Chemical Formula 1 above is represented by any one of the following compounds:

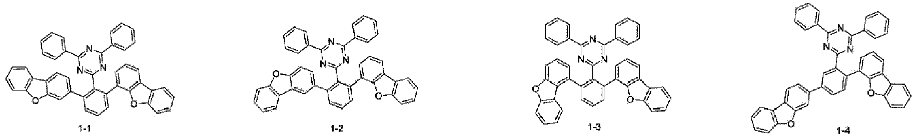

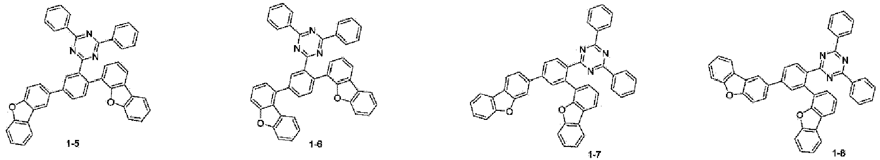

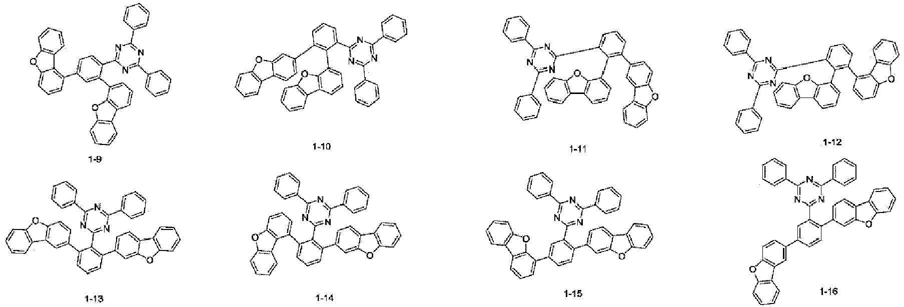

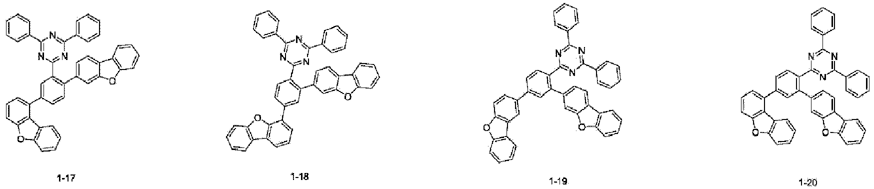

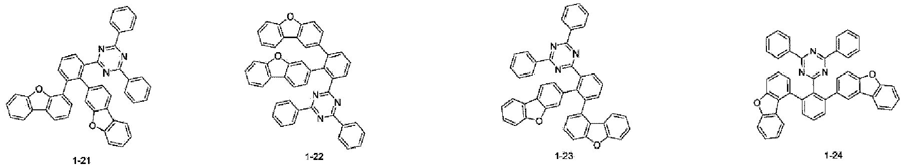

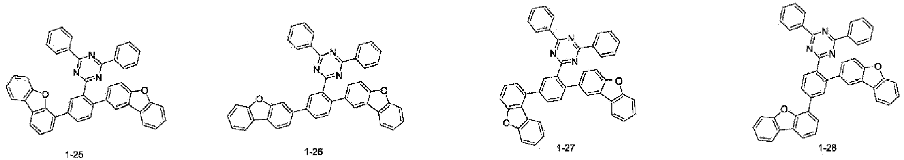

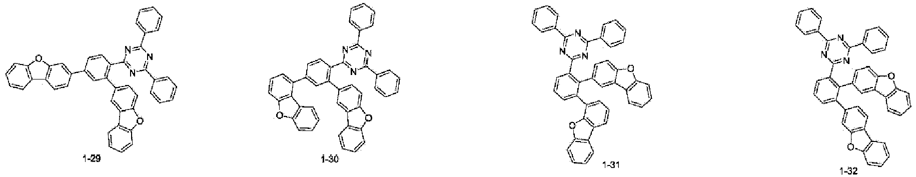

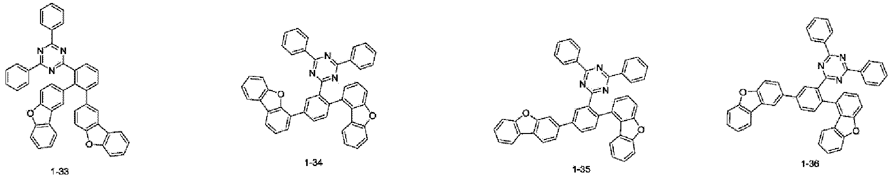

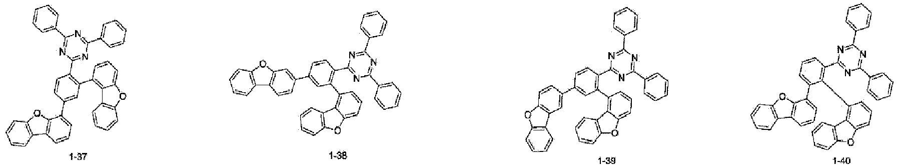

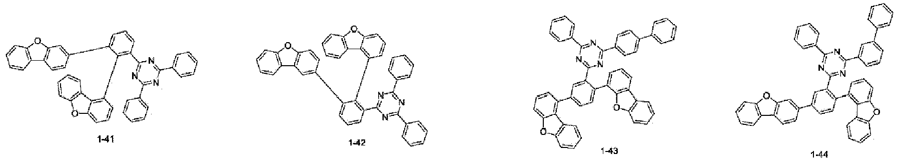

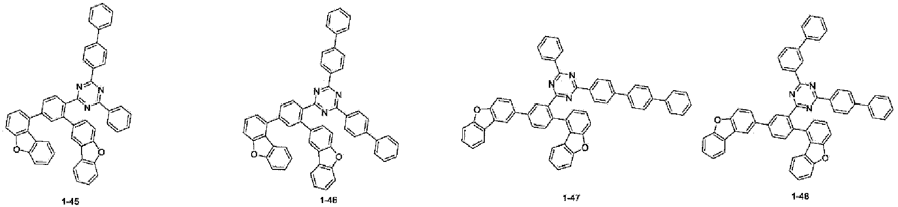

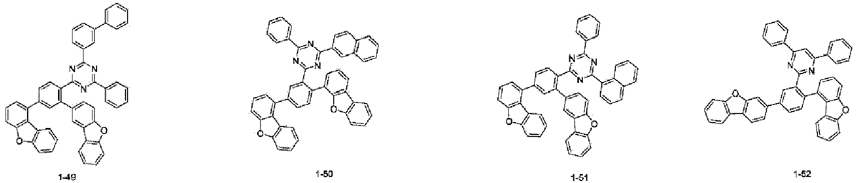

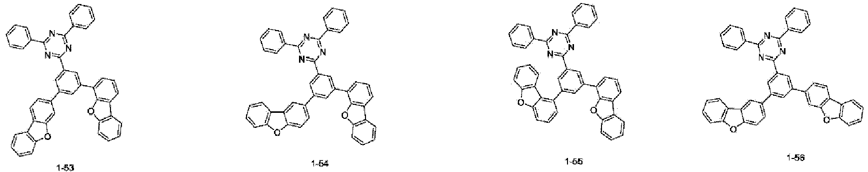

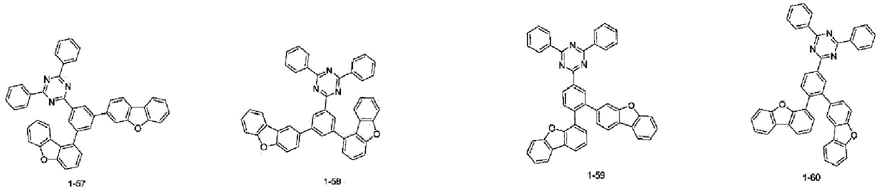

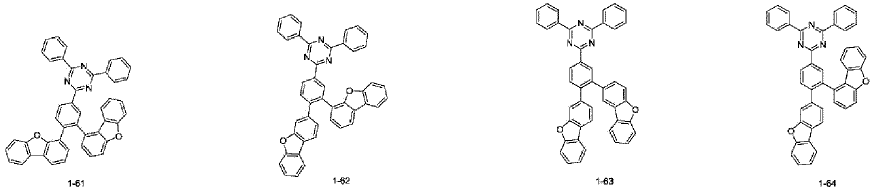

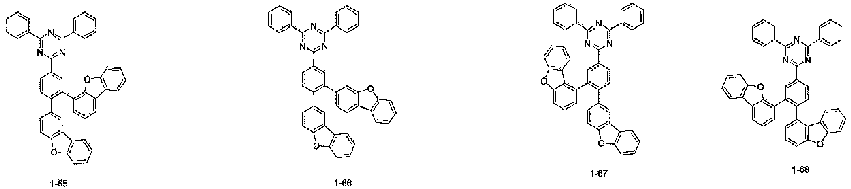

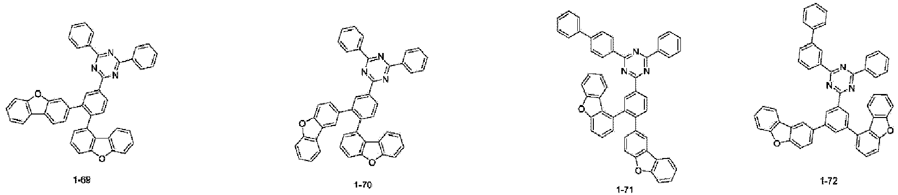

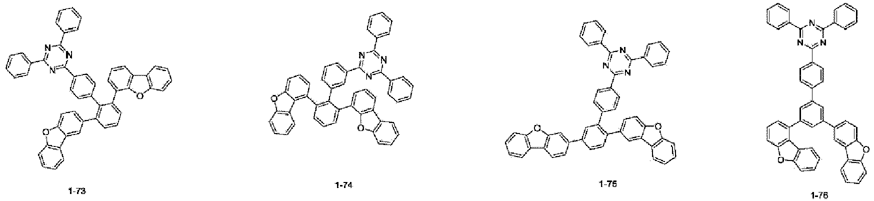

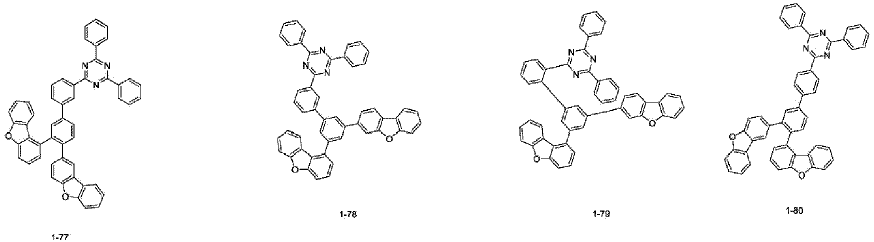

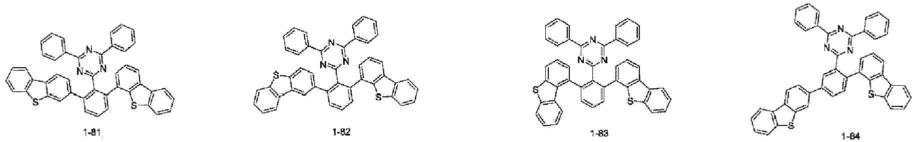

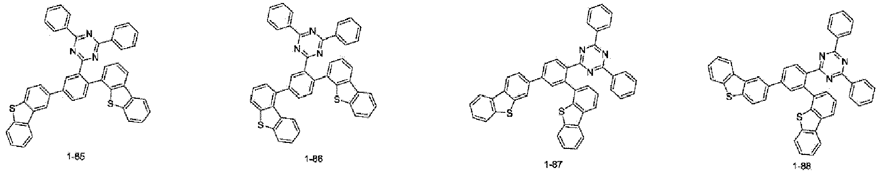

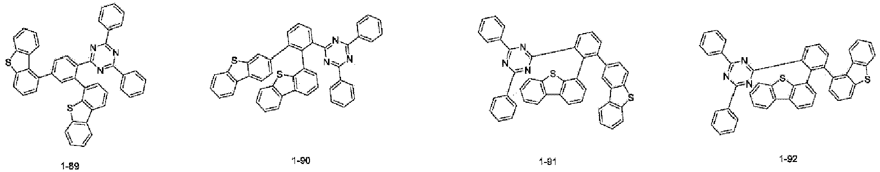

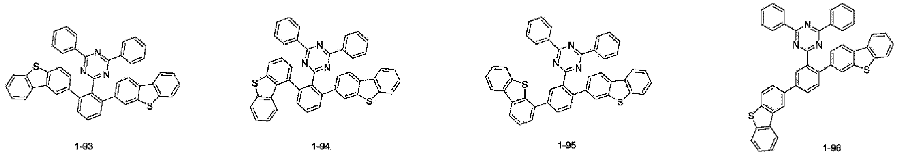

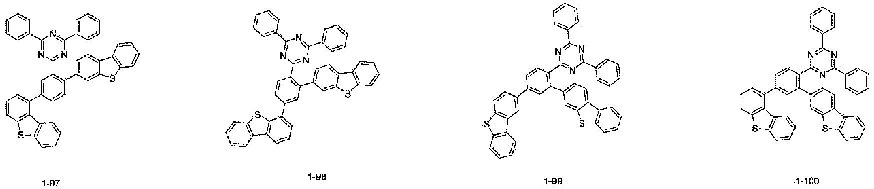

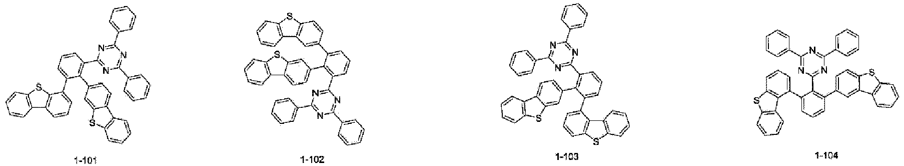

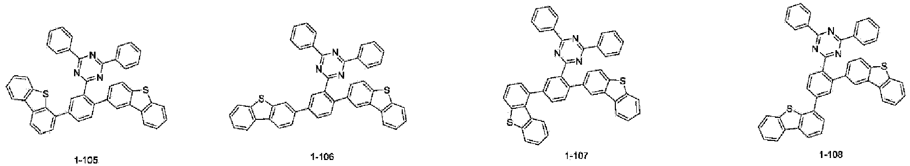

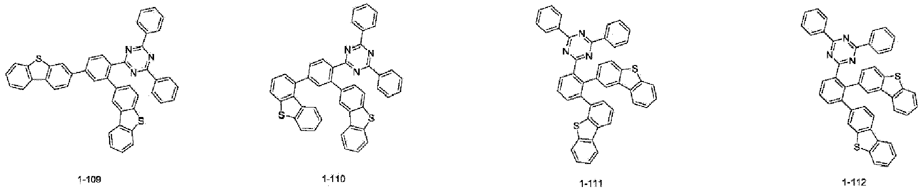

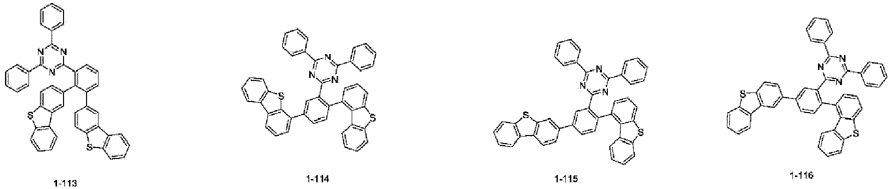

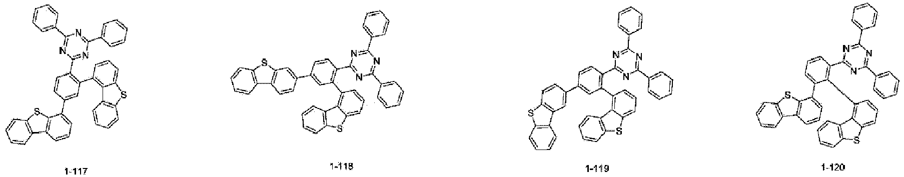

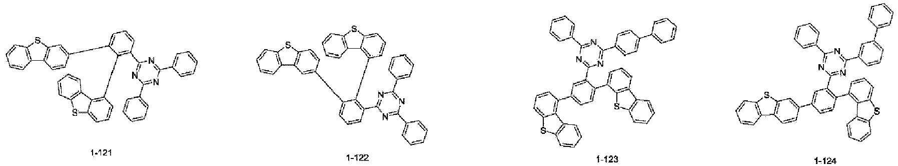

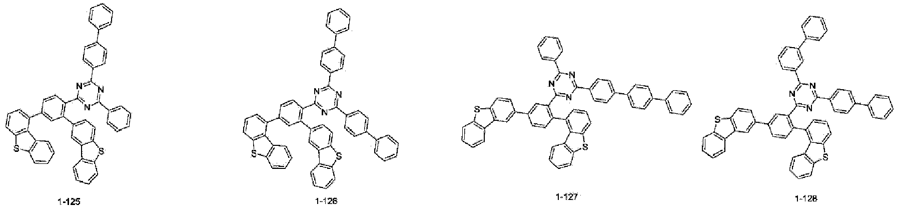

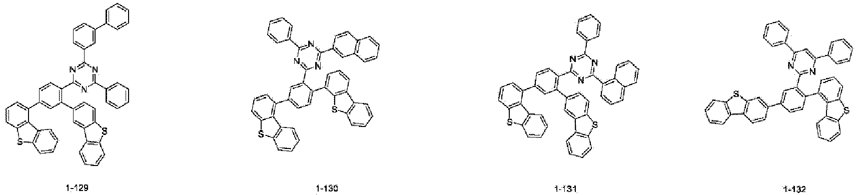

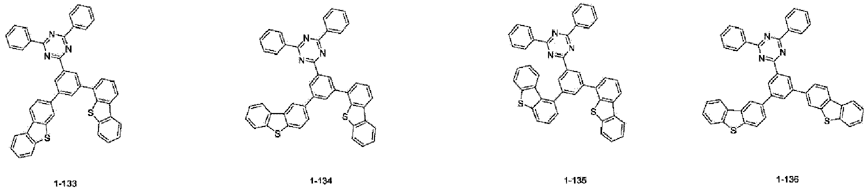

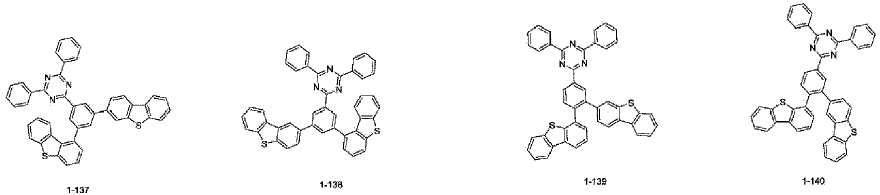

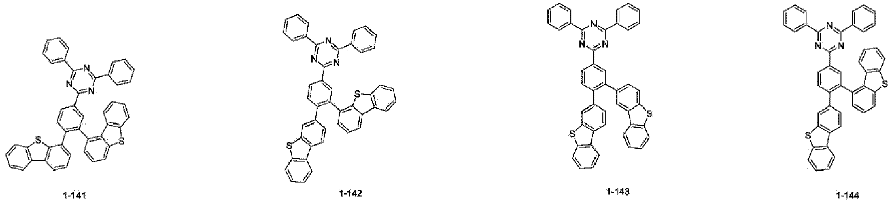

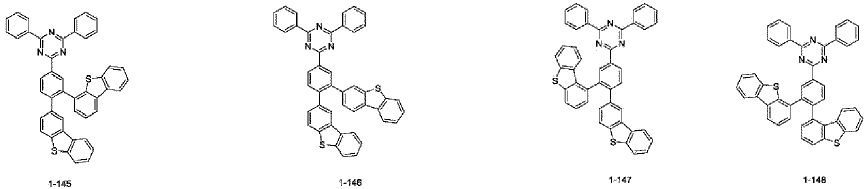

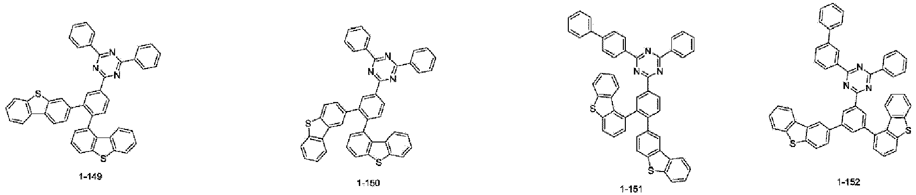

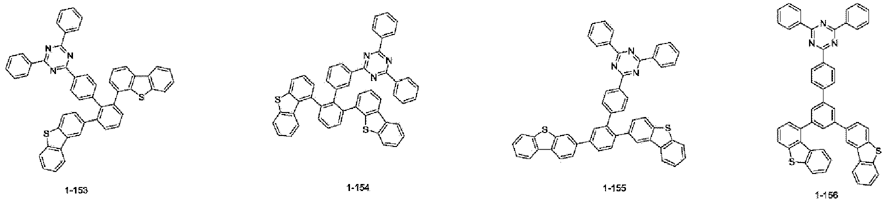

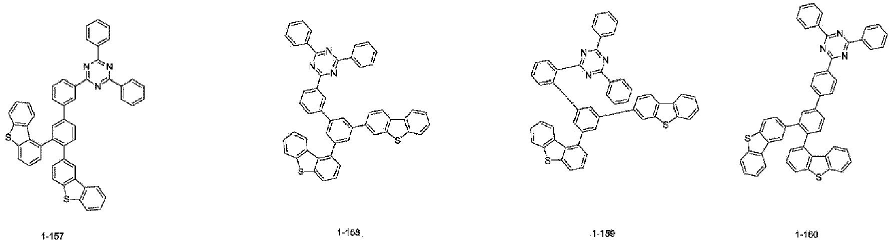

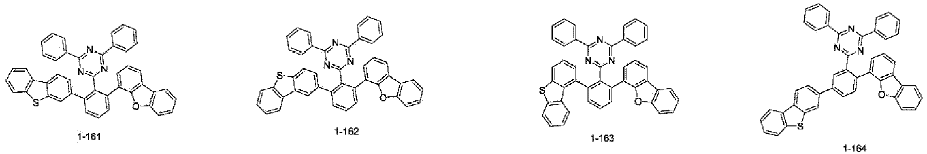

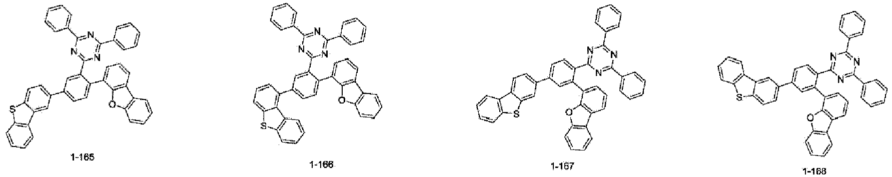

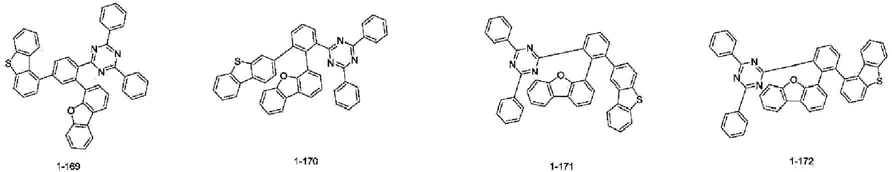

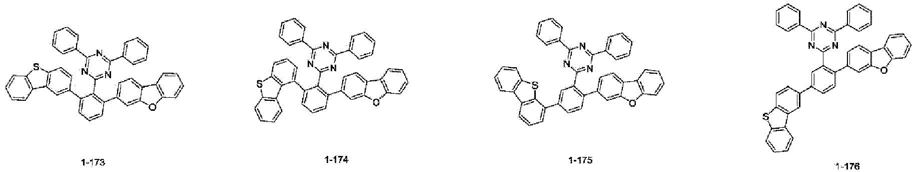

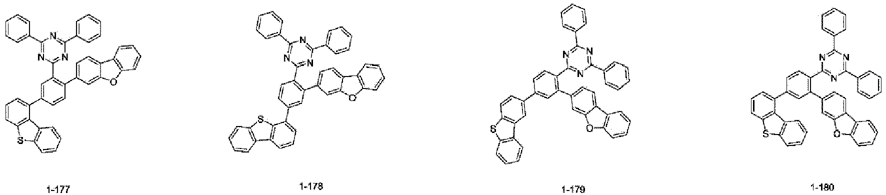

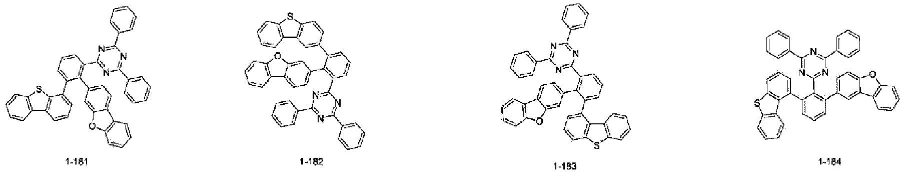

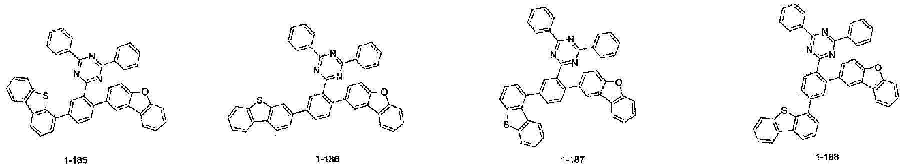

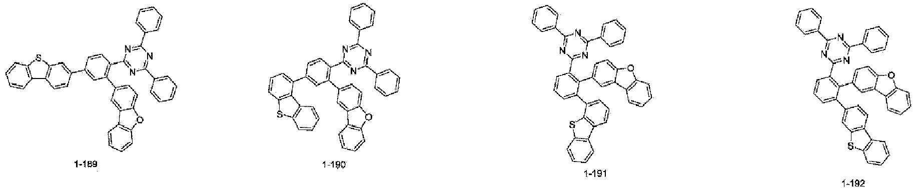

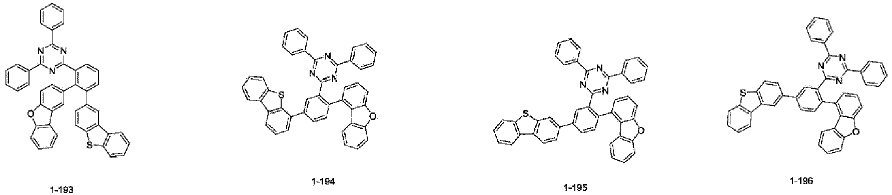

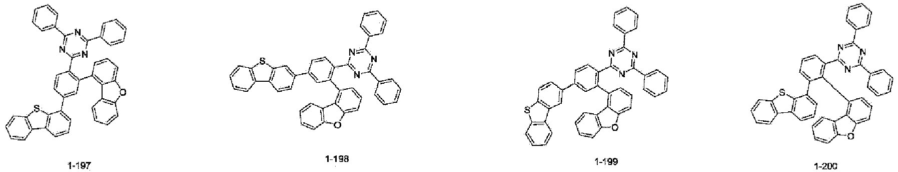

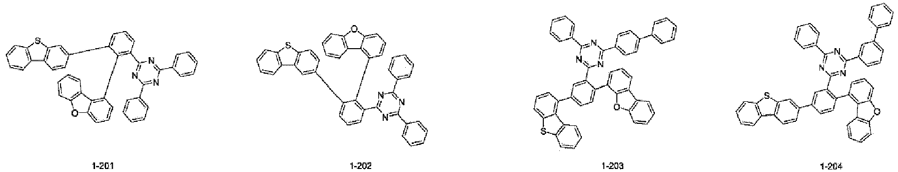

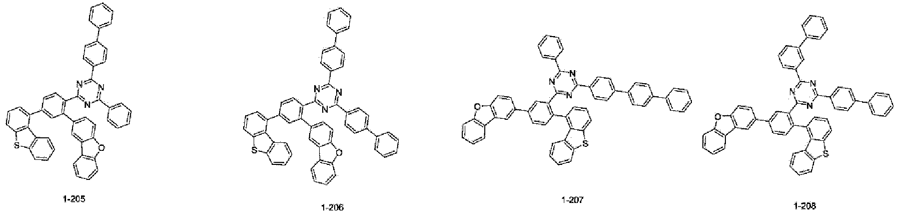

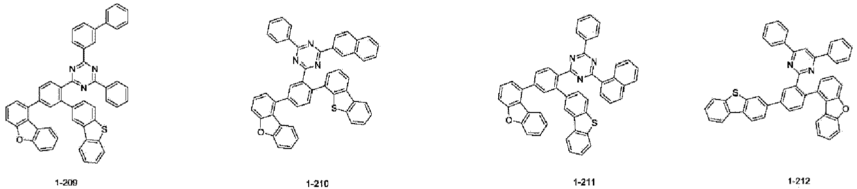

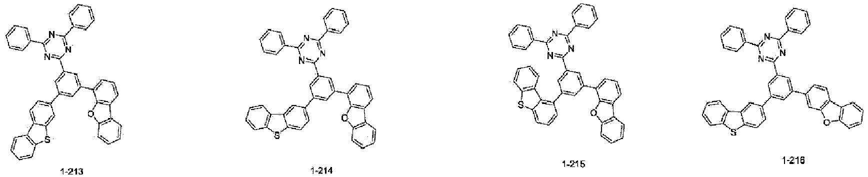

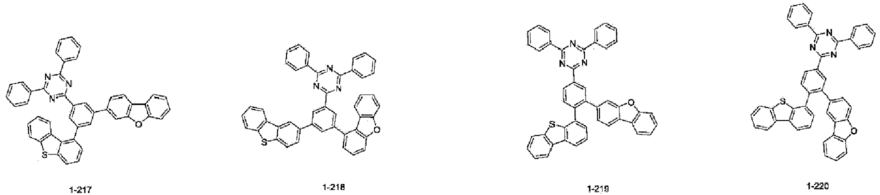

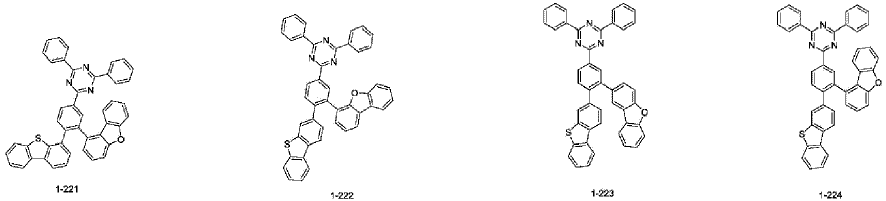

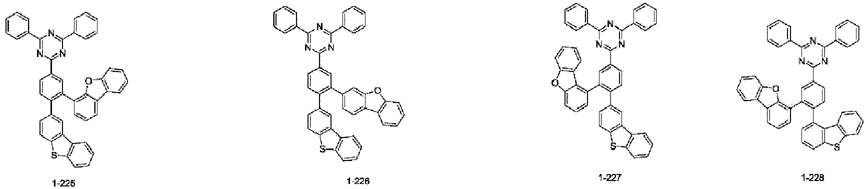

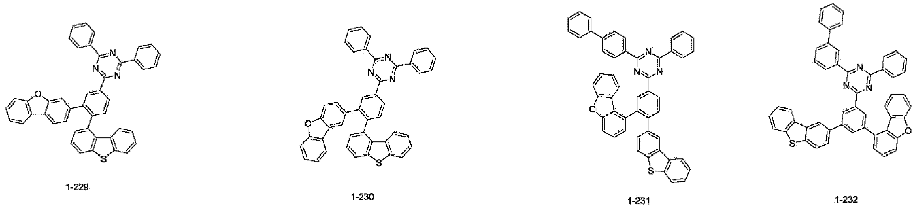

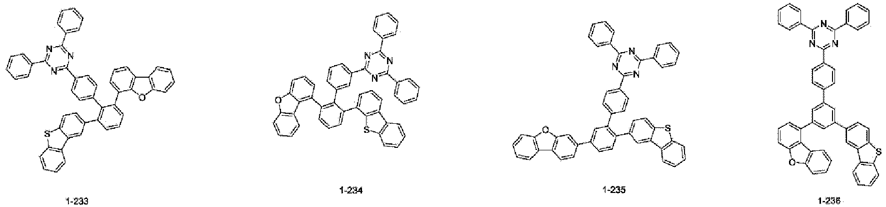

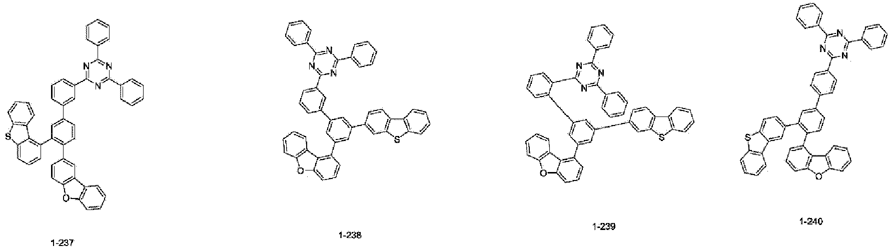

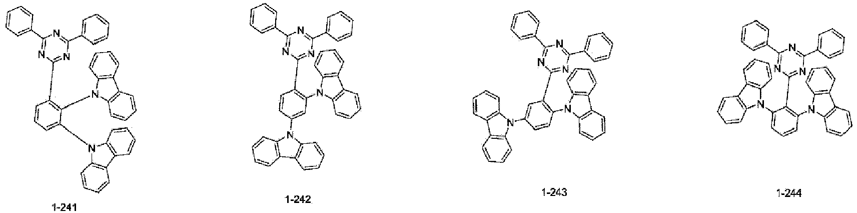

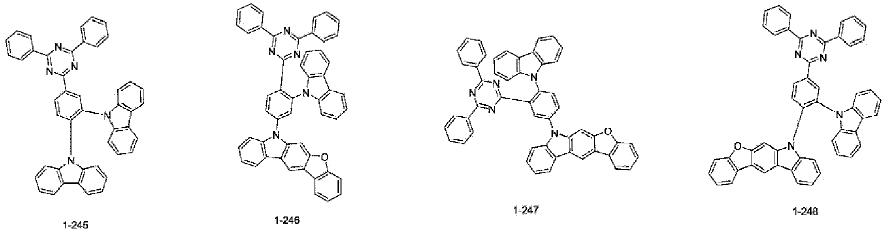

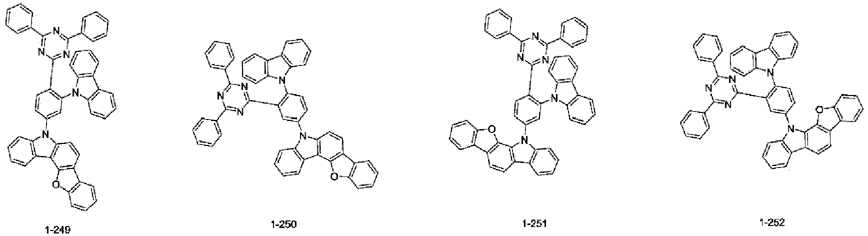

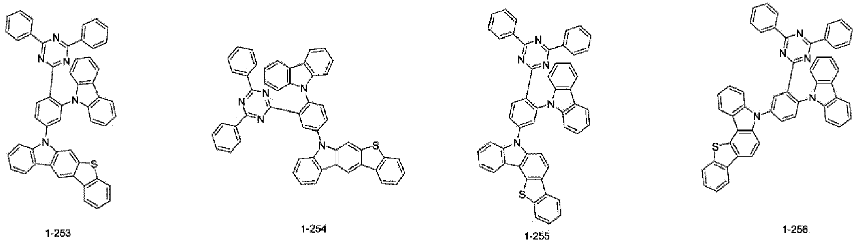

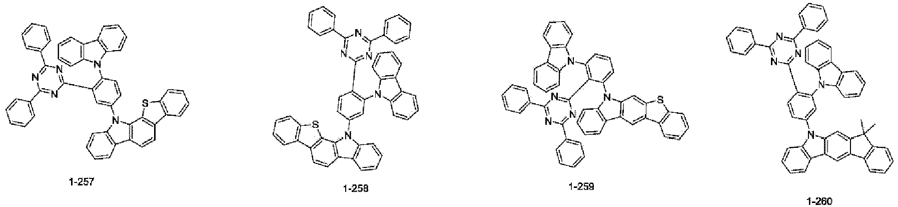

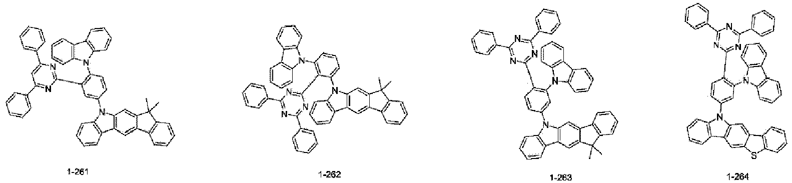

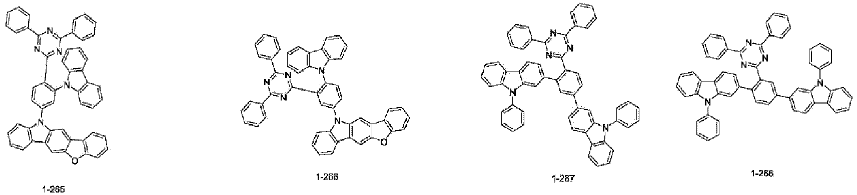

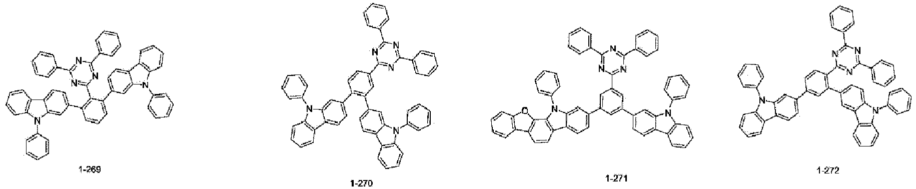

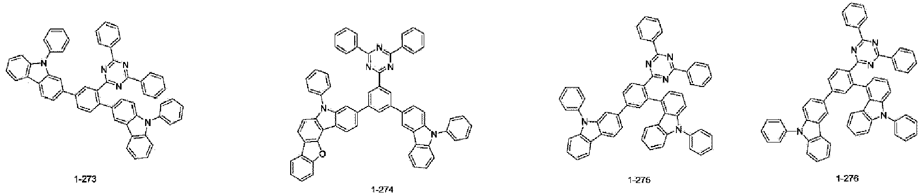

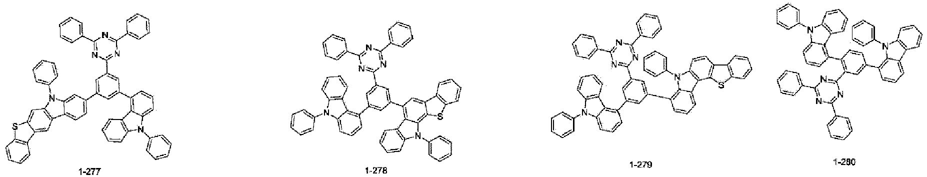

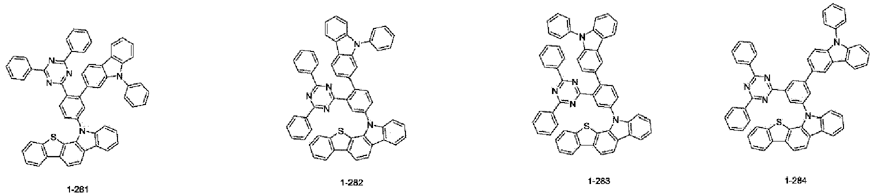

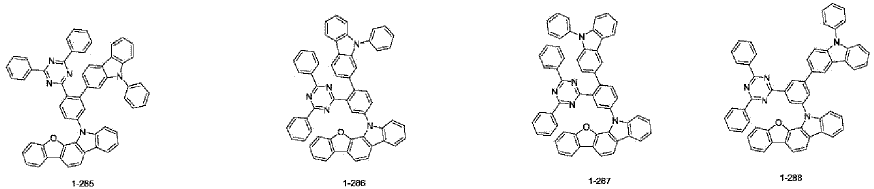

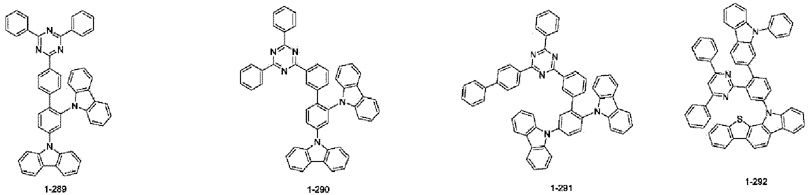

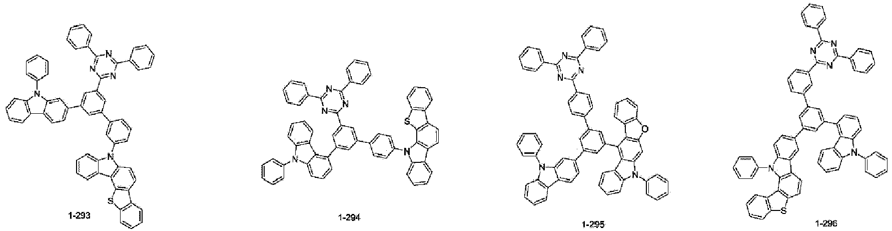

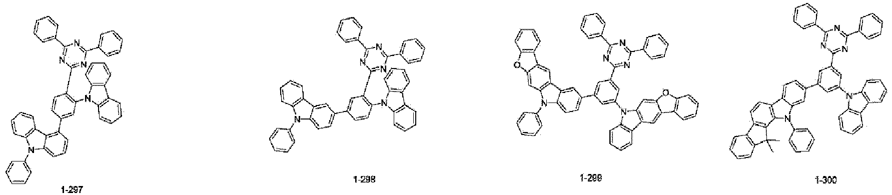

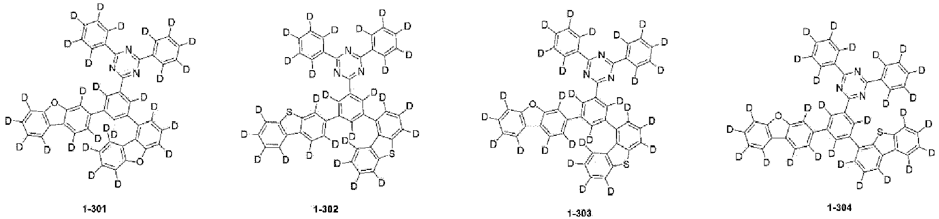

6. An organic light emitting device comprising:
a first electrode;
a second electrode provided opposite to the first electrode; and
one or more organic material layers provided between the first electrode and the second electrode,
wherein one or more layers of the organic material layers comprise the heterocyclic compound according to any one of claims 1 to 5.

7. The organic light emitting device of claim 6, wherein the organic material layer comprising the heterocyclic compound further comprises a heterocyclic compound represented by the following Chemical Formula 2: 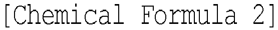 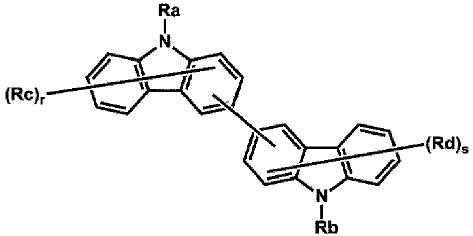
in Chemical Formula 2,
Rc and Rd are the same as or different from each other, and are each independently selected from the group consisting of: hydrogen; deuterium; a halogen group; -CN; a substituted or unsubstituted C₁-C₆₀ alkyl group; a substituted or unsubstituted C₂-C₆₀ alkenyl group; a substituted or unsubstituted C₂-C₆₀ alkynyl group; a substituted or unsubstituted C₁-C₆₀ alkoxy group; a substituted or unsubstituted C₃-C₆₀ cycloalkyl group; a substituted or unsubstituted C₂-C₆₀ heterocycloalkyl group; a substituted or unsubstituted C₆-C₆₀ aryl group; a substituted or unsubstituted C₂-C₆₀ heteroaryl group; -SiR101R102R103; - P(=O)R101R102; and -NR101R102, or two or more groups adjacent to each other are bonded to each other to form a substituted or unsubstituted C₆-C₆₀ aromatic hydrocarbon ring or a substituted or unsubstituted C₂-C₆₀ heteroring,
Ra and Rb are the same as or different from each other, and are each independently a substituted or unsubstituted C₆-C₆₀ aryl group; a substituted or unsubstituted C₂-C₆₀ heteroaryl group; -SiR101R102R103; or -P(=O)R101R102,
R101, R102, and R103 are the same as or different from each other, and are each independently hydrogen; deuterium; -CN; a substituted or unsubstituted C₁-C₆₀ alkyl group; a substituted or unsubstituted C₆-C₆₀ aryl group; or a substituted or unsubstituted C₂-C₆₀ heteroaryl group, and
r and s are integers of 0 to 7, and when r and s are each 2 or more, the substituents in parentheses are the same as or different from each other.

8. The organic light emitting device of claim 7, wherein the heterocyclic compound represented by Chemical Formula 2 above is any one selected from the following compounds:

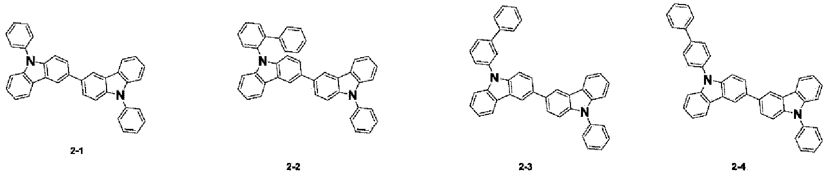

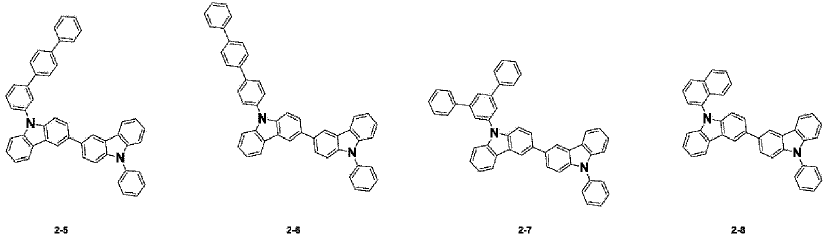

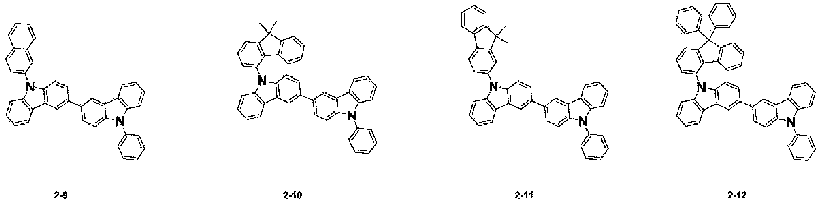

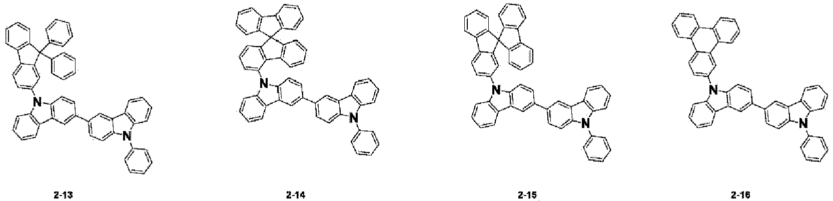

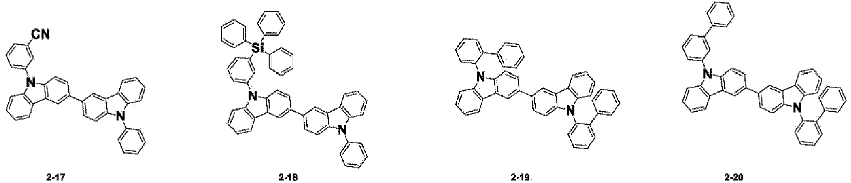

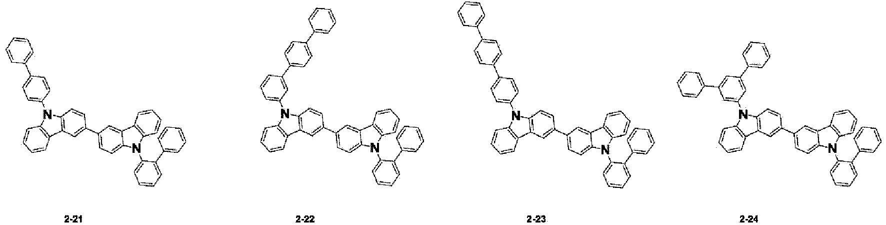

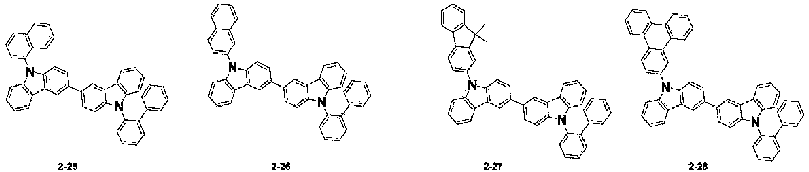

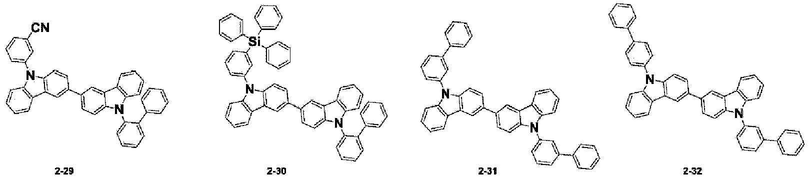

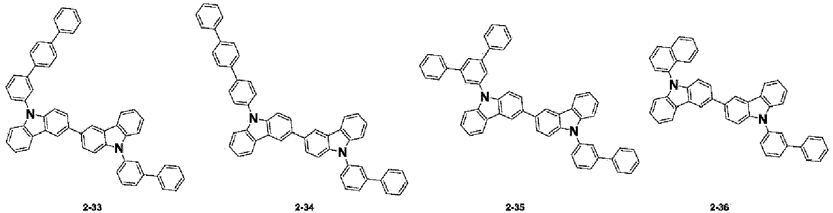

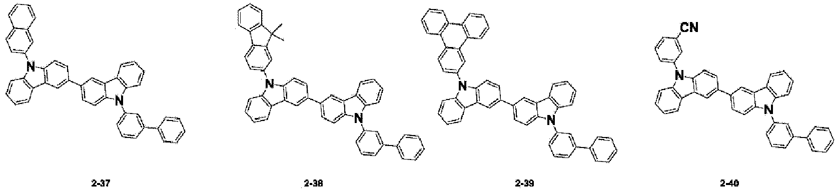

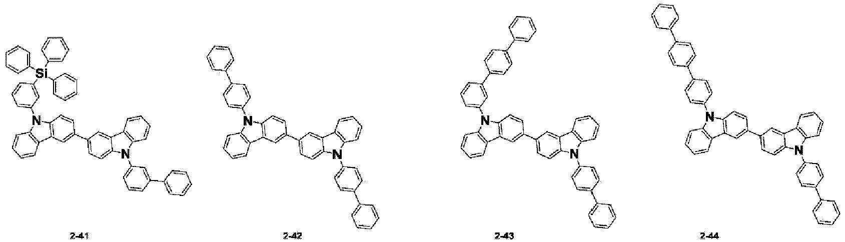

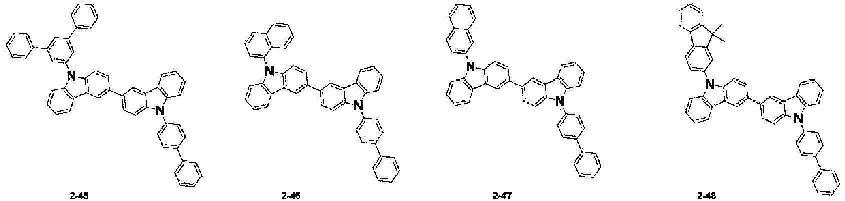

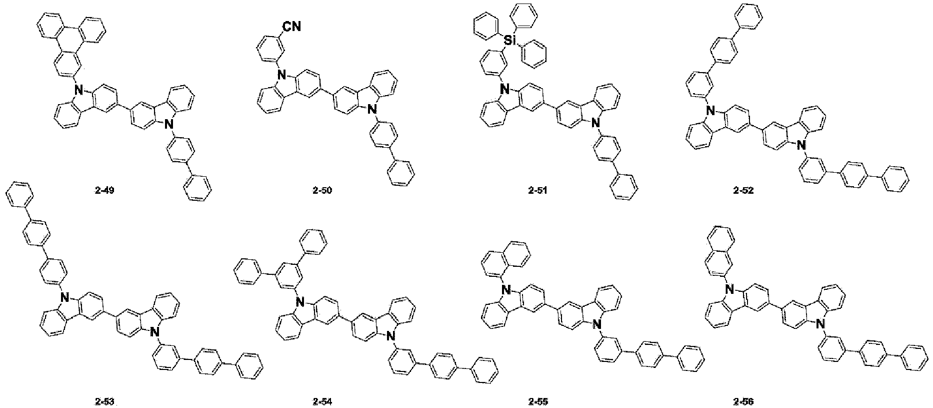

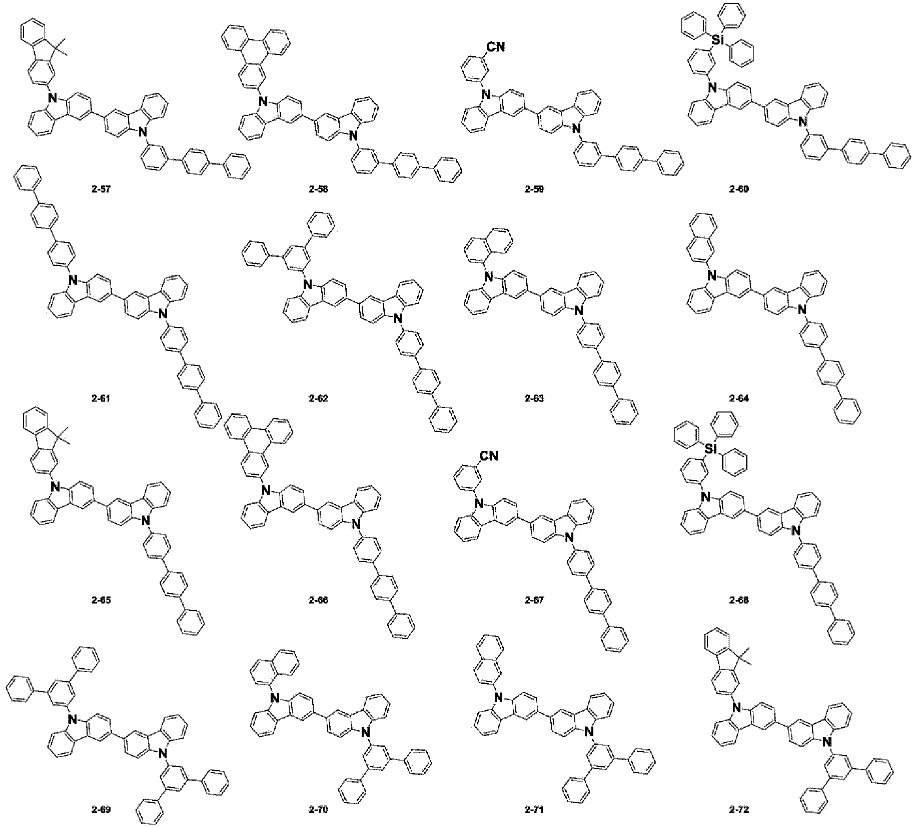

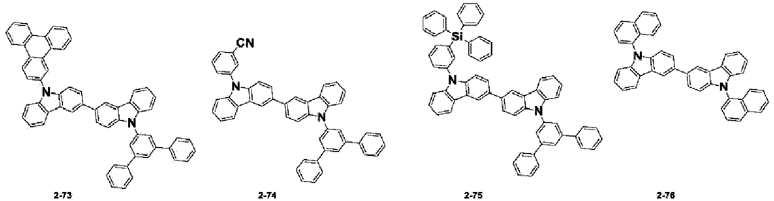

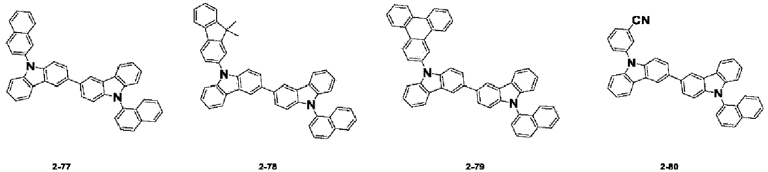

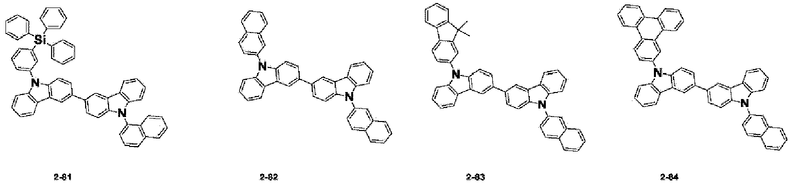

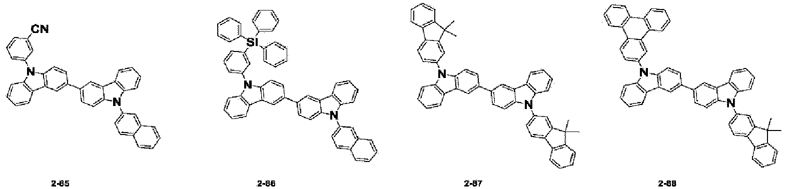

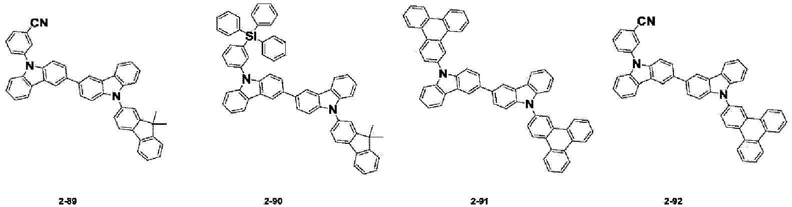

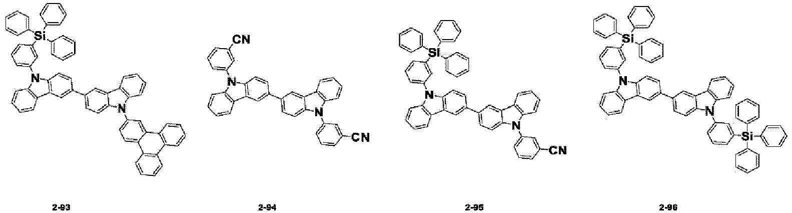

9. The organic light emitting device of claim 7, wherein Rc and Rd are hydrogen; or deuterium.

10. The organic light emitting device of claim 6, wherein the organic material layer comprises a light emitting layer, and the light emitting layer comprises the heterocyclic compound of Chemical Formula 1 above.

11. The organic light emitting device of claim 6, wherein the organic material layer comprises a light emitting layer, the light emitting layer comprises a host material, and the host material comprises the heterocyclic compound of Chemical Formula 1 above.

12. The organic light emitting device of claim 6, further comprising one or more layers selected from the group consisting of a light emitting layer, a hole injection layer, a hole transport layer, an electron injection layer, an electron transport layer, an electron blocking layer, and a hole blocking layer.

13. An organic material layer composition of an organic light emitting device, comprising the heterocyclic compound according to any one of claims 1 to 5 and a heterocyclic compound represented by the following Chemical Formula 2: 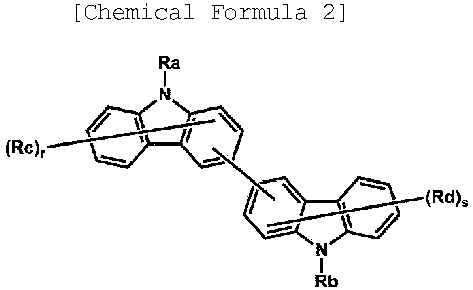
in Chemical Formula 2,
Rc and Rd are the same as or different from each other, and are each independently selected from the group consisting of: hydrogen; deuterium; a halogen group; -CN; a substituted or unsubstituted C₁-C₆₀ alkyl group; a substituted or unsubstituted C₂-C₆₀ alkenyl group; a substituted or unsubstituted C₂-C₆₀ alkynyl group; a substituted or unsubstituted C₁-C₆₀ alkoxy group; a substituted or unsubstituted C₃-C₆₀ cycloalkyl group; a substituted or unsubstituted C₂-C₆₀ heterocycloalkyl group; a substituted or unsubstituted C₆-C₆₀ aryl group; a substituted or unsubstituted C₂-C₆₀ heteroaryl group; -SiR101R102R103; - P(=O)R101R102; and -NR101R102, or two or more groups adjacent to each other are bonded to each other to form a substituted or unsubstituted C₆-C₆₀ aromatic hydrocarbon ring or a substituted or unsubstituted C₂-C₆₀ heteroring,
Ra and Rb are the same as or different from each other, and are each independently a substituted or unsubstituted C₆-C₆₀ aryl group; a substituted or unsubstituted C₂-C₆₀ heteroaryl group; -SiR101R102R103; or -P(=O)R101R102,
R101, R102, and R103 are the same as or different from each other, and are each independently hydrogen; deuterium; -CN; a substituted or unsubstituted C₁-C₆₀ alkyl group; a substituted or unsubstituted C₆-C₆₀ aryl group; or a substituted or unsubstituted C₂-C₆₀ heteroaryl group, and
r and s are integers of 0 to 7, and when r and s are each 2 or more, the substituents in parentheses are the same as or different from each other.

14. The organic material layer composition of the organic light emitting device of claim 13, wherein the weight ratio of the heterocyclic compound to the heterocyclic compound represented by Chemical Formula 2 above in the composition is 1:10 to 10:1.

15. A method for manufacturing an organic light emitting device, the method comprising the steps of:
preparing a substrate;
forming a first electrode on the substrate;
forming one or more organic material layers on the first electrode; and
forming a second electrode on the organic material layer,
wherein the step of forming the organic material layer comprises a step of forming one or more organic material layers using the organic material layer composition according to claim 13.

16. The method of claim 15, wherein the step of forming the organic material layer is forming the organic material layer using a thermal vacuum deposition method by premixing the heterocyclic compound of Chemical Formula 1 above and the heterocyclic compound of Chemical Formula 2 above.
